# EUROPEAN PATENT APPLICATION

(11) **EP 4 261 284 A1**
(43) Date of publication of application: **18.10.2023**
(21) Application number: 21903444.4
(22) Date of filing: 08.12.2021
(51) Int. Cl.: C12N 15/11, A61K 31/7105, A61K 31/711, A61K 31/712, A61K 31/7125, A61K 47/60, A61K 48/00, A61P 11/00, A61P 11/06, A61P 11/08, A61P 21/00, A61P 25/14, A61P 25/28, A61P 43/00, C07H 21/02, C07H 21/04, C12N 9/78, C12N 15/09

(54) **STABLE TARGET-EDITING GUIDE RNA TO WHICH CHEMICALLY MODIFIED NUCLEIC ACID IS INTRODUCED**

(30) Priority: 08.12.2020 JP 2020203658; 27.09.2021 JP 2021157151
(71) Applicant: Fukuoka University, Fukuoka-shi, Fukuoka 814-0180 (JP); Daiichi Sankyo Company, Limited, Tokyo 103-8426 (JP)
(72) Inventor: FUKUDA, Masatora, Fukuoka-shi, Fukuoka 814-0180 (JP); KOIZUMI, Makoto, Tokyo 103-8426 (JP); IWASHITA, Shinzo, Tokyo 103-8426 (JP)
(74) Representative: Vossius & Partner Patentanwälte Rechtsanwälte mbB
(86) International application number: PCT/JP2021/045184
(87) International publication number: WO 2022/124345

(57) **Abstract**

Provided is an oligonucleotide which may induce an editing activity of ADRC in cell and has excellent stability in a living body. The oligonucleotide includes a first oligonucleotide identifying a target RNA and a second oligonucleotide linked to the 5'-side of the first oligonucleotide. The first oligonucleotide consists of a target-corresponding nucleotide residue, an oligonucleotide of 10 to 24 residues at the 3'-side, and an oligonucleotide of 3 to 6 residues at the 5'-side. The second oligonucleotide has no nucleotide residue corresponding to a nucleotide residue of the target RNA or has a nucleotide residue which does not form a complementary pair at the 3'-end thereof and the number of residue is 3 to 6. The residue at the 3'-side of the target-corresponding nucleotide residue is a 2'-deoxynucleotide residue, and the third nucleotide residue counted in the 3'-direction from the target-corresponding nucleotide in the oligonucleotide at the 3'-side of the target-corresponding nucleotide residue is a 2'-deoxy-2'-fluoronucleotide residue.

## Description

### Technical Field

The present invention relates to a stable target-editing guide RNA to which a chemically modified nucleic acid is introduced.

### Background Art

The development of genome editing techniques has triggered the use of methods for controlling biological phenomena by alteration of genetic information serving as design drawings of organisms, namely, DNA information in cells, as disease treatment approaches in the medical and drug discovery fields. DNAs are invariant and unchangeable molecules in cells, and thus the effect of DNA alteration permanently remains in objective cells or objective organisms. On the other hand, RNAs are transient genetic information molecules. Accordingly, alteration of RNA information can impart the effect of non-permanent and temporary genetic information alteration to objective organisms.

For example, WO2016/097212 describes, as an RNA alteration technique, an oligonucleotide construct for site-specific editing of nucleotides in a target RNA sequence, comprising a target-directing portion containing an antisense sequence complementary to one portion of a target RNA, and a recruitment portion capable of binding and recruiting an RNA editing entity which exists in cells and can edit nucleotides. For example, WO2017/010556 describes a site-specific RNA mutation introduction method involving allowing a double-stranded specific adenosine deaminase (ADAR) to act on a complex of a target RNA and a target-editing guide RNA. Nature Biotechnology 37, 133-138 (2019) describes introduction of a modified nucleic acid to an antisense oligonucleotide that recruits ADAR.

Isoforms of ADAR1 and ADAR2 are known as adenosine deaminases having an RNA editing activity, and these are considered to be expressed *in vivo* specifically to cell species. For example, ADAR1 is considered almost not to be expressed in human skeletal muscle cells. On the other hand, ADAR2 is considered almost not to be expressed in human bone marrow cells. In general, ADAR1 is considered to be more expressed in human cells.

### Summary of Invention

### Technical Problem

It is considered that, if the editing activity of ADAR1 can be induced in cells, the RNA editing specific to cell species can be made. It is also considered that more efficient RNA editing can be made in human cells. Accordingly, an object of one embodiment of the present invention is to provide an oligonucleotide that may induce the editing activity of ADAR1 in cells and that is excellent in stability *in vivo.*

### Solution to Problem

Specific solutions for solving the above problems are as follows, and the present invention encompasses the following embodiments.
(1-1) An oligonucleotide, or a pharmaceutically acceptable salt thereof, comprising: a first oligonucleotide identifying a target RNA; and a second oligonucleotide linked to the 5'-side of the first oligonucleotide, wherein the first oligonucleotide consists of a target-corresponding nucleotide residue corresponding to an adenosine residue in the target RNA, an oligonucleotide of 10 to 24 residues, linked to the 3'-side of the target-corresponding nucleotide residue and having a base sequence complementary to the target RNA, and an oligonucleotide of 3 to 6 residues, linked to the 5'-side of the target-corresponding nucleotide residue and having a base sequence complementary to the target RNA, the second oligonucleotide has no nucleotide residue corresponding to a nucleotide residue of the target RNA or has a nucleotide residue which does not form a complementary pair with a nucleotide residue of the target RNA, at the 3'-end thereof, a number of residues in the second oligonucleotide is 2 to 10, and at least the nucleotide residues at other than the 3'-end form a double-stranded structure complementary to the target RNA, at least one residue selected from a counter region consisting of the target-corresponding nucleotide residue and each one residue at the 3'-side and the 5'-side thereof is a nucleotide residue other than a natural ribonucleotide residue, the oligonucleotide, or a pharmaceutically acceptable salt thereof induces site-specific editing for the target RNA.
(1-2) The oligonucleotide, or a pharmaceutically acceptable salt thereof, according to (1-1), wherein the number of residues in the second oligonucleotide is 4 to 8.
(1-3) The oligonucleotide, or a pharmaceutically acceptable salt thereof, according to (1-1) or (1-2), wherein the 3'-end of the second oligonucleotide has no nucleotide residue corresponding to a nucleotide residue of the target RNA.
(1-4) The oligonucleotide, or a pharmaceutically acceptable salt thereof, according to any of (1-1) to (1-3), wherein the site-specific editing is due to an enzyme reaction by adenosine deaminase 1.
(1-5) The oligonucleotide, or a pharmaceutically acceptable salt thereof, according to any of (1-1) to (1-4), comprising a linking portion containing an alkyleneoxy unit between the first oligonucleotide and the second oligonucleotide.
(1-6) The oligonucleotide, or a pharmaceutically acceptable salt thereof, according to any of (1-1) to (1-5), wherein the first oligonucleotide contains a phosphorothioate bond.
(1-7) The oligonucleotide, or a pharmaceutically acceptable salt thereof, according to any of (1-1) to (1-6), wherein the first oligonucleotide contains at least one modified nucleotide residue selected from the group consisting of a 2'-O-alkyl ribonucleotide residue, a 2'-deoxy-2'-fluoro ribonucleotide residue, a bridged nucleotide residue, and a 2'-deoxyribonucleotide.
(1-8) The oligonucleotide, or a pharmaceutically acceptable salt thereof, according to any of (1-1) to (1-7), wherein the counter region contains a phosphorothioate bond.
(1-9) The oligonucleotide, or a pharmaceutically acceptable salt thereof, according to any of (1-1) to (1-8), wherein the target-corresponding nucleotide residue contains a phosphorothioate bond.
(1-10) The oligonucleotide, or a pharmaceutically acceptable salt thereof, according to any of (1-1) to (1-9), wherein at least one residue of the each residue at the 3'-side and the 5'-side of the target-corresponding nucleotide residue is a 2'-O-alkyl ribonucleotide residue or a deoxynucleotide residue.
(1-11) The oligonucleotide, or a pharmaceutically acceptable salt thereof, according to any of (1-1) to (1-10), wherein the second oligonucleotide contains at least one modified nucleotide residue selected from the group consisting of a 2'-O-alkyl ribonucleotide residue, a 2'-deoxy-2'-fluoro ribonucleotide residue, and a bridged nucleotide residue.
(1-12) The oligonucleotide, or a pharmaceutically acceptable salt thereof, according to any of (1-1) to (1-11), wherein the oligonucleotide linked to the 3'-side of the target-corresponding nucleotide residue has a base sequence in which a 2'-deoxy-2'-fluoronucleotide residue and a 2'-O-alkyl ribonucleotide residue are alternately linked.
(1-13) The oligonucleotide, or a pharmaceutically acceptable salt thereof, according to (1-12), wherein in an oligonucleotide linked to the 3'-side of the target-corresponding nucleotide residue, a third nucleotide residue counted in the 3'-direction from the target-corresponding nucleotide is a 2'-deoxy-2'-fluoronucleotide residue.
(1-14) The oligonucleotide, or a pharmaceutically acceptable salt thereof, according to any of (1-1) to (1-11), wherein the oligonucleotide linked to the 3'-side of the target-corresponding nucleotide residue has a base sequence in which a bridged nucleotide residue and a 2'-O-alkyl ribonucleotide residue are alternately linked.
(1-15) The oligonucleotide, or a pharmaceutically acceptable salt thereof, according to (1-14), wherein in an oligonucleotide linked to the 3'-side of the target-corresponding nucleotide residue, a third nucleotide residue counted in the 3'-direction from the target-corresponding nucleotide is a bridged nucleotide residue.
(1-16) The oligonucleotide, or a pharmaceutically acceptable salt thereof, according to any of (1-1) to (1-11), wherein the oligonucleotide linked to the 3'-side of the target-corresponding nucleotide residue has a base sequence in which a 2'-deoxy-2'-fluoronucleotide residue and a bridged nucleotide residue are alternately linked.
(1-17) The oligonucleotide, or a pharmaceutically acceptable salt thereof, according to (1-16), wherein in an oligonucleotide linked to the 3'-side of the target-corresponding nucleotide residue, a third nucleotide residue counted in the 3'-direction from the target-corresponding nucleotide is a 2'-deoxy-2'-fluoronucleotide.
(1-18) The oligonucleotide, or a pharmaceutically acceptable salt thereof, according to any of (1-1) to (1-17), wherein an oligonucleotide linked to the 5'-side of the target-corresponding nucleotide residue in the first oligonucleotide has a base sequence in which a 2'-O-alkyl ribonucleotide residue is linked.
(1-19) The oligonucleotide, or a pharmaceutically acceptable salt thereof, according to any of (1-1) to (1-17), wherein an oligonucleotide linked to the 5'-side of the target-corresponding nucleotide residue in the first oligonucleotide has a base sequence in which a 2'-O-alkyl ribonucleotide residue and a bridged nucleotide residue are alternately linked.
(1-20) The oligonucleotide, or a pharmaceutically acceptable salt thereof, according to any of (1-1) to (1-17), wherein an oligonucleotide linked to the 5'-side of the target-corresponding nucleotide residue in the first oligonucleotide has a base sequence in which a 2'-O-alkyl ribonucleotide residue and a 2'-deoxy-2'-fluoronucleotide residue are alternately linked.
(1-21) The oligonucleotide, or a pharmaceutically acceptable salt thereof, according to any of (1-1) to (1-20), wherein the second oligonucleotide has a base sequence in which a 2'-O-alkyl ribonucleotide residue is linked.
(1-22) The oligonucleotide, or a pharmaceutically acceptable salt thereof, according to any of (1-1) to (1-20), wherein the second oligonucleotide has a base sequence in which a 2'-O-alkyl ribonucleotide residue and a bridged nucleotide residue are alternately linked.
(1-23) The oligonucleotide, or a pharmaceutically acceptable salt thereof, according to any of (1-1) to (1-20), wherein the second oligonucleotide has a base sequence in which a 2'-O-alkyl ribonucleotide residue and a 2'-deoxy-2'-fluoronucleotide residue are alternately linked.
(1-24) The oligonucleotide, or a pharmaceutically acceptable salt thereof, according to any of (1-1) to (1-23), wherein a cytidine residue is linked to the 5'-side of the adenosine residue to be edited in the target RNA, and the nucleotide residue linked to the 3'-side of the target-corresponding nucleotide residue in the first oligonucleotide has a hypoxanthine residue as a base.
(1-25) The oligonucleotide, or a pharmaceutically acceptable salt thereof, according to any of (1-1) to (1-24), wherein the target-corresponding nucleotide residue is an *N-*alkylpyrimidine nucleotide residue or a 2'-deoxycytidine residue.
(1-26) The oligonucleotide, or a pharmaceutically acceptable salt thereof, according to any of (1-1) to (1-25), wherein the first oligonucleotide and the second oligonucleotide are each formed by linking nucleotide residues by a phosphorothioate bond.
(1-27) A hereditary disease therapeutic agent comprising the oligonucleotide, or a pharmaceutically acceptable salt thereof, according to any of (1-1) to (1-26).
(1-28) A pharmaceutical composition comprising the oligonucleotide, or a pharmaceutically acceptable salt thereof, according to any of (1-1) to (1-26), as an active component.
(1-29) The pharmaceutical composition according to (1-28), for the prevention or therapy of a hereditary disease.
(1-30) The pharmaceutical composition according to (1-29), wherein the hereditary disease is a disease curable by conversion of an adenosine residue into an inosine residue in the target RNA.
(1-31) The pharmaceutical composition according to (1-29), wherein the hereditary disease is a hereditary disease caused by mutation of a guanosine residue to an adenosine residue in a gene.
(1-32) Use of the oligonucleotide, or a pharmaceutically acceptable salt thereof, according to any of (1-1) to (1-26), for production of a medicine for prevention or therapy of a disease associated with the target RNA.
(1-33) The oligonucleotide, or a pharmaceutically acceptable salt thereof, according to any of (1-1) to (1-26), for use in the prevention or therapy of a disease.
(1-34) A method comprising administering a pharmacologically effective amount of the oligonucleotide, or a pharmaceutically acceptable salt thereof, according to any of (1-1) to (1-2), to a warm-blooded animal, to prevent or cure a disease associated with the target RNA.
(1-35) The method according to (1-34), wherein the disease is a hereditary disease.
(1-36)The method according to (1-35), wherein the hereditary disease is a disease curable by conversion of an adenosine residue into an inosine residue in the target RNA.
(1-37) The method according to (1-35), wherein the hereditary disease is a hereditary disease caused by mutation of a guanosine residue to an adenosine residue in a gene.
(1-38) The method according to any of (1-34) to (1-37), wherein the warm-blooded animal is human.
(1-39) An oligonucleotide, or a pharmaceutically acceptable salt thereof, comprising: a first oligonucleotide identifying a target RNA; a second oligonucleotide linked to the 5'-side of the first oligonucleotide; and a linking portion linking the first oligonucleotide and the second oligonucleotide and conatining a polyalkyleneoxy group; wherein the first oligonucleotide consists of a target-corresponding nucleotide residue corresponding to an adenosine residue in the target RNA, an oligonucleotide of 10 to 24 residues, linked to the 3'-side of the target-corresponding nucleotide residue and having a base sequence complementary to the target RNA, and an oligonucleotide of 3 to 6 residues, linked to the 5'-side of the target-corresponding nucleotide residue and having a base sequence complementary to the target RNA, wherein a number of residues in the second oligonucleotide is 2 to 10, wherein the second oligonucleotide has no nucleotide residue corresponding to a nucleotide residue of the target RNA or has a nucleotide residue which does not form a complementary pair with a nucleotide residue of the target RNA, at the 3'-end thereof, and at least the nucleotide residues at other than the 3'-end form a double-stranded structure complementary to the target RNA or have a complementary base sequence to he target RNA to form a double-stranded structure complementary to the target RNA, wherein at least one residue selected from a counter region consisting of the target-corresponding nucleotide residue and each one residue at the 3'-side and the 5'-side thereof is a nucleotide residue other than a natural ribonucleotide residue, and wherein the oligonucleotide or a pharmaceutically acceptable salt thereof induces site-specific editing for the target RNA.
(2-1) An oligonucleotide, or a pharmaceutically acceptable salt thereof, comprising: a first oligonucleotide identifying a target RNA; and a second oligonucleotide linked to the 5'-side of the first oligonucleotide, wherein the first oligonucleotide consists of a target-corresponding nucleotide residue corresponding to an adenosine residue in the target RNA, an oligonucleotide of 10 to 24 residues, linked to the 3'-side of the target-corresponding nucleotide residue and having a base sequence complementary to the target RNA, and an oligonucleotide of 3 to 6 residues, linked to the 5'-side of the target-corresponding nucleotide residue and having a base sequence complementary to the target RNA, the second oligonucleotide has no nucleotide residue corresponding to a nucleotide residue of the target RNA or has a nucleotide residue which does not form a complementary pair with a nucleotide residue of the target RNA, at the 3'-end thereof, (hereinafter, a nucleotide residue that does not form a complementary pair with the nucleotide residues of the target RNA in the second oligonucleotide and the target-corresponding nucleotide residues are collectively also referred to as "non-complementary nucleotide residues".), a number of residues in the second oligonucleotide is 2 to 10, and at least the nucleotide residues at other than the 3'-end form a double-stranded structure complementary to the target RNA, a counter region consisting of the target-corresponding nucleotide residue and each one residue at the 3'-side and the 5'-side thereof is contained, the nucleotide residue linked to the 3'-side of the target-corresponding nucleotide residue is a 2'-deoxynucleotide residue, the third nucleotide residue counted in the 3'-direction from the target-corresponding nucleotide in the oligonucleotide linked to the 3'-side of the target-corresponding nucleotide residue is a 2'-deoxy-2'-fluoronucleotide residue, and the oligonucleotide, or a pharmaceutically acceptable salt thereof induces site-specific editing for the target RNA.
(2-2) The oligonucleotide, or a pharmaceutically acceptable salt thereof, according to (2-1), wherein the number of residues in the second oligonucleotide is 4 to 8.
(2-3) The oligonucleotide, or a pharmaceutically acceptable salt thereof, according to (2-1) or (2-2), wherein the 3'-end of the second oligonucleotide has no nucleotide residue corresponding to a nucleotide residue of the target RNA.
(2-4) The oligonucleotide, or a pharmaceutically acceptable salt thereof, according to any of (2-1) to (2-3), wherein the site-specific editing is due to an enzyme reaction by adenosine deaminase 1.
(2-5) The oligonucleotide, or a pharmaceutically acceptable salt thereof, according to any of (2-1) to (2-4), comprising a linking portion containing an alkyleneoxy unit between the first oligonucleotide and the second oligonucleotide.
(2-6) The oligonucleotide, or a pharmaceutically acceptable salt thereof, according to any of (2-1) to (2-5), wherein the first oligonucleotide contains a phosphorothioate bond.
(2-7) The oligonucleotide, or a pharmaceutically acceptable salt thereof, according to any of (2-1) to (2-6), wherein the first oligonucleotide contains at least one modified nucleotide residue selected from the group consisting of a 2'-O-alkyl ribonucleotide residue, a 2'-deoxy-2'-fluoro ribonucleotide residue, a bridged nucleotide residue, and a 2'-deoxyribonucleotide.
(2-8) The oligonucleotide, or a pharmaceutically acceptable salt thereof, according to any of (2-1) to (2-7), wherein the counter region contains a phosphorothioate bond.
(2-9) The oligonucleotide, or a pharmaceutically acceptable salt thereof, according to any of (2-1) to (2-8), wherein the target-corresponding nucleotide residue contains a phosphorothioate bond.
(2-10) The oligonucleotide, or a pharmaceutically acceptable salt thereof, according to any of (2-1) to (2-9), wherein the nucleotide residue linked to the 5'-side of the target-corresponding nucleotide residue is a 2'-O-alkyl ribonucleotide residue or a deoxynucleotide residue.
(2-11) The oligonucleotide, or a pharmaceutically acceptable salt thereof, according to any of (2-1) to (2-10), wherein the second oligonucleotide contains at least one modified nucleotide residue selected from the group consisting of a 2'-O-alkyl ribonucleotide residue, a 2'-deoxy-2'-fluoro ribonucleotide residue, and a bridged nucleotide residue.
(2-12) The oligonucleotide, or a pharmaceutically acceptable salt thereof, according to any of (2-1) to (2-11), wherein the oligonucleotide linked to the 3'-side of the target-corresponding nucleotide residue has a base sequence in which a 2'-deoxy-2'-fluoronucleotide residue and a 2'-O-alkyl ribonucleotide residue are alternately linked.
(2-13) The oligonucleotide, or a pharmaceutically acceptable salt thereof, according to any of (2-1) to (2-12), wherein the oligonucleotide linked to the 3'-side of the target-corresponding nucleotide residue has a base sequence in which a bridged nucleotide residue and a 2'-O-alkyl ribonucleotide residue are alternately linked.
(2-14) The oligonucleotide, or a pharmaceutically acceptable salt thereof, according to any of (2-1) to (2-13), wherein the oligonucleotide linked to the 3'-side of the target-corresponding nucleotide residue has a base sequence in which a 2'-deoxy-2'-fluoronucleotide residue and a bridged nucleotide residue are alternately linked.
(2-15) The oligonucleotide, or a pharmaceutically acceptable salt thereof, according to any of (2-1) to (2-14), wherein an oligonucleotide linked to the 5'-side of the target-corresponding nucleotide residue in the first oligonucleotide has a base sequence in which a 2'-O-alkyl ribonucleotide residue is linked.
(2-16) The oligonucleotide, or a pharmaceutically acceptable salt thereof, according to any of (2- 1) to (2-15), wherein an oligonucleotide linked to the 5'-side of the target-corresponding nucleotide residue in the first oligonucleotide has a base sequence in which a 2'-O-alkyl ribonucleotide residue and a bridged nucleotide residue are alternately linked.
(2-17) The oligonucleotide, or a pharmaceutically acceptable salt thereof, according to any of (2- 1) to (2-16), wherein an oligonucleotide linked to the 5'-side of the target-corresponding nucleotide residue in the first oligonucleotide has a base sequence in which a 2'-O-alkyl ribonucleotide residue and a 2'-deoxy-2'-fluoronucleotide residue are alternately linked.
(2-18) The oligonucleotide, or a pharmaceutically acceptable salt thereof, according to any of (2-1) to (2-17), wherein the second oligonucleotide has a base sequence in which a 2'-O-alkyl ribonucleotide residue is linked.
(2-19) The oligonucleotide, or a pharmaceutically acceptable salt thereof, according to any of (2-1) to (2-18), wherein the second oligonucleotide has a base sequence in which a 2'-O-alkyl ribonucleotide residue and a bridged nucleotide residue are alternately linked.
(2-20) The oligonucleotide, or a pharmaceutically acceptable salt thereof, according to any of (2-1) to (2-19), wherein the second oligonucleotide has a base sequence in which a 2'-O-alkyl ribonucleotide residue and a 2'-deoxy-2'-fluoronucleotide residue are alternately linked.
(2-21) The oligonucleotide, or a pharmaceutically acceptable salt thereof, according to any of (2-1) to (2-20), wherein a cytidine residue is linked to the 5'-side of the adenosine residue to be edited in the target RNA, and the nucleotide residue linked to the 3'-side of the target-corresponding nucleotide residue in the first oligonucleotide is a 2'-deoxyinosine residue.
(2-22) The oligonucleotide, or a pharmaceutically acceptable salt thereof, according to any of (2-1) to (2-20), wherein a uridine residue is linked to the 5'-side of the adenosine residue to be edited in the target RNA, and the nucleotide residue linked to the 3'-side of the target-corresponding nucleotide residue in the first oligonucleotide is a 2'-deoxyadenosine residue.
(2-23) The oligonucleotide, or a pharmaceutically acceptable salt thereof, according to any of (2-1) to (2-20), wherein an adenosine residue is linked to the 5'-side of the adenosine residue to be edited in the target RNA, and the nucleotide residue linked to the 3'-side of the target-corresponding nucleotide residue in the first oligonucleotide is a thymidine residue or a 2'-deoxyuridine residue.
(2-24) The oligonucleotide, or a pharmaceutically acceptable salt thereof, according to any of (2-1) to (2-20), wherein a guanosine residue is linked to the 5'-side of the adenosine residue to be edited in the target RNA, and the nucleotide residue linked to the 3'-side of the target-corresponding nucleotide residue in the first oligonucleotide is a 2'-deoxyinosine residue.
(2-25) The oligonucleotide, or a pharmaceutically acceptable salt thereof, according to any of (2-1) to (2-24), wherein the target-corresponding nucleotide residue is an N-alkylpyrimidine nucleotide residue or a 2'-deoxycytidine residue.
(2-26) The oligonucleotide, or a pharmaceutically acceptable salt thereof, according to any of (2-1) to (2-25), wherein the first oligonucleotide and the second oligonucleotide are each formed by linking nucleotide residues by a phosphorothioate bond.
(2-27) The oligonucleotide, or a pharmaceutically acceptable salt thereof, according to any of (2-1) to (2-26), wherein the first oligonucleotide contains a bridged nucleotide residue in the tenth or subsequent nucleotide residues counted in the 3'-direction from the target-corresponding nucleotide.
(2-28) The oligonucleotide, or a pharmaceutically acceptable salt thereof, according to (2-27), wherein an oligonucleotide composed of the tenth or subsequent nucleotide residues counted in the 3'-direction from the target-corresponding nucleotide in the first oligonucleotide has a base sequence in which a 2'-O-alkyl ribonucleotide residue and a bridged nucleotide residue are alternately linked.
(2-29) The oligonucleotide, or a pharmaceutically acceptable salt thereof, according to any of (2-1) to (2-28), wherein the second oligonucleotide contains a bridged nucleotide residue in the second or subsequent nucleotide residues counted in the 5'-direction from the nucleotide residue at the 3'-end thereof.
(1-30) The oligonucleotide, or a pharmaceutically acceptable salt thereof, according to (2-29), wherein an oligonucleotide composed of the second or subsequent nucleotide residues counted in the 5'-direction from the nucleotide residue at the 3'-end of the second oligonucleotide has a base sequence in which a 2'-O-alkyl ribonucleotide residue and a bridged nucleotide residue are alternately linked.
(2-31) The oligonucleotide, or a pharmaceutically acceptable salt thereof, according to any of (2-1) to (2-30), represented by any of the following formulae:

U(M)^T(L)^G(M)^A(L)^U(M)^G(F)^A(F)^C(M)^C(M)^c^i^G(M)^U(F)^G(
M)^G(F)^C(M)^A(F)^U(M)^C(F)^A(M)^G(L)^U(M)^T(L)^G(M) (AD1_ASS1.39)

A(M)^A(L)^G(M)^A(L)^A(M)^A(F)^U(F)^U(M)^C(M)^c^a^A(M)^C(F)^A
(M)^A(F)^A(M)^U(F)^A(M)^C(F)^C(M)^A(L)^C(M)^C(L)^U(M)
(AD1_PANK2.39)

A(M)^T(L)^G(M)^T(L)^C(M)^C(F)^A(F)^G(M)^U(M)^c^i^G(M)^A(F)^A(
M)^U(F)^A(M)^U(F)^A(M)^A(F)^U(M)^T(L)^A(M)^C(L)^U(M)
(AD1_NPHS2.39)

G(M)^C(L)^A(M)^T(L)^C(M)^C(F)^U(F)^G(M)^C(M)^c^i^C(M)^A(F)^U(

M)^A(F)^A(M)^A(F)^G(M)^G(F)^C(M)^A(L)^C(M)^C(L)^C(M)
(AD1_GRIA2.39)

U(M)^G(L)^A(M)^U(L)^G(F)^A(F)^C(M)^C(M)^c^i^G(M)^U(F)^G(M)^G(
F)^C(M)^A(F)^U(M)^C(F)^A(L)^G(M)^U(L)^U(M) (AD1_ASS1.52)

G(M)^C(E)^A(M)^T(E)^C(M)^C(F)^U(F)^G(M)^C(M)^c^i^C(M)^A(F)^U(

M)^A(F)^A(M)^A(F)^G(M)^G(F)^C(M)^A(E)^C(M)^C(E)^C(M)

(AD1_GRIA2.39e)

U(M)^T(E)^G(M)^A(E)^U(M)^G(F)^A(F)^C(M)^C(M)^c^i^G(M)^U(F)^G(
M)^G(F)^C(M)^A(F)^U(M)^C(F)^A(M)^G(E)^U(M)^T(E)^G(M)
(AD1_ASS1.39e)

A(M)^A(E)^G(M)^A(E)^A(M)^A(F)^U(F)^U(M)^C(M)^c^a^A(M)^C(F)^A
(M)^A(F)^A(M)^U(F)^A(M)^C(F)^C(M)^A(E)^C(M)^C(E)^U(M)
(AD1_PANK2.39e)

A(M)^T(E)^G(M)^T(E)^C(M)^C(F)^A(F)^G(M)^U(M)^c^i^G(M)^A(F)^A(
M)^U(F)^A(M)^U(F)^A(M)^A(F)^U(M)^T(E)^A(M)^C(E)^U(M)
(AD1_NPHS2.39e)

C(M)^A(E)^U(M)^C(E)^C(F)^U(F)^G(M)^C(M)^c^i^C(M)^A(F)^U(M)^A(
F)^A(M)^A(F)^G(M)^G(F)^C(E)^A(M)^C(E)^C(M)) (AD1_GRIA2.52e)

U(M)^G(E)^A(M)^T(E)^G(F)^A(F)^C(M)^C(M)^c^i^G(M)^U(F)^G(M)^G(
F)^C(M)^A(F)^U(M)^C(F)^A(E)^G(M)^T(E)^U(M) (AD1_ASS1.52e)

A(M)^G(E)^A(M)^A(E)^A(F)^U(F)^U(M)^C(M)^c^a^A(M)^C(F)^A(M)^A
(F)^A(M)^U(F)^A(M)^C(F)^C(E)^A(M)^C(E)^C(M) (AD1_PANK2.52e)

U(M)^G(E)^U(M)^C(E)^C(F)^A(F)^G(M)^U(M)^c^i^G(M)^A(F)^A(M)^U(
F)^A(M)^U(F)^A(M)^A(F)^T(E)^U(M)^A(E)^C(M) (AD1_NPHS2.52e)

G(M)^T(L)^C(M)^C(L)^C(M)^U(F)^U(F)^C(M)^U(M)^c^i^U(M)^C(F)^G(
M)^A(F)^U(M)^G(F)^G(M)^U(F)^C(M)^A(L)^G(M)^C(L)^A(M)
(AD1_A1AT.39).

In the formulae, a capital letter denotes a ribonucleotide residue, a small letter represents a 2'-deoxyribonucleotide residue, N(M) represents a 2'-O-methyl-ribonucleotide residue, N(F) represents a 2'-fluoro-2'-deoxyribonucleotide residue, N(L) represents a 2'-O,4'-C-methylenated ribonucleotide residue, N(E) represents a 2'-O,4'-C-ethylenated ribonucleotide residue, and "^" represents that nucleoside units are linked by -P(=S)(OH)-.
(2-32) The oligonucleotide, or a pharmaceutically acceptable salt thereof, according to any of (2-1) to (2-31), wherein a delivery molecule containing GalNAc, cholesterol, and a fatty acid is bound via a linker or a phosphodiester bond (including a phosphorothioate bond) at the 5'-end or the 3'-end of the oligonucleotide and the pharmaceutically acceptable salt thereof.
(2-33) A hereditary disease therapeutic agent comprising the oligonucleotide, or a pharmaceutically acceptable salt thereof, according to any of (2-1) to (2-32), for use in the treatment of a disease associated with the target RNA.
(2-34) A pharmaceutical composition comprising the oligonucleotide, or a pharmaceutically acceptable salt thereof, according to any of (2-1) to (2-32), as an active component, for use in the therapy of a disease associated with the target RNA.
(2-35) The pharmaceutical composition according to (2-34), for the prevention or therapy of a hereditary disease.
(2-36) The pharmaceutical composition according to (2-35), wherein the hereditary disease is a disease curable by conversion of an adenosine residue into an inosine residue in the target RNA.
(2-37) The pharmaceutical composition according to (2-35), wherein the hereditary disease is a hereditary disease caused by mutation of a guanosine residue to an adenosine residue in a gene.
(2-38) Use of the oligonucleotide, or a pharmaceutically acceptable salt thereof, according to any of (2-1) to (2-32), for production of a medicine for prevention or therapy of a disease associated with the target RNA.
(2-39) The oligonucleotide, or a pharmaceutically acceptable salt thereof, according to any of (2-1) to (2-32), for use in the prevention or therapy of a disease associated with the target RNA.
(2-40) A method comprising administering a pharmacologically effective amount of the oligonucleotide, or a pharmaceutically acceptable salt thereof, according to any of (2-1) to (2-32), to a warm-blooded animal, to prevent or cure a disease associated with the target RNA.
(2-41) The method according to (2-40), wherein the disease is a hereditary disease.
(2-42) The method according to (2-41), wherein the hereditary disease is a disease curable by conversion of an adenosine residue into an inosine residue in the target RNA.
(2-43) The method according to (2-41), wherein the hereditary disease is a hereditary disease caused by mutation of a guanosine residue to an adenosine residue in a gene.
(2-44) The pharmaceutical composition according to any of (2-34) to (2-37), wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.
(2-45) The use of the oligonucleotide, or a pharmaceutically acceptable salt thereof, according to (2-38), wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.
(2-46) The oligonucleotide, or a pharmaceutically acceptable salt thereof, according to (2-39), wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.
(2-47) The method according to any one of claims 40 to 43, wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.
(3-1) An oligonucleotide, or a pharmaceutically acceptable salt thereof, represented by the following formula and containing 20 to 40 residues which contain a phosphorothioate bond between the nucleotide residues, the oligonucleotide, or a pharmaceutically acceptable salt thereof, is used in gene editing for conversion of the adenosine residue to be edited in a target RNA, into an inosine residue;
   5'-O1-O2-N1-N2-N3-N4-N5-O3-O4-3';
      wherein N1 is a 2'-O-alkyl ribonucleotide residue, a ribonucleotide residue, a 2'-fluoro-2'-deoxyribonucleotide residue or a 2'-deoxyribonucleotide residue,
   N2 is a ribonucleotide residue, a 2'-O-alkyl ribonucleotide residue, or a 2'-deoxyribonucleotide residue, in which the base is cytosine or 3-methyluracil,
   N3 is a 2'-deoxyribonucleotide residue,
   N4 is a 2'-O-alkyl ribonucleotide residue or a 2'-fluoro-2'-deoxyribonucleotide residue,
   N5 is a 2'-fluoro-2'-deoxyribonucleotide residue,
   O1 is absent or an oligonucleotide of 2 to 10 residues, and contains at least one bridged nucleotide residue, in which any other than the bridged nucleotide residue is a 2'-fluoro-2'-deoxyribonucleotide residue or a 2'-O-alkyl ribonucleotide residue,
   O2 is an oligonucleotide of 2 to 10 residues and is composed of a 2'-fluoro-2'-deoxyribonucleotide residue and a 2'-O-alkyl ribonucleotide residue,
   O3 is an oligonucleotide of 5 to 20 residues and is composed of a 2'-fluoro-2'-deoxyribonucleotide residue and a 2'-O-alkyl ribonucleotide residue, and
   O4 is absent or an oligonucleotide of 2 to 10 residues, and contains at least one bridged nucleotide residue, in which any other than the bridged nucleotide residue is a 2'-fluoro-2'-deoxyribonucleotide residue or a 2'-O-alkyl ribonucleotide residue;
   in the oligonucleotide, N2 corresponds to the adenosine residue to be edited and O2 has no nucleotide residue corresponding to a corresponding nucleotide residue of the target RNA or has a nucleotide residue which does not form a complementary pair with the corresponding nucleotide residue of the target RNA in the second to fifth regions from the 3'-side,
   in the base sequence of the oligonucleotide, the base of N3 is optionally a base complementary or non-complementary to the base adjacent to the 5'-side of the adenosine residue to be edited, and the bases of N1, N4, N5, O1, O3, and O4 are completely complementary to the bases of the corresponding nucleotide residues of the target RNA, and
   the base of O2, other than a lack nucleotide residue and a nucleotide residue which does not form a complementary pair with the corresponding nucleotide residue of the target RNA in O2, is completely complementary to the base of the corresponding nucleotide residue of the target RNA.
(3-2) The oligonucleotide, or a pharmaceutically acceptable salt thereof, according to (3-1), wherein, when the nucleotide residue at the 5'-side of the adenosine residue to be edited, and N3 form a complementary pair, N3 is a 2'-deoxyadenosine residue, a 2'-deoxyinosine residue (provided that a case is excluded in which the nucleotide residue at the 5'-side of the adenosine residue to be edited is an adenosine residue or a uridine residue), a thymidine residue or a 2'-deoxyuridine residue (provided that a case is excluded in which the nucleotide residue at the 5'-side of the adenosine residue to be edited is a guanosine residue), and when the nucleotide residue at the 5'-side of the adenosine residue to be edited, and N3 do not form a complementary pair, N3 is a 2'-deoxyinosine residue.
(3-3) The oligonucleotide, or a pharmaceutically acceptable salt thereof, according to (3-1), wherein, when a cytidine residue is linked to the 5'-side of the adenosine residue to be edited in the target RNA, N3 is a 2'-deoxyinosine residue, when a uridine residue is linked to the 5'-side of the adenosine residue to be edited in the target RNA, N3 is a 2'-deoxyadenosine residue, when the adenosine residue is linked to the 5'-side of the adenosine residue to be edited in the target RNA, N3 is a thymidine residue or a 2'-deoxyuridine residue, and when a guanosine residue is linked to the 5'-side of the adenosine residue to be edited in the target RNA, N3 is a 2'-deoxyinosine residue.
(3-4) The oligonucleotide, or a pharmaceutically acceptable salt thereof, according to any of (3-1) to (3-3), wherein N1 and N4 are 2'-O-alkyl ribonucleotide residues.
(3-5) The oligonucleotide, or a pharmaceutically acceptable salt thereof, according to any of (3-1) to (3-4), wherein at least one of O2 and O3 contains a sequence in which a 2'-fluoro-2'-deoxyribonucleotide residue and a 2'-O-alkyl ribonucleotide residue is alternately disposed.
(3-6) The oligonucleotide, or a pharmaceutically acceptable salt thereof, according to any of (3-1) to (3-5), wherein O3 is composed of a sequence in which a 2'-fluoro-2'-deoxyribonucleotide residue and a 2'-O-alkyl ribonucleotide residue is alternately disposed.
(3-7) The oligonucleotide, or a pharmaceutically acceptable salt thereof, according to any of (3-1) to (3-6), wherein at least one of O1 and O4 contains 1, 2, or 3 bridged nucleotide residues.
(3-8) The oligonucleotide, or a pharmaceutically acceptable salt thereof, according to (3-7), wherein the bridged nucleotide residues are diposed so as not to be adjacent to each other.
(3-9) The oligonucleotide, or a pharmaceutically acceptable salt thereof, according to any of (3-1) to (3-8), wherein at least one of O1 and O4 contains a sequence in which a bridged nucleotide residue and a 2'-O-alkyl ribonucleotide residue are alternately disposed.
(3-10) The oligonucleotide, or a pharmaceutically acceptable salt thereof, according to any of (3-1) to (3-9), wherein the bridged nucleotide residue is a 2'-O,4'-C-methylene-bridged nucleotide (LNA) residue or a 2'-O,4'-C-ethylene-bridged nucleotide (ENA) residue in D-ribofuranose.
(3-11) The oligonucleotide, or a pharmaceutically acceptable salt thereof, according to any of (3-1) to (3-10), wherein N2 has cytosine as the base, and is a ribonucleotide residue, a 2'-O-alkyl ribonucleotide residue or a 2'-deoxyribonucleotide residue.
(3-12) The oligonucleotide, or a pharmaceutically acceptable salt thereof, according to any of (3-1) to (3-11), wherein all bonds between nucleotide residues are phosphorothioate bonds.
(3-13) The oligonucleotide, or a pharmaceutically acceptable salt thereof, according to any of (3-1) to (3-12), containing a base sequence of SEQ ID NO: 51, 54, 61, 62 or 63 (wherein U in the base sequence is optionally replaced with T).
(3-14) The oligonucleotide, or a pharmaceutically acceptable salt thereof, according to (3-1), represented by any of the following formulae: 
U(M)^T(L)^G(M)^A(L)^U(M)^G(F)^A(F)^C(M)^C(M)^c^i^G(M)^U(F)^G(
M)^G(F)^C(M)^A(F)^U(M)^C(F)^A(M)^G(L)^U(M)^T(L)^G(M) (AD1_ASS1.39)
 
A(M)^A(L)^G(M)^A(L)^A(M)^A(F)^U(F)^U(M)^C(M)^c^a^A(M)^C(F)^A
(M)^A(F)^A(M)^U(F)^A(M)^C(F)^C(M)^A(L)^C(M)^C(L)^U(M)
(AD1_PANK2.39)
 
A(M)^T(L)^G(M)^T(L)^C(M)^C(F)^A(F)^G(M)^U(M)^c^i^G(M)^A(F)^A(
M)^U(F)^A(M)^U(F)^A(M)^A(F)^U(M)^T(L)^A(M)^C(L)^U(M)
(AD1_NPHS2.39)
 
G(M)^C(L)^A(M)^T(L)^C(M)^C(F)^U(F)^G(M)^C(M)^c^i^C(M)^A(F)^U(
M)^A(F)^A(M)^A(F)^G(M)^G(F)^C(M)^A(L)^C(M)^C(L)^C(M)
(AD1_GRIA2.39)
 
U(M)^G(L)^A(M)^U(L)^G(F)^A(F)^C(M)^C(M)^c^i^G(M)^U(F)^G(M)^G(
F)^C(M)^A(F)^U(M)^C(F)^A(L)^G(M)^U(L)^U(M) (AD1_ASS1.52)
 
G(M)^C(E)^A(M)^T(E)^C(M)^C(F)^U(F)^G(M)^C(M)^c^i^C(M)^A(F)^U(
M)^A(F)^A(M)^A(F)^G(M)^G(F)^C(M)^A(E)^C(M)^C(E)^C(M)
(AD1_GRIA2.39e)
 
U(M)^T(E)^G(M)^A(E)^U(M)^G(F)^A(F)^C(M)^C(M)^c^i^G(M)^U(F)^G(
M)^G(F)^C(M)^A(F)^U(M)^C(F)^A(M)^G(E)^U(M)^T(E)^G(M)
(AD1_ASS1.39e)
 
A(M)^A(E)^G(M)^A(E)^A(M)^A(F)^U(F)^U(M)^C(M)^c^a^A(M)^C(F)^A
(M)^A(F)^A(M)^U(F)^A(M)^C(F)^C(M)^A(E)^C(M)^C(E)^U(M)
(AD1_PANK2.39e)
 
A(M)^T(E)^G(M)^T(E)^C(M)^C(F)^A(F)^G(M)^U(M)^c^i^G(M)^A(F)^A(
M)^U(F)^A(M)^U(F)^A(M)^A(F)^U(M)^T(E)^A(M)^C(E)^U(M)
(AD1_NPHS2.39e)
 
C(M)^A(E)^U(M)^C(E)^C(F)^U(F)^G(M)^C(M)^c^i^C(M)^A(F)^U(M)^A(
F)^A(M)^A(F)^G(M)^G(F)^C(E)^A(M)^C(E)^C(M)) (AD1_GRIA2.52e)
 
U(M)^G(E)^A(M)^T(E)^G(F)^A(F)^C(M)^C(M)^c^i^G(M)^U(F)^G(M)^G(
F)^C(M)^A(F)^U(M)^C(F)^A(E)^G(M)^T(E)^U(M) (AD1_ASS1.52e)
 
A(M)^G(E)^A(M)^A(E)^A(F)^U(F)^U(M)^C(M)^c^a^A(M)^C(F)^A(M)^A
(F)^A(M)^U(F)^A(M)^C(F)^C(E)^A(M)^C(E)^C(M) (AD1_PANK2.52e)
 
U(M)^G(E)^U(M)^C(E)^C(F)^A(F)^G(M)^U(M)^c^i^G(M)^A(F)^A(M)^U(
F)^A(M)^U(F)^A(M)^A(F)^T(E)^U(M)^A(E)^C(M) (AD1_NPHS2.52e)
 
G(M)^T(L)^C(M)^C(L)^C(M)^U(F)^U(F)^C(M)^U(M)^c^i^U(M)^C(F)^G(
M)^A(F)^U(M)^G(F)^G(M)^U(F)^C(M)^A(L)^G(M)^C(L)^A(M) (AD1_A1AT.39)
 wherein a capital letter denotes a ribonucleotide residue, a small letter represents a 2'-deoxyribonucleotide residue, N(M) represents a 2'-O-methyl- ribonucleotide residue, N(F) represents a 2'-fluoro-2'-deoxyribonucleotide residue, N(L) represents a 2'-O,4'-C-methylenated ribonucleotide residue, N(E) represents a 2'-O,4'-C-ethylenated ribonucleotide residue, and "^" represents that nucleoside units are linked by -P(=S)(OH)-.
(3-15) The oligonucleotide, or a pharmaceutically acceptable salt thereof, according to any of (3-1) to (3-14), wherein a delivery molecule containing GalNAc, cholesterol, and a fatty acid is bound via a linker or a phosphodiester bond (including a phosphorothioate bond) at the 5'-end or the 3'-end of the oligonucleotide and the pharmaceutically acceptable salt thereof.
(3-16) A pharmaceutical composition comprising the oligonucleotide, or a pharmaceutically acceptable salt thereof, according to any of (3-1) to (3-15), as an active component.
(3-17) A single-base editing inducer comprising the oligonucleotide, or a pharmaceutically acceptable salt thereof, according to any of (3-1) to (3-15), as an active component.
(3-18) The pharmaceutical composition according to (3-16), for the prevention or therapy of a disease preventable or curable by conversion of an adenosine residue into an inosine residue in the target RNA.
(3-19) The pharmaceutical composition according to (3-18), wherein the disease is a hereditary disease.
(3-20) The pharmaceutical composition according to (3-19), wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.
(3-21) A pharmaceutical composition comprising the oligonucleotide, or a pharmaceutically acceptable salt thereof, according to (3-13) or (3-14), as an active component.
(3-22) A single-base editing inducer comprising the oligonucleotide, or a pharmaceutically acceptable salt thereof, according to (3-13) or (3-14), as an active component.
(3-23) The pharmaceutical composition according to (3-21), for the prevention or therapy of a disease preventable or curable by conversion of an adenosine residue into an inosine residue in the target RNA.
(3-24) The pharmaceutical composition according to (3-23), wherein the disease is a hereditary disease.
(3-21) A pharmaceutical composition comprising the oligonucleotide, or a pharmaceutically acceptable salt thereof, according to (3-13) or (3-14), as an active component.
(3-22) A single-base editing inducer comprising the oligonucleotide, or a pharmaceutically acceptable salt thereof, according to (3-13) or (3-14), as an active component.
(3-23) The pharmaceutical composition according to (3-21), for the prevention or therapy of a disease preventable or curable by conversion of an adenosine residue into an inosine residue in the target RNA.
(3-24) The pharmaceutical composition according to (3-23), wherein the disease is a hereditary disease.
(4-1) The oligonucleotide, or a pharmaceutically acceptable salt thereof, according to any of (3-1) to (3-15), wherein the target RNA is mRNA of an ASS1 gene, a target to be edited is the 1524-th nucleotide of the ASS1 mRNA, and a nucleotide sequence excluding any non-complementary nucleotide residue and N3 is completely complementary to a region of consecutive 20 to 40 bases contained in the 1500 to 1540-th of the ASS1 mRNA.
(4-2) The oligonucleotide, or a pharmaceutically acceptable salt thereof, according to any of (3-1) to (3-15), wherein the target RNA is mRNA of a F5 gene, a target to be edited is the 1696-th nucleotide of the F5 mRNA, and a nucleotide sequence excluding any non-complementary nucleotide residue and N3 is completely complementary to a region of consecutive 20 to 40 bases contained in 1672-th to 1712-th of the mRNA.
(4-3) The oligonucleotide, or a pharmaceutically acceptable salt thereof, according to any of (3-1) to (3-15), wherein the target RNA is mRNA of a GRIA2 gene, a target to be edited is the 2135-th nucleotide of the GRIA2 mRNA, and a nucleotide sequence excluding any non-complementary nucleotide residue and N3 is completely complementary to a region of consecutive 20 to 40 bases contained in the 2111-th to 2151-th of the GRIA2 mRNA.
(4-4) The oligonucleotide, or a pharmaceutically acceptable salt thereof, according to any of (3-1) to (3-15), wherein the target RNA is mRNA of a PANK2 gene, a target to be edited is the 1728-th nucleotide of the PANK2 mRNA, and a nucleotide sequence excluding any non-complementary nucleotide residue and N3 is completely complementary to a region of consecutive 20 to 40 bases contained in the 1704-th to 1744-th of the PANK2 mRNA.
(4-5) The oligonucleotide, or a pharmaceutically acceptable salt thereof, according to any of (3-1) to (3-15), wherein the target RNA is mRNA of a CBS gene, a target to be edited is the 1575-th nucleotide of the CBS mRNA, and a nucleotide sequence excluding any non-complementary nucleotide residue and N3 is completely complementary to a region of consecutive 20 to 40 bases contained in the 1551-th to 1591-th of the CBS mRNA.
(4-6) The oligonucleotide, or a pharmaceutically acceptable salt thereof, according to any of (3-1) to (3-15), wherein the target RNA is mRNA of an NPHS2 gene, a target to be edited is the 497-th nucleotide of the NPHS2 mRNA, and a nucleotide sequence excluding any non-complementary nucleotide residue and N3 is completely complementary to a region of consecutive 20 to 40 bases contained in the 473-th to 513-th of the NPHS2 mRNA.
(4-7) The oligonucleotide, or a pharmaceutically acceptable salt thereof, according to any of (3-1) to (3-15), wherein the target RNA is mRNA of a SERPINA1 gene, a target to be edited is the 1143-th nucleotide of the SERPINA1 mRNA, and a nucleotide sequence excluding any non-complementary nucleotide residue and N3 is completely complementary to a region of consecutive 20 to 40 bases contained in the 1119-th to 1159-th of the SERPINA1 mRNA.
(4-8) The oligonucleotide, or a pharmaceutically acceptable salt thereof, according to any of (3-1) to (3-15), wherein the target RNA is mRNA of a PAH gene, a target to be edited is the 896-th nucleotide of the PAH mRNA, and a nucleotide sequence excluding any non-complementary nucleotide residue and N3 is completely complementary to a region of consecutive 20 to 40 bases contained in the 872-th to 912-th of the PAH mRNA.
(4-9) The oligonucleotide, or a pharmaceutically acceptable salt thereof, according to any of (3-1) to (3-15), wherein the target RNA is mRNA of a SLCO2A1 gene, a target to be edited is the 81034-th nucleotide of the SLCO2A1 pre-mRNA, and a nucleotide sequence excluding any non-complementary nucleotide residue and N3 is completely complementary to a region of consecutive 20 to 40 bases contained in the 81010-th to 81050-th of the SLCO2A1 pre-mRNA.
(4-10) The oligonucleotide, or a pharmaceutically acceptable salt thereof, according to any of (3-1) to (3-15), wherein the target RNA is mRNA of a GFAP gene, a target to be edited is the 2168-th nucleotide of the GFAP mRNA, and a nucleotide sequence excluding any non-complementary nucleotide residue and N3 is completely complementary to a region of consecutive 20 to 40 bases contained in the 2144-th to 2184-th of the GFAP mRNA.
(4-11) The oligonucleotide, or a pharmaceutically acceptable salt thereof, according to any of (3-1) to (3-15), wherein the target RNA is mRNA of an AGXT gene, a target to be edited is the 550-th nucleotide of the AGXT mRNA, and a nucleotide sequence excluding any non-complementary nucleotide residue and N3 is completely complementary to a region of consecutive 20 to 40 bases contained in the 526-th to 566-th of the AGXT mRNA.
(4-12) The oligonucleotide, or a pharmaceutically acceptable salt thereof, according to any of (3-1) to (3-15), wherein the target RNA is mRNA of a UGT1A1 gene, a target to be edited is the 229-th nucleotide of the UGT1A1 mRNA, and a nucleotide sequence excluding any non-complementary nucleotide residue and N3 is completely complementary to a region of consecutive 20 to 40 bases contained in the 205-th to 245-th of the UGT1A1 mRNA.
(4-13) The oligonucleotide, or a pharmaceutically acceptable salt thereof, according to any of (3-1) to (3-15), wherein the target RNA is mRNA of an EYS gene, a target to be edited is the 8478-th nucleotide of the EYS mRNA, and a nucleotide sequence excluding any non-complementary nucleotide residue and N3 is completely complementary to a region of consecutive 20 to 40 bases contained in the 8454-th to 8494-th of the EYS mRNA.
(4-14) The oligonucleotide, or a pharmaceutically acceptable salt thereof, according to any of (3-1) to (3-15), wherein the target RNA is mRNA of a GNE gene, a target to be edited is the 924-th nucleotide of the GNE mRNA, and a nucleotide sequence excluding any non-complementary nucleotide residue and N3 is completely complementary to a region of consecutive 20 to 40 bases contained in the 900-th to 940-th of the GNE mRNA.
(4-15) The oligonucleotide, or a pharmaceutically acceptable salt thereof, according to any of (3-1) to (3-15), wherein the target RNA is mRNA of an HFE gene, a target to be edited is the 1005-th nucleotide of the HFE mRNA, and a nucleotide sequence excluding any non-complementary nucleotide residue and N3 is completely complementary to a region of consecutive 20 to 40 bases contained in the 981-th to 1021-th of the HFE mRNA.

### Advantageous Efftect of Invantion

According to one embodiment of the present invention, an oligonucleotide that may induce the editing activity of ADAR1 in cells and that is excellent in stability *in vivo* may be provided.

### Brief Description of Drawings

Fig. 1A is a graph showing the editing rate of a model target RNA with oligonucleotides in cells.
Fig. 1B is a graph showing the change in emission intensity due to RNA editing of oligonucleotides in cells.
Fig. 2A is a graph showing the editing rate of a model target RNA with oligonucleotides in cells.
Fig. 2B is a graph showing the change in emission intensity due to RNA editing of oligonucleotides in cells.
Fig. 3 is a graph showing the change in emission intensity due to RNA editing of oligonucleotides derived from endogenous ADAR in cells.
Fig. 4A is a graph showing the editing rate of hGAPDH with oligonucleotides in cells.
Fig. 4B is a graph showing the editing rate of hGAPDH with oligonucleotides in cells.
Fig. 5 is a graph showing the editing rate of hGAPDH with oligonucleotides in cells.
Fig. 6 is a graph showing the editing rate of hGAPDH with oligonucleotides in cells.
Fig. 7A is a graph showing the editing rate of hGAPDH with oligonucleotides in cells.
Fig. 7B is a graph showing the editing rate of hGAPDH with oligonucleotides derived from endogenous ADAR in cells.
Fig. 8 is a graph showing the editing rate of a model target RNA having a sequence derived from a disease, with oligonucleotides.
Fig. 9 is a graph showing the editing rate of a model target RNA having a sequence derived from a disease, with oligonucleotides in cells.
Fig. 10 is a graph showing the editing rate of hGAPDH with oligonucleotides in cells.
Fig. 11 is a graph showing the editing rate of hGAPDH with oligonucleotides in cells.
Fig. 12A is a graph showing the editing rate of hGAPDH with oligonucleotides in cells.
Fig. 12B is a graph showing the editing rate of hGAPDH with oligonucleotides in cells.
Fig. 13A is a graph showing the editing rate of hGAPDH with oligonucleotides in cells.
Fig. 13B is a graph showing the editing rate of hGAPDH with oligonucleotides in cells.
Fig. 14A is a graph showing the editing rate in the presence of ADAR1, of a model target RNA having a sequence derived from a disease, with oligonucleotides in cells.
Fig. 14B is a graph showing the editing rate in the presence of ADAR2, of a model target RNA having a sequence derived from a disease, with oligonucleotides in cells.
Fig. 15A is a graph showing the editing rate of a model target RNA having a sequence derived from a disease, with oligonucleotides in cells.
Fig. 15B is a graph showing the editing rate of a model target RNA having an ASS1 gene sequence being a sequence derived from a disease, with oligonucleotides in cells.
Fig. 15C is a graph showing the editing rate of a model target RNA having a PANK2 gene sequence being a sequence derived from a disease, with oligonucleotides in cells.
Fig. 15D is a graph showing the editing rate of a model target RNA having an NPHS2 gene sequence being a sequence derived from a disease, with oligonucleotides in cells.
Fig. 15E is a graph showing the editing rate of a model target RNA having a GRIA2 gene sequence being a sequence derived from a disease, with oligonucleotides in cells.
Fig. 16 is a graph showing the editing rate of a model target RNA having a PAH.2 gene sequence being a sequence derived from a disease, with oligonucleotides in cells.
Fig. 17A is a graph showing the editing rate of a model target RNA having an ASS1 gene sequence being a sequence derived from a disease, with oligonucleotides in cells.
Fig. 17B is a graph showing the editing rate of a model target RNA having a PANK2 gene sequence being a sequence derived from a disease, with oligonucleotides in cells.
Fig. 17C is a graph showing the editing rate of a model target RNA having an NPHS2 gene sequence being a sequence derived from a disease, with oligonucleotides in cells.
Fig. 18A is a graph showing the editing rate of a model target RNA having an ASS1 gene sequence being a sequence derived from a disease, with oligonucleotides in cells.
Fig. 18B is a graph showing the editing rate of a model target RNA having a PANK2 gene sequence being a sequence derived from a disease, with oligonucleotides in cells.
Fig. 18C is a graph showing the editing rate of a model target RNA having an NPHS2 gene sequence being a sequence derived from a disease, with oligonucleotides in cells.
Fig. 18D is a graph showing the editing rate of a model target RNA having a GRIA2 gene sequence being a sequence derived from a disease, with oligonucleotides in cells.
Fig. 19 is a graph showing the editing rate of a model target RNA having a guanosine residue at the 5'-side of a target to be edited, with oligonucleotides in cells.
Fig. 20 is a graph showing the editing rate of a model target RNA having an adenosine residue at the 5'-side of a target to be edited, with oligonucleotides in cells.
Fig. 21A is a graph showing the editing rate of a model target RNA having a guanosine residue at the 5'-side of a target to be edited, with oligonucleotides in cells.
Fig. 21B is a graph showing the editing rate of a model target RNA having an adenosine residue at the 5'-side of a target to be edited, with oligonucleotides in cells.
Fig. 22 is a graph showing the editing rate of a model target RNA having a guanosine residue or an adenosine residue at the 5'-side of a target to be edited, with oligonucleotides in cells.
Fig. 23A is a graph showing the editing rate of a model target RNA having an A1AT gene sequence being a sequence derived from a disease, with oligonucleotides in cells.
Fig. 23B is a graph showing the editing rate of a model target RNA having an A1AT gene sequence being a sequence derived from a disease, with oligonucleotides in cells.
Fig. 24 is a graph showing the repair of a model target RNA having an A1AT gene sequence being a sequence derived from a disease, with oligonucleotides in cells.

### Description of Embodiments

The term "step" as used herein comprises not only an independent step but also a step not clearly distinguishable from another step as long as the intended purpose of the step is achieved. If multiple substances correspond to a component in a composition, the content of the component in the composition means the total amount of the multiple substances present in the composition unless otherwise specified. Furthermore, with respect to an upper limit and a lower limit of numerical ranges described herein, the numerical values exemplified as the numerical range can be freely selected and combined. Embodiments of the present invention will now be described in detail. It should be noted that the embodiments described below are exemplifications of oligonucleotides, pharmaceutical compositions, and methods for prevention or treatment of a disease for embodying the technical ideas of the present disclosure, and the present disclosure is not limited to the oligonucleotides, pharmaceutical compositions, and methods for prevention or treatment of a disease described below.

### Target-editing guide RNA

An oligonucleotide inducing site-specific editing for a target RNA (hereinafter, also referred to as "target-editing guide RNA; gRNA") includes a first oligonucleotide identifying a target RNA, and a second oligonucleotide linked to the 5'-side of the first oligonucleotide. The first oligonucleotide may be composed of a target-corresponding nucleotide residue corresponding to an adenosine residue in the target RNA, an oligonucleotide of 10 to 24 residues, linked to the 3'-side of the target-corresponding nucleotide residue and having a base sequence complementary to the target RNA, and an oligonucleotide of 3 to 6 residues, linked to the 5'-side of the target-corresponding nucleotide residue and having a base sequence complementary to the target RNA. The second oligonucleotide may have no nucleotide residue corresponding to a nucleotide residue of the target RNA or have a nucleotide residue which does not form a complementary pair with a nucleotide residue of the target RNA, at the 3'-end thereof. The number of residues in the second oligonucleotide may be 2 to 10, and at least the nucleotide residues at other than the 3'-end may form a double-stranded structure complementary to the target RNA. At least one residue selected from a counter region composed of the target-corresponding nucleotide residue and each one residue at the 3'-side and the 5'-side thereof may be a nucleotide residue other than a natural ribonucleotide residue.

The second oligonucleotide constituting the target-editing guide RNA has a base sequence in which one nucleotide residue of the target RNA does not form a complementary pair and the corresponding nucleotide residues other than such one nucleotide residue form a double-stranded structure complementary to the target RNA. In other words, the second oligonucleotide has a base sequence in which an incomplete double-stranded structure is formed with the target RNA. The target-editing guide RNA, which has a short-chain second oligonucleotide having such a characteristic base sequence, at the 5'-side of the first oligonucleotide identifying the target RNA, may preferentially induce the editing activity of ADAR1 in cells. The reason for this may be considered because, for example, the presence of a nucleotide residue which does not form a complementary pair has an advantage for recruiting ADAR1. The counter region contains a nucleotide residue other than a natural ribonucleotide residue, and thus stability *in vivo* is excellent.

In one embodiment, an oligonucleotide for target-editing includes a first oligonucleotide identifying a target RNA, and a second oligonucleotide linked to the 5'-side of the first oligonucleotide. The first oligonucleotide may be composed of a target-corresponding nucleotide residue corresponding to an adenosine residue in the target RNA, an oligonucleotide of 10 to 24 residues, linked to the 3'-side of the target-corresponding nucleotide residue and having a base sequence complementary to the target RNA, and an oligonucleotide of 3 to 6 residues, linked to the 5'-side of the target-corresponding nucleotide residue and having a base sequence complementary to the target RNA. The second oligonucleotide may have no nucleotide residue corresponding to a nucleotide residue of the target RNA or have a nucleotide residue which does not form a complementary pair with a nucleotide residue of the target RNA, at the 3'-end thereof. The number of residues in the second oligonucleotide may be 2 to 10, and at least the nucleotide residues at other than the 3'-end may form a double-stranded structure complementary to the target RNA. In the oligonucleotide for target-editing, the nucleotide residue linked to the 3'-side of the target-corresponding nucleotide residue may be a 2'-deoxynucleotide residue, and the third nucleotide residue counted in the 3'-direction from the target-corresponding nucleotide in the oligonucleotide linked to the 3'-side of the target-corresponding nucleotide residue may be a 2'-deoxy-2'-fluoronucleotide residue.

The oligonucleotide for target-editing has a 2'-deoxynucleotide residue at the 3'-side of the target-corresponding nucleotide residue and has a 2'-deoxy-2'-fluoronucleotide residue at the third nucleotide residue counted in the 3'-direction from the target-corresponding nucleotide in the oligonucleotide linked to the 3'-side of the target-corresponding nucleotide residue, and thus may be excellent in stability *in vivo* and may more effectively induce the editing activity of ADAR in cells.

In one embodiment, the oligonucleotide for target-editing may be an oligonucleotide, or a pharmaceutically acceptable salt thereof, represented by the following formula (I) and having 20 to 40 residues which contain a phosphorothioate bond between nucleotide residues. The length of the oligonucleotide for target-editing may be preferably 20 to 30 residues, more preferably 21, 22, 23, 24, 25, 26, 27, or 28 residues. All of such bonds between nucleotide residues may be preferably phosphorothioate bonds. The oligonucleotide for target-editing may be used in, for example, gene editing for conversion of the adenosine residue to be edited in a target RNA, into an inosine residue.

5'-O1-O2-N1-N2-N3-N4-N5-O3-O4-3' (I)

In the formula (I), N1 may be a 2'-O-alkyl ribonucleotide residue, a ribonucleotide residue, a 2'-fluoro-2'-deoxyribonucleotide residue or a 2'-deoxyribonucleotide residue, and may be preferably a 2'-O-alkyl ribonucleotide residue, a ribonucleotide residue or a 2'-deoxyribonucleotide residue. N2 may be a ribonucleotide residue, a 2'-O-alkyl ribonucleotide residue or a 2'-deoxyribonucleotide residue, in which the base is cytosine or 3-methyluracil. N3 may be a 2'-deoxyribonucleotide residue. N4 may be a 2'-O-alkyl ribonucleotide residue or a 2'-fluoro-2'-deoxyribonucleotide residue. N5 may be a 2'-fluoro-2'-deoxyribonucleotide residue.

O1 may be absent or an oligonucleotide of 2 to 10 residues. O1 may contain at least one bridged nucleotide residue, in which any other than the bridged nucleotide residue may be a 2'-fluoro-2'-deoxyribonucleotide residue or a 2'-O-alkyl ribonucleotide residue. O2 may be an oligonucleotide of 2 to 10 residues and may be composed of a 2'-fluoro-2'-deoxyribonucleotide residue and a 2'-O-alkyl ribonucleotide residue. O2 may be an oligonucleotide of preferably 3, 4, 5, 6, 7 or 8 residues, more preferably 3 or 4 residues, and may be composed of a 2'-fluoro-2'-deoxyribonucleotide residue and a 2'-O-alkyl ribonucleotide residue. O3 may be an oligonucleotide of 5 to 20 residues, and may be composed of a 2'-fluoro-2'-deoxyribonucleotide residue and a 2'-O-alkyl ribonucleotide residue. O3 may be an oligonucleotide of preferably 5, 6, 7, 8, 9, 10, 11, 12, 13, or 14 residues, more preferably 6 or 7 residues, and may be composed of a 2'-fluoro-2'-deoxyribonucleotide residue and a 2'-O-alkyl ribonucleotide residue. O4 may absent or an oligonucleotide of 2 to 10 residues, and may be an oligonucleotide of preferably 2, 3, 4, 5, 6, 7, or 8 residues, more preferably 4 or 5 residues. O4 may contain at least one bridged nucleotide residue, in which any other than the bridged nucleotide residue may be a 2'-fluoro-2'-deoxyribonucleotide residue or a 2'-O-alkyl ribonucleotide residue.

At least one of O2 and O3 may contain a sequence in which a 2'-fluoro-2'-deoxyribonucleotide residue and a 2'-O-alkyl ribonucleotide residue are alternately disposed, and is preferably composed of a sequence in which a 2'-fluoro-2'-deoxyribonucleotide residue and a 2'-O-alkyl ribonucleotide residue is alternately disposed. The number of repetitions of linking of a 2'-fluoro-2'-deoxyribonucleotide residue and a 2'-O-alkyl ribonucleotide residue in O2 may be, for example, 1 or more and 3 or less, and may be preferably 1 or 2. The number of repetitions of linking of a 2'-fluoro-2'-deoxyribonucleotide residue and a 2'-O-alkyl ribonucleotide residue in O3 may be, for example, 1 or more and 10 or less, may be preferably 2 or more and 6 or less, and may be more preferably 3 or 4. Such a base sequence in which a 2'-deoxyribonucleotide residue and a 2'-O-alkyl ribonucleotide residue are alternately linked may be such that the alternate linking is in the 3'-direction from the 2'-deoxyribonucleotide residue or may be such that the alternate linking is in the 3'-direction from the 2'-O-alkyl ribonucleotide residue. At least one of O1 and O4 may contain 1, 2, or 3 bridged nucleotide residues, and in this case, these bridged nucleotide residues may be disposed so as not to be adjacent to each other. At least one of O1 and O4 may contain a sequence in which a bridged nucleotide residue and a 2'-O-alkyl ribonucleotide residue are alternately disposed. The number of repetitions of linking of a bridged nucleotide residue and a 2'-O-alkyl ribonucleotide residue, in O1 and O4, may be for example, 1 or more and 3 or less, and may be preferably 2. Such a base sequence in which a 2'-O-alkyl ribonucleotide residue and a bridged nucleotide residue are alternately linked may be such that the alternate linking is in the 3'-direction from the 2'-O-alkyl ribonucleotide residue or may be such that the alternate linking is in the 3'-direction from the bridged nucleotide residue.

N1 and N4 may be preferably 2'-O-alkyl ribonucleotide residues. N2 may preferably have cytosine as the base, and may be a ribonucleotide residue, a 2'-O-alkyl ribonucleotide residue, or a 2'-deoxyribonucleotide residue.

In the oligonucleotide for target-editing, N2 may correspond to the adenosine residue to be edited, and O2 may have no nucleotide residue corresponding to a corresponding nucleotide residue of the target RNA or have a nucleotide residue which does not form a complementary pair with the corresponding nucleotide residue of the target RNA in the second to fifth regions from the 3'-side.

In the base sequence of the oligonucleotide for target-editing, the base of N3 is optionally a base complementary or non-complementary to the base adjacent to the 5'-side of the adenosine residue to be edited. The bases of N1, N4, N5, O1, O3, and O4 may be completely complementary to the bases of the corresponding nucleotide residues of the target RNA. The base of O2, other than an absent nucleotide residue and a nucleotide residue which does not form a complementary pair with the corresponding nucleotide residue of the target RNA in O2, may be completely complementary to the base of the corresponding nucleotide residue of the target RNA.

In the oligonucleotide for target-editing represented by the formula (I), when the nucleotide residue at the 5'-side of the adenosine residue to be edited, and N3 form a complementary pair, N3 may be a 2'-deoxyadenosine residue, a 2'-deoxyinosine residue (provided that a case is excluded in which the nucleotide residue at the 5'-side of the adenosine residue to be edited is an adenosine residue or a uridine residue), a thymidine residue or a 2'-deoxyuridine residue (provided that a case is excluded in which the nucleotide residue at the 5'-side of the adenosine residue to be edited is a guanosine residue). When the nucleotide residue at the 5'-side of the adenosine residue to be edited and N3 do not form a complementary pair, N3 may be a 2'-deoxyinosine residue.

The oligonucleotide for target-editing has the structure represented by the formula (I), and thus may be excellent in stability *in vivo* and may more effectively induce the editing activity of ADAR in cells.

The target-editing guide RNA of the present embodiment is simply reduced in production cost because of having a short length as compared with a conventional target-editing guide RNA. The target-editing guide RNA is easily chemically synthesized, and a modified nucleotide such as an existing artificial nucleic acid for use in the development or the like of nucleic acid medicines is easily applied to the target-editing guide RNA. Thus, a more highly functional target-editing guide RNA may be provided by an enhancement in degradation resistance in cells, an enhancement in cell introduction efficiency, and the like. Furthermore, the target-editing guide RNA is constituted by the minimum number of residues necessary for editing induction, and thus not only specificity to the target RNA is kept, but also offtarget editing at a position other than that of the adenosine residue to be edited may be suppressed.

The target-editing guide RNA induces site-specific editing for the target RNA by, for example, preferentially recruiting ADAR1, among ADAR catalyzing target editing, to the target RNA. ADAR is an enzyme converting an adenosine residue in double-stranded RNA into an inosine residue by hydrolytic deamination reaction, and is widely present in mammalian cells. The inosine residue is similar in structure to the guanosine residue, and thus is translated as the guanosine residue in translation of RNA information, and as a result, the RNA information is edited. When such RNA editing is generated in a portion encoding an amino acid, amino acid substitution or the like is generated and the function of a target gene may be controlled, even though no DNA mutation exists on genome.

The target-editing guide RNA, when introduced into mammalian cells, may preferentially recruit ADAR1 present in such cells, to the target RNA, and induce site-specific editing for the target RNA.

The target-editing guide RNA of the present embodiment may induce the editing activity in cells in an ADAR1-preferential manner. The ADAR1-preferential manner means that, for example, the ratio of the editing induction activity to ADAR1 relative to the editing induction activity to ADAR2 *in vitro* is more than 1, preferably 1.5 or more, 2 or more, or 5 or more. It is noted with respect to the editing induction activities to ADAR1 and ADAR2 in cells that such a preferential manner refers to a case where, when an expression is made by transfection under the same conditions in cells by use of an ADAR1 expression plasmid and an ADAR2 expression plasmid constructed with the same expression vector, ADAR1 is higher in editing induction efficiency to the same site to be edited.

The first oligonucleotide contained in the target-editing guide RNA identifies the target RNA. The target RNA is not particularly limited as long as it contains the adenosine residue to be edited, may be either cellular RNA or viral RNA, and is usually an mRNA precursor (containing pre-mRNA) or mRNA encoding a protein. An editing site in the target RNA may be present in an untranslated region, a splice region, exon, intron, or any region affecting the stability, structure or function of RNA. The target RNA may also contain mutation to be modified or altered. Alternatively, the target RNA may have a sequence that is mutated so as to encode a phenotype different from the natural form.

The target RNA is preferably an RNA encoding a protein. Specific examples of the protein to be encoded may include a serotonin receptor, a glutaminate receptor, a membrane potential-dependent potassium channel, phosphorylated proteins in signaling, such as STAT3, NFkBIA, and MAPK14.

The target-editing guide RNA may be applied, for example, to treatment of hereditary disease. The hereditary disease may be, for example, a disease curable by conversion of an adenosine residue into an inosine residue in the target RNA. The hereditary disease may be, for example, a hereditary disease caused by mutation of a guanosine residue to an adenosine residue in a gene. Examples of the hereditary disease include mucoviscidosis, albinism, α-1 anti-trypsin deficiency, Alzheimer's disease, amyotrophic lateral sclerosis, asthma, β-thalassemia, CADASIL syndrome, Charcot Marie Tooth disease, chronic obstructive pulmonary disease (COPD), distal spinal muscular atrophy (DSMA), Duchenne/Becker muscular dystrophy, dystrophic epidermolysis bullosa, epidermolysis bullosa, Fabry's disease, Factor V Leiden-related disorder, familial adenoma, polyposis, galactosemia, Gaucher's disease, glucose-6-phosphate dehydrogenase deficiency, hemophilia, hereditary hemochromatosis, Hunter's syndrome, Huntington's disease, Hurler's syndrome, inflammatory bowel disease (IBD), hereditary polyagglutination syndrome, Leber's congenital amaurosis, Lesch-Nyan syndrome, Lynch syndrome, Marfan syndrome, mucopolysaccharidosis, muscular dystrophy, myotonic dystrophy I and II, neurofibromatosis, Niemann-Pick disease Type A, B and C, NY-esol-related pancreatic cancer, Parkinson's disease, Peutz-Jeghers syndrome, phenyl ketonuria, Pompe's disease, primary ciliary disease, prothrombin mutation-related diseases such as prothrombin G20210A mutation, pulmonary hypertension, retinitis pigmentosa, Sandhoff's disease, severe combined immunodeficiency syndrome (SCID), sickle cell anemia, spinal muscular atrophy, Stargardt's disease, Tay-Sachs disease, Usher's syndrome, X-linked immunodeficiency, and various forms of cancer (for example, BRCA1- and 2-related breast cancers and ovarian cancers).

Specific examples of the hereditary disease may include type I citrullinemia (ASS1), type II citrullinemia (SLC25A13), hemophilia (Factor V Leiden), primary hyperoxaluria (AGXT), distal myopathy (GNE), mucoviscidosis: cystic fibrosis (CFCFTR), homocystinuria (CBS), Hurler syndrome: mucopolysaccharidosis (IDUA (SLC26A1)), Alexander disease (GFAP), retinitis pigmentosa (EYS), phenyl ketonuria (PAH), hemochromatosis (HFE), and Gilbert syndrome (UGT1A1). Those in parentheses are the target gene names.

The target-editing guide RNA applied to treatment of the hereditary disease may contain, for example, any base sequence selected from the group consisting of SEQ ID NOs: 49 to 55 and SEQ ID NOs: 60 to 63, and may preferably contain any base sequence selected from the group consisting of SEQ ID NOs: 51, 54, 61, 62, and 63. Herein, the target-editing guide RNA may contain a base sequence in which U is substituted with T in a base sequence identified by SEQ ID NO.

c.1168G>A mutation (rs121908641) of an ASS1 gene causing type I citrullinemia is a mutation from G to A in the 1524-th nucleotide of Homo sapiens argininosuccinate synthase 1 (ASS1), transcript variant 1, mRNA (GenBank Accession No:NM_000050.4). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 1500 to 1540-th of the ASS1 mRNA, and may be preferably completely complementary to a region of consecutive 20 to 29 bases contained in the 1504-th to 1534-th of the mRNA, more preferably a region of consecutive 20 to 25 bases contained in the 1510-th to 1534-th of the mRNA.

c.1601G>A mutation (rs6025) of a Factor V gene causing hemophilia (thrombosis) is a mutation from G to A in the 1696-th nucleotide of Homo sapiens coagulation factor V (F5), mRNA (GenBank Accession No:NM_000130.5). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 1672-th to 1712-th of the F5 mRNA, and may be preferably completely complementary to a region of consecutive 20 to 29 bases contained in the 1676-th to 1706-th of the mRNA, more preferably a region of consecutive 20 to 25 bases contained in the 1682-th to 1706-th of the mRNA.

The abnormality of posttranscriptional modification of a GRIA2 gene may cause amyotrophic lateral sclerosis (ALS). The abnormality of the posttranscriptional modification may correspond to the abnormality of A-to-I RNA editing in adenosine at the 2135-th of Homo sapiens glutamate ionotropic receptor AMPA type subunit 2 (GRIA2), transcript variant 1, mRNA (GenBank Accession No: nm_000826.4). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 2111-th to 2151-th of the GRIA2 mRNA, and may be preferably completely complementary to a region of consecutive 20 to 29 bases contained in the 2115-th to 2145-th of the mRNA, more preferably a region of consecutive 20 to 25 bases contained in the 2121-th to 2145-th of the mRNA.

c.1561G>A mutation (rs137852959) of a PANK2 gene causing a pantothenic acid-related neurodegenerative disease is a mutation from G to A on Homo sapiens pantothenate kinase 2 (PANK2), transcript variant 1, mRNA (GenBank Accession No: NM_153638.3). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 1704-th to 1744-th of the PANK2 mRNA, and may be preferably completely complementary to a region of consecutive 20 to 29 bases contained in the 1708-th to 1738-th of the mRNA, more preferably a region of consecutive 20 to 25 bases contained in the 1714-th to 1738-th of the mRNA.

c.1330G>A mutation (rs28934891) of a CBS gene causing homocystinuria is a mutation from G to A in the 1575-th nucleotide of Homo sapiens cystathionine beta-synthase (CBS), transcript variant 1, mRNA (GenBank Accession No: NM_000071.2). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 1551-th to 1591-th of the CBS mRNA, and may be preferably completely complementary to a region of consecutive 20 to 29 bases contained in the 1555-th to 1585-th of the mRNA, more preferably a region of consecutive 20 to 25 bases contained in the 1561-th to 1585-th of the mRNA.

c.413G>A mutation (rs74315342) of an NPHS2 gene causing focal segmental glomerulosclerosis is a mutation from G to A in the 497-th nucleotide of Homo sapiens NPHS2 stomatin family member, podocin (NPHS2), transcript variant 1, mRNA (GenBank Accession No: NM 014625.4). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 473-th to 513-th of the NPHS2 mRNA, and may be preferably completely complementary to a region of consecutive 20 to 29 bases contained in the 477-th to 507-th of the mRNA, more preferably a region of consecutive 20 to 25 bases contained in the 483-th to 507-th of the mRNA.

c.1096G>A mutation (rs28929474) of a SERPINA1 gene causing α1 anti-trypsin deficiency is a mutation from G to A in the 1143-th nucleotide of Homo sapiens serpin family A member 1 (SERPINA1), transcript variant 1, mRNA (GenBank Accession No: NM_000295.5). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 1119-th to 1159-th of the SERPINA1 mRNA, and may be preferably completely complementary to a region of consecutive 20 to 29 bases contained in the 1123-th to 1153-th of the mRNA, more preferably a region of consecutive 20 to 25 bases contained in the 1129-th to 1153-th of the mRNA.

c.782G>A mutation (rs5030849) of a PAH gene causing phenyl ketonuria is a mutation from G to A in the 896-th nucleotide of Homo sapiens phenylalanine hydroxylase (PAH), transcript variant 1, mRNA (GenBank Accession No: NM_000277.3). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 872-th to 912-th of the PAH mRNA, and may be preferably completely complementary to a region of consecutive 20 to 29 bases contained in the 876-th to 906-th of the mRNA, more preferably a region of consecutive 20 to 25 bases contained in the 882-th to 906-th of the mRNA.

c.940+1G>A mutation (rs765249238) of a SLCO2A1 gene causing pachydermoperiostosis is a mutation from G to A in the 81034-th nucleotide on pre-mRNA of Homo sapiens solute carrier organic anion transporter family member 2A1 (SLCO2A1), mRNA (GenBank Accession No: NM_005630.3) (the target to be edited is located on the 133948892-th (-) strand of chromosome 3). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 81010-th to 81050-th of the SLCO2A1 pre-mRNA, and may be preferably completely complementary to a region of consecutive 20 to 29 bases contained in the 81014-th to 81044-th of the mRNA, more preferably a region of consecutive 20 to 25 bases contained in the 81020-th to 81044-th of the mRNA.

c.716G>A mutation (rs59565950) of a GFAP gene causing Alexander disease is a mutation from G to A in the 2168-th nucleotide of Homo sapiens glial fibrillary acidic protein (GFAP), transcript variant 1, mRNA (GenBank Accession No: NM_002055.5). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 2144-th to 2184-th of the GFAP mRNA, and may be preferably completely complementary to a region of consecutive 20 to 29 bases contained in the 2148-th to 2178-th of the mRNA, more preferably a region of consecutive 20 to 25 bases contained in the 2154-th to 2178-th of the mRNA.

c.508G>A mutation (rs121908529) of an AGXT gene causing primary hyperoxaluria is a mutation from G to A in the 550-th nucleotide of Homo sapiens alanine--glyoxylate and serine--pyruvate aminotransferase (AGXT), mRNA (GenBank Accession No: NM_000030.3). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 526-th to 566-th of the AGXT mRNA, and may be preferably completely complementary to a region of consecutive 20 to 29 bases contained in the 530-th to 560-th of the mRNA, more preferably a region of consecutive 20 to 25 bases contained in the 536-th to 560-th of the mRNA.

c.211G>A mutation (rs4148323) of a UGT1A1 gene causing Gilbert syndrome is a mutation from G to A in the 229-th nucleotide of Homo sapiens UDP glucuronosyltransferase family 1 member A1 (UGT1A1), mRNA (GenBank Accession No: NM_000463.3). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 205-th to 245-th of the UGT1A1 mRNA, and may be preferably completely complementary to a region of consecutive 20 to 29 bases contained in the 209-th to 239-th of the mRNA, more preferably a region of consecutive 20 to 25 bases contained in the 215-th to 239-th of the mRNA.

c.7919G>A mutation (rs527236066) of an EYS gene causing retinitis pigmentosa is a mutation from G to A in the 8478-th nucleotide of Homo sapiens eyes shut homolog (EYS), transcript variant 1, mRNA (GenBank Accession No: NM_001142800.2). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 8454-th to 8494-th of the EYS mRNA, and may be preferably completely complementary to a region of consecutive 20 to 29 bases contained in the 8458-th to 8488-th of the mRNA, more preferably a region of consecutive 20 to 25 bases contained in the 8464-th to 8488-th of the mRNA.

c.797G>A mutation (rs121908622) of a GNE gene causing distal myopathy is a mutation from G to A in the 924-th nucleotide of Homo sapiens glucosamine (UDP-A-acetyl)-2-epimerase/7V-acetylmannosamine kinase (GNE), transcript variant 1, mRNA (GenBank Accession No: NM_001128227.3). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 900-th to 940-th of the GNE mRNA, and may be preferably completely complementary to a region of consecutive 20 to 29 bases contained in the 904-th to 934-th of the mRNA, more preferably a region of consecutive 20 to 25 bases contained in the 910-th to 934-th of the mRNA.

c.845G>A mutation (rs1800562) of a HFE gene causing hemochromatosis is a mutation from G to A in the 1005-th nucleotide of Homo sapiens homeostatic iron regulator (HFE), transcript variant 1, mRNA (GenBank Accession No: NM_000410.3). In one embodiment, the oligonucleotide targets the adenosine residue. For example, in one embodiment, in the oligonucleotide, a nucleotide sequence excluding any non-complementary nucleotide residue and N3 may be completely complementary to a region of consecutive 20 to 40 bases contained in the 981-th to 1021-th of the HFE mRNA, and may be preferably completely complementary to a region of consecutive 20 to 29 bases contained in the 985-th to 1015-th of the mRNA, more preferably a region of consecutive 20 to 25 bases contained in the 991-th to 1015-th of the mRNA.

The first oligonucleotide is composed of a target-corresponding nucleotide residue corresponding to the adenosine residue to be edited in the target RNA, a 3'-side oligonucleotide of 10 to 24 residues, linked to the 3'-side of the target-corresponding nucleotide residue and having a base sequence complementary to the corresponding base sequence of the target RNA, and a 5'-side oligonucleotide of 3 to 6 residues, linked to the 5'-side of the target-corresponding nucleotide residue and having a base sequence complementary to the corresponding base sequence of the target RNA. The target RNA and the site to be edited in the target RNA are identified by taking the oligonucleotides respectively linked to the 3'-side and the 5'-side of the target-corresponding nucleotide residue, with the target RNA, to form a double-stranded structure and form a complementary strand (hereinafter, also referred to as "first complementary strand") as a whole. The "complementary base sequence" herein encompasses, in addition to a base sequence forming a Watson-click type base pair, for example, a base sequence forming a thermodynamically stable non-Watson-click type wobble base pair such as a G-U base pair. The base pair forming the first complementary strand, excluding the target-corresponding nucleotide residue, may be entirely a Watson-click type base pair, or may be at least partially a wobble base pair. When the first complementary strand contains a wobble base pair, the number of such pairs may be 1 or 2. Hereinafter, the region composed of the target-corresponding nucleotide residue, one residue at the 5'-side thereof, and one residue at the 3'-side thereof is also referred to as "counter region" for convenience, and the region other than the counter region of the first oligonucleotide is also referred to as "non-counter region".

In one embodiment, in the 3'-side oligonucleotide of 10 to 24 residues, linked to the 3'-side of the target-corresponding nucleotide residue in the first oligonucleotide, the first nucleotide residue at the 3'-side of the target-corresponding nucleotide residue is a nucleotide residue which does not form a complementary pair with the corresponding nucleotide residue of the target RNA, and any nucleotide residue other than the first nucleotide residue may form a complementary pair with the corresponding nucleotide residue of the target RNA to form a double-stranded structure with the target RNA. Specifically, for example, when a guanosine residue is linked to the 5'-side of the adenosine residue to be edited in the target RNA, the first nucleotide residue at the 3'-side of the target-corresponding nucleotide residue may be a 2'-deoxyinosine residue.

The target-corresponding nucleotide residue is a nucleotide residue corresponding to the adenosine residue to be edited, for example, a cytidine residue, a guanosine residue, an adenosine residue or a derivative thereof. The target-corresponding nucleotide residue is preferably a base which does not form a base pair with the adenosine residue to be edited, more preferably a cytidine residue or a derivative thereof, further preferably a cytidine residue. In one embodiment, the target-corresponding nucleotide residue may be an N-alkylpyrimidine nucleotide residue, preferably an N-alkylcytidine residue, an N-alkyl-2'-deoxycytidine residue, an N-alkyluridine residue, or an *N-*alkyldeoxyuridine residue. The alkyl group in the N-alkylpyrimidine nucleotide residue may be an alkyl group having 1 to 6 or 1 to 4 carbon atoms, preferably a methyl group or an ethyl group. The target-corresponding nucleotide residue may be the *N*-alkylpyrimidine nucleotide residue to result in an enhancement in editing induction activity. At least one of a sugar moiety and a phosphoester bond moiety in the *N*-alkylpyrimidine nucleotide residue may be further modified. Such modification of a sugar moiety and a phosphoester bond moiety is described below.

In one embodiment, the target-corresponding nucleotide residue may be a 2'-deoxycytidine residue. A phosphoester bond moiety in the 2'-deoxycytidine residue may be further modified.

The number of residues in the oligonucleotide linked to the 3'-side of the target-corresponding nucleotide residue and having a base sequence complementary to the target RNA, in the first oligonucleotide, may be 10 or more and 24 or less, preferably 11 or more, 12 or more, or 13 or more, and is preferably 22 or less, 20 or less, 18 or less, 16 or less or 15 or less. In one embodiment, the number of residues in an oligonucleotide having a base sequence complementary to the target RNA may be 12 or more and 20 or less, 12 or more and 18 or less, 12 or more and 16 or less, 14 or more and 18 or less, or 14 or more and 16 or less. In one embodiment, the number of residues in an oligonucleotide having a base sequence complementary to the target RNA may be 14. When the number of residues is in the above range, the selectivity to ADAR1 tends to be more enhanced.

The 3'-side oligonucleotide linked to the 3'-side of the target-corresponding nucleotide residue in the first oligonucleotide may have a complementary base sequence not containing any mismatched base pair, to the target RNA. In this case, no guanosine residue is preferably linked to the 5'-side of the adenosine residue to be edited in the target RNA. In other words, when the base linked to the 5'-side of the adenosine residue to be edited in the target RNA is an adenosine residue, a cytidine residue or a uridine residue, the oligonucleotide linked to the 3'-side of the target-corresponding nucleotide residue in the first oligonucleotide preferably has a complementary base sequence not containing any mismatched base pair, to the target RNA.

The 3'-side oligonucleotide linked to the 3'-side of the target-corresponding nucleotide residue in the first oligonucleotide may optionally contain a base non-complementary to the base sequence of the target RNA. For example, in the case of a target RNA in which a guanosine residue is linked at the 5'-side of the adenosine residue to be edited, editing induction activity may be deteriorated. Even in this case, the first oligonucleotide, which has a base sequence containing a base non-complementary to the guanosine residue, may thus allow for an enhancement in editing induction activity. The base non-complementary to the guanosine residue may be, for example, a guanosine residue. In other words, a target-editing guide RNA to a target RNA in which a guanosine residue is linked at the 5'-side of the adenosine residue to be edited may be one in which a guanosine residue is linked at the 3'-side of the target-corresponding nucleotide residue.

The 3'-side oligonucleotide linked to the 3'-side of the target-corresponding nucleotide residue in the first oligonucleotide may optionally form a non-Watson-click type wobble base pair with the corresponding nucleotide residue of the target RNA, and the 3'-side oligonucleotide linked to the 3'-side of the target-corresponding nucleotide residue may be optionally a 2'-deoxynucleotide residue. This may result in an enhancement in an editing target activity.

For example, when a cytidine residue is linked to the 5'-side of the adenosine residue to be edited in the target RNA, a nucleotide residue having a hypoxanthine residue as a base may be linked to the 3'-side of the target-corresponding nucleotide residue in the first oligonucleotide. In other words, the cytidine residue linked to the 5'-side of the adenosine residue to be edited and the nucleotide residue having a hypoxanthine residue as a base in the first oligonucleotide may form a wobble base pair. The nucleotide residue having a hypoxanthine residue as a base may be an inosine residue or a 2'-deoxyinosine residue, and is preferably a 2'-deoxyinosine residue. Furthermore, at least one of a sugar moiety and a phosphoester bond moiety in the nucleotide residue having a hypoxanthine residue as a base may be further modified. Such modification of a sugar moiety and a phosphoester bond moiety is described below.

For example, when a uridine residue is linked to the 5'-side of the adenosine residue to be edited in the target RNA, a nucleotide residue having an adenyl group as a base may be linked to the 3'-side of the target-corresponding nucleotide residue in the first oligonucleotide. In other words, the uridine residue linked to the 5'-side of the adenosine residue to be edited and the nucleotide residue having an adenyl group as a base in the first oligonucleotide may form a base pair. The nucleotide residue having an adenyl group as a base may be an adenosine residue or a 2'-deoxyadenosine residue, and is preferably a 2'-deoxyadenosine residue. Furthermore, at least one of a sugar moiety and a phosphoester bond moiety in the nucleotide residue having an adenyl group as a base may be further modified. Such modification of a sugar moiety and a phosphoester bond moiety is described below.

For example, when an adenosine residue is linked to the 5'-side of the adenosine residue to be edited in the target RNA, a nucleotide residue having a pyrimidinyl group as a base may be linked to the 3'-side of the target-corresponding nucleotide residue in the first oligonucleotide. In other words, the adenosine residue linked to the 5'-side of the adenosine residue to be edited and the nucleotide residue having a pyrimidinyl group as a base in the first oligonucleotide may form a base pair. The nucleotide residue having a pyrimidyl group as a base may be a thymidine residue or a 2'-deoxyuridine residue. Furthermore, at least one of a sugar moiety and a phosphoester bond moiety in the nucleotide residue having a pyrimidinyl group as a base may be further modified. Such modification of a sugar moiety and a phosphoester bond moiety is described below.

For example, when a guanosine residue is linked to the 5'-side of the adenosine residue to be edited in the target RNA, a nucleotide residue having a hypoxanthine residue as a base may be linked to the 3'-side of the target-corresponding nucleotide residue in the first oligonucleotide. The hypoxanthine residue may be a 2'-deoxyinosine residue. Furthermore, at least one of a sugar moiety and a phosphoester bond moiety in the hypoxan residue may be further modified. Such modification of a sugar moiety and a phosphoester bond moiety is described below.

The first oligonucleotide may have at least one bridged nucleotide residue in the tenth or later, preferably eleventh or later nucleotide residues counted in the 3'-direction from the target-corresponding nucleotide residue. The bridged nucleotide residue may have a bridge between the 2'-position and the 4'-position of D-ribofuranose. Specific examples of the bridged nucleotide residue are described below. The bridged nucleotide residue may contain at least one of a 2'-O,4'-C-methylene-bridged nucleotide (LNA) and a 2'-O,4'-C-ethylene-bridged nucleotide (ENA). At least one of a base moiety and a phosphoester bond moiety in the bridged nucleotide residue may be further modified. Such modification of a base moiety and a phosphoester bond moiety is described below.

The oligonucleotide composed of the tenth or later, preferably the eleventh or later nucleotide residues counted in the 3'-direction from the target-corresponding nucleotide in the first oligonucleotide may have a base sequence in which a 2'-O-alkyl ribonucleotide residue and a bridged nucleotide residue are alternately linked. The number of repetitions of linking of a 2'-O-alkyl ribonucleotide residue and a bridged nucleotide residue may be, for example, 1 or more and 3 or less, and may be preferably 2. Such a base sequence in which a 2'-O-alkyl ribonucleotide residue and a bridged nucleotide residue are alternately linked may be such that the alternate linking is in the 3'-direction from the 2'-O-alkyl ribonucleotide residue, or may be such that the alternate linking is in the 3'-direction from the bridged nucleotide residue. Specific examples of the 2'-O-alkyl ribonucleotide residue and the bridged nucleotide residue are described below. At least one of a base moiety and a phosphoester bond moiety of the 2'-O-alkyl ribonucleotide residue or the bridged nucleotide residue may be further modified. Such modification of a base moiety and a phosphoester bond moiety is described below.

The number of residues in the 5'-side oligonucleotide linked to the 5'-side of the target-corresponding nucleotide residue and having a base sequence complementary to the target RNA in the first oligonucleotide may be 3 or more and 6 or less, and is preferably 3 or more and 5 or less, 3 or more and 4 or less, or 3. When the number of residues is in the above range, the selectivity to ADAR1 tends to be more enhanced.

The second oligonucleotide is taken with the target RNA to form a double-stranded structure containing one or more nucleotide residues which do not form a complementary base pair (hereinafter, also referred to as "second complementary strand"). The number of residues in the second oligonucleotide may be 2 or more and 10 or less, or 4 or more and 8 or less. The number of residues in the second oligonucleotide is, for example, 3 or more, preferably 4 or more, or 5 or more, and, for example, 10 or less, preferably 9 or less, 8 or less, or 7 or less. In one embodiment, the number of residues in the second oligonucleotide may be 6. When the number of residues is in the above range, editing induction tends to be more promoted. The number of nucleotide residues which do not form a complementary base pair in the second complementary strand is, for example, 3 or less, or 2 or less, preferably 1.

Such a nucleotide residue which does not form a complementary base pair in the second complementary strand may be a nucleotide residue which is present in one of the target RNA and the second oligonucleotide without being formed into a base pair due to no nucleotide residue corresponding to the nucleotide residue in another thereof. Such a nucleotide residue which does not form a complementary base pair in the second complementary strand may be a nucleotide residue which is present in both the target RNA and the second oligonucleotide due to a base pair composed of the corresponding nucleotide residues in the target RNA and the second oligonucleotide being a mismatched base pair (non-complementary base pair) having nucleobases not complementary to each other. In one embodiment, such a nucleotide residue which does not form a complementary base pair may be a nucleotide residue which does not form a base pair present in one of the target RNA and the second oligonucleotide, and may be preferably a nucleotide residue which does not form a base pair present in the target RNA. This results in a tendency to more promote editing induction. The mismatched base pair here means that the nucleobases in the two corresponding nucleotide residues in the target RNA and the second oligonucleotide correspond to a combination which does not form a stable base pair.

The position of the nucleotide residue which does not form a complementary base pair in the second complementary strand is preferably at any of the first residue to the third residue at the 3'-side from the linking position of the first complementary strand and the second complementary strand on the target RNA, and is more preferably at the first residue. The position of the nucleotide residue which does not form a complementary base pair in the second complementary strand is preferably at any of the first residue to the third residue at the 5'-side from the linking position of the first complementary strand and the second complementary strand on the second oligonucleotide, and is more preferably at the first residue.

In the nucleotide residue which does not form a complementary base pair in the second complementary strand, the nucleotide residue corresponding to the nucleotide residue as the first residue at the 3'-side from the linking position of the first complementary strand and the second complementary strand in the target RNA may be due to deletion from the second oligonucleotide. The nucleotide residue which does not form a complementary base pair in the second complementary strand may also be derived from a mismatched base pair formed from the nucleotide residue as the first residue at the 3'-side from the linking position of the first complementary strand and the second complementary strand in the target RNA, and the nucleotide residue at the 3'-end of the second oligonucleotide.

When a nucleotide residue corresponding to a nucleotide residue of the target RNA is absent in the second oligonucleotide, the second oligonucleotide may have a base sequence complementary to the target RNA, except for a position where the nucleotide residue is absent. When the second oligonucleotide has a nucleotide residue which does not form a complementary pair with a nucleotide residue corresponding to a nucleotide residue of the target RNA, the second oligonucleotide may have a base sequence complementary to the target RNA, except for the nucleotide residue which does not form a complementary pair.

The second oligonucleotide may have at least one bridged nucleotide residue in the second or later, preferably third or later nucleotide residues counted in the 5'-direction from the nucleotide residue at the 3'-end thereof. The 2'-position and the 4'-position of D-ribofuranose in the bridged nucleotide residue may be bridged. Specific examples of the bridged nucleotide residue are described below. The bridged nucleotide residue may contain at least one of a 2'-O,4'-C-methylene-bridged nucleotide (LNA) and a 2'-O,4'-C-ethylene-bridged nucleotide (ENA). At least one of a base moiety and a phosphoester bond moiety in the bridged nucleotide residue may be further modified. Such modification of a base moiety and a phosphoester bond moiety is described below.

The oligonucleotide composed of the second or later, preferably the third or later nucleotide residues counted in the 5'-direction from the nucleotide residue at the 3'-end of the second oligonucleotide may have a base sequence in which a 2'-O-alkyl ribonucleotide residue and a bridged nucleotide residue are alternately linked. The number of repetitions of linking of a 2'-O-alkyl ribonucleotide residue and a bridged nucleotide residue may be, for example, 1 or more and 3 or less, and may be preferably 2. Such a base sequence in which a 2'-O-alkyl ribonucleotide residue and a bridged nucleotide residue are alternately linked may be such that the alternate linking is in the 5'-direction from the 2'-O-alkyl ribonucleotide residue or may be such that the alternate linking is in the 5'-direction from the bridged nucleotide residue. Specific examples of the 2'-O-alkyl ribonucleotide residue and the bridged nucleotide residue are described below. The 2'-O-alkyl ribonucleotide residue or the bridged nucleotide residue may be further modified in at least one of a base moiety and a phosphoester bond moiety. Such modification of a base moiety and a phosphoester bond moiety is described below.

In one embodiment of the target-editing guide RNA, the first oligonucleotide that is taken with the target RNA to form the first complementary strand contains a cytidine residue as the target-corresponding nucleotide residue, a 3'-side oligonucleotide having a base sequence complementary to the base sequence of the target RNA, in which the number of residues linked to the 3'-side of the target-corresponding nucleotide residue is 12 to 18 or 14 to 16, and a 5'-side oligonucleotide having a base sequence complementary to the base sequence of the target RNA, in which the number of residues linked to the 5'-side of the target-corresponding nucleotide residue is 3, and the second oligonucleotide contains an oligonucleotide having no nucleotide residue corresponding to the first residue at the 3'-side from the linking position of the first complementary strand and the second complementary strand in the target RNA, having a complementary base sequence at the second or later residues, and having 4 to 8 residues.

Nucleotides constituting the target-editing guide RNA may be each selected from natural ribonucleotide (RNA), natural deoxyribonucleotide (DNA), RNA/DNA chimeras, and modified nucleotides as modified forms thereof. The nucleotides constituting the target-editing guide RNA may be linked by phosphodiester bonds linked to hydroxyl groups of sugar moieties of the nucleosides. The phosphodiester bonds may be each formed by utilizing a hydroxyl group at the 2'-position, a hydroxyl group at the 3'-position, or a hydroxyl group at the 5'-position of each of the sugar moieties. The nucleotides constituting the target-editing guide RNA may form natural 3'-5'phosphodiester bonds. At least one of the nucleotides constituting the target-editing guide RNA may be a modified nucleotide. Examples of the modified nucleotide may include one with a sugar moiety modified, one with a phosphodiester bond modified, one with a base modified, and a combination thereof.

Examples of the modification of a sugar moiety may include 2'-O-alkylated ribonucleotides of D-ribofuranose (for example, 2'-O-methylated, 2'-O-aminoethylated, 2'-O-propylated, 2'-O-allylated, 2'-O-methoxyethylated, 2'-O-butylated, 2'-O-pentylated, and 2'-O-propargylated); bridged ribonucleotides in which the 2'-position and the 4'-position of D-ribofuranose are bridged (for example, 2'-O,4'-C-ethylenated, 2'-O,4'-C-methylenated, 2'-O,4'-C-propylenated, 2'-O,4'-C-tetramethylenated, 2'-O,4'-C-pentamethylenated, 2'-S,4'-C-methylenated, a 2'-deoxy-2'-C,4'-C-methyleneoxymethylenated form of D-ribofuranose, S-cEt (2',4'-constrained ethyl), and AmNA); and 3' -deoxy-3' -amino-2' -deoxy-D-ribofuranose; 3' -deoxy-3' -amino-2' -deoxy-2' - fluoro-D-ribofuranose; 2'-deoxy-2'-fluoro-D-ribofuranose; and D-deoxyribose.

Examples of the modification of a phosphodiester bond moiety may include a phosphorothioate bond (including an optically active substance due to asymmetry of phosphorus atom), a methyl phosphonate bond, a methyl thiophosphonate bond, a phosphorodithioate bond, and a phosphoramidate bond.

Examples of the modification of a base moiety may include halogenation; methylation, ethylation, propylation, isopropylation, cyclopropylation, butylation, isobutylation, s-butylation, t-butylation, alkylation where the number of carbon atoms is 1 to 6, or 1 to 4, such as cyclobutylation; hydroxylation; amination; deamination; and demethylation. Specific examples thereof may include 5-methylation, 5-fluorination, 5-bromination, 5-iodination, N4-methylation, and the like of cytosine; 5-demethylation (uracil), 5-fluorination, 5-bromination, 5-iodination, and the like of thymine; N6-methylation, 8-bromination, and the like of adenine; and N2-methylation, 8-bromination, and the like of guanine.

In the target-editing guide RNA, at least one residue, at least two residues, or all the three residues selected from the counter region may be nucleotide residues other than a natural ribonucleotide (RNA) residue, and such at least one residue, at least two residues, or all the three residues are preferably modified nucleotide residues. In such a modified nucleotide residue in the counter region, at least one of the sugar moiety and the phosphodiester bond may be modified, at least the sugar moiety may be modified, at least the phosphodiester bond may be modified, or the sugar moiety and the phosphodiester bond may be modified. For example, the target-corresponding nucleotide residue may be a cytidine residue having a phosphorothioate bond. For example, in each one residue at the 5'-side or the 3'-side of the target-corresponding nucleotide residue, at least one of the sugar moiety and the phosphodiester bond may be modified, at least the sugar moiety may be modified, at least the phosphodiester bond may be modified, or the sugar moiety and the phosphodiester bond may be modified. The modification of the sugar moiety in the counter region may be, for example, 2'-O-alkylation, 2'-deoxy-2'-fluorination, or 2'-deoxylation.

In at least one residue, at least three residues, or all the residues in any oligonucleotide residue (non-counter region) other than the counter region of the first oligonucleotide, at least one of the sugar moiety and the phosphodiester bond may be modified, at least the sugar moiety may be modified, at least the phosphodiester bond may be modified, or the sugar moiety and the phosphodiester bond may be modified. The modification of the sugar moiety in the non-counter region may be, for example, 2'-O-alkylation, 2'-deoxy-2'-fluorination, bridge formation between the 2'-position and the 4'-position, or 2'-deoxylation (DNA production). When the first oligonucleotide has a plurality of modified nucleotide residues, the plurality of modified nucleotide residues may be disposed in succession or may be disposed in space. The first oligonucleotide may be configured so that all the nucleotide residues are linked by phosphorothioate bonds.

The 3'-side oligonucleotide linked to the 3'-side of the target-corresponding nucleotide residue in the first oligonucleotide may have a base sequence in which two modified nucleotide residues selected from the group consisting of a 2'-deoxy-2'-fluoronucleotide residue, a 2'-O-alkyl ribonucleotide residue, and a bridged nucleotide residue are alternately linked. In other words, the 3'-side oligonucleotide linked to the 3'-side of the target-corresponding nucleotide residue may have a base sequence in which a 2'-deoxy-2'-fluoronucleotide residue and a 2'-O-alkyl ribonucleotide residue are alternately linked, may have a base sequence in which a bridged nucleotide residue and a 2'-O-alkyl ribonucleotide residue are alternately linked, or may have a base sequence in which a 2'-deoxy-2'-fluoronucleotide residue and a bridged nucleotide residue are alternately linked. The "having a base sequence in which two modified nucleotide residues are alternately linked", here used, means both the "oligonucleotide of interest (here corresponding to the 3'-side oligonucleotide of the first oligonucleotide) having a base sequence in which two modified nucleotide residues are partially alternately linked", and the "oligonucleotide of interest having a base sequence in which two modified nucleotide residues are entirely alternately linked", and is used in the same sense also in the following description of the present specification.

The 3'-side oligonucleotide in the first oligonucleotide may be configured so that some nucleotide residues are linked by phosphorothioate bonds, or may be configured so that all the nucleotide residues are linked by phosphorothioate bonds.

The third nucleotide residue counted in the 3'-direction from the target-corresponding nucleotide residue, in the 3'-side oligonucleotide in the first oligonucleotide, may be a modified nucleotide residue selected from the group consisting of a2'-deoxy-2'-fluoronucleotide residue, a 2'-O-alkyl ribonucleotide residue, and a bridged nucleotide residue. Furthermore, the third nucleotide residue counted in the 3'-direction from the target-corresponding nucleotide residue, in the 3'-side oligonucleotide, may be a modified nucleotide residue selected from the group consisting of a 2'-deoxy-2'-fluoronucleotide residue, a 2'-O-alkyl ribonucleotide residue and a bridged nucleotide residue, and the fourth nucleotide residue may be a modified nucleotide residue different from the third nucleotide residue. The third nucleotide residue counted in the 3'-direction from the target-corresponding nucleotide residue, here mentioned, does not contain the target-corresponding nucleotide residue by itself, and exhibits the third nucleotide residue counted in the 3'-direction, with the adjacent nucleotide residue in the 3'-direction of the target corresponding oligonucleotide residue, as the first residue.

The 5'-side oligonucleotide linked to the 5'-side of the target-corresponding nucleotide residue in the first oligonucleotide may have a base sequence of consecutive 2'-O-alkyl ribonucleotide residues. The base sequence of consecutive 2'-O-alkyl ribonucleotide residues may be adjacent to the target-corresponding nucleotide residue. The number of residues in the base sequence of consecutive 2'-O-alkyl ribonucleotide residues may be, for example, 2 or 3. The 5'-side oligonucleotide may have a base sequence in which a 2'-O-alkyl ribonucleotide residue and a 2'-deoxy-2'-fluoronucleotide residue, or a bridged nucleotide residue are linked.

The 5'-side oligonucleotide in the first oligonucleotide may be configured so that some nucleotide residues are linked by phosphorothioate bonds, or may be configured so that all the nucleotide residues are linked by phosphorothioate bonds.

In at least one residue, at least three residues, or all the residues in the second oligonucleotide, at least one of the sugar moiety and the phosphodiester bond may be modified, at least the sugar moiety may be modified, at least the phosphodiester bond may be modified, or the sugar moiety and the phosphodiester bond may be modified. The modification of the sugar moiety in the second oligonucleotide may be, for example, 2'-O-alkylation, 2'-deoxy-2'-fluorination, bridge formation between the 2'-position and the 4'-position, or 2'-deoxylation (DNA production). When the second oligonucleotide has a plurality of modified nucleotide residues, the plurality of modified nucleotide residues may be disposed in succession or may be disposed in space.

The second oligonucleotide may have a base sequence of consecutive 2'-O-alkyl ribonucleotide residues. The second oligonucleotide may have a base sequence in which two selected from the group consisting of a 2'-deoxy-2'-fluoronucleotide residue, a 2'-O-alkyl ribonucleotide residue, and a bridged nucleotide residue are alternately linked, may have a base sequence in which a 2'-O-alkyl ribonucleotide residue and a 2'-deoxy-2'-fluoronucleotide residue are alternately linked, or may have a base sequence in which a 2'-O-alkyl ribonucleotide residue and a bridged nucleotide residue are alternately linked. Furthermore, the second nucleotide residue counted in the 5'-direction from the nucleotide residue at the 3'-end of the second oligonucleotide, in the second oligonucleotide, may be one selected from the group consisting of a 2'-deoxy-2'-fluoronucleotide residue, a 2'-O-alkyl ribonucleotide residue, and a bridged nucleotide residue, or may be a 2'-O-alkyl ribonucleotide residue, from the viewpoint of ADAR1 selectivity. The second nucleotide residue counted in the 5'-direction from the nucleotide residue at the 3'-end of the second oligonucleotide, here mentioned, does not contain the nucleotide residue by itself at the 3'-end, and exhibits the second nucleotide residue counted in the 5'-direction, with the adjacent nucleotide residue in the 5'-direction of the oligonucleotide residue at the 3'-end, as the first residue.

The target-editing guide RNA may include a linking portion containing an alkyleneoxy unit between the first oligonucleotide and the second oligonucleotide. The linking portion may contain an alkyleneoxy group having 1 to 8 carbon atoms, or may contain a polyalkyleneoxy group composed of an alkyleneoxy unit having 1 to 8 carbon atoms. The linking portion may be made with a phosphodiester bond or a modified phosphodiester bond to each of the 5'-side of the first oligonucleotide and the 3'-side of the second oligonucleotide. In other words, the linking portion may be made with a phosphodiester bond or a modified phosphodiester bond to each of a hydroxyl group of a sugar moiety at the 5'-end of the first oligonucleotide and a hydroxyl group of a sugar moiety at the 3'-end of the second oligonucleotide. The target-editing guide RNA may exhibit the excellent target-editing activity even if the linking portion contains an alkyleneoxy unit.

When the linking portion contains a polyalkyleneoxy group, the number of carbon atoms of the alkyleneoxy unit constituting the polyalkyleneoxy group may be, for example, 2 to 4, 2 to 3, or 2. In other words, the alkyleneoxy unit may be an ethyleneoxy unit, a propyleneoxy unit, or a butyleneoxy unit, and may be an ethyleneoxy unit. The number of alkyleneoxy units constituting the polyalkyleneoxy group may be, for example, 2 to 10, 2 to 8, 2 to 7, or 3 to 6. The alkyleneoxy units constituting the polyalkyleneoxy group may be the same as or different from each other.

When the linking portion contains an alkyleneoxy group, the number of carbon atoms of the alkyleneoxy group may be, for example, 2 to 8, 2 to 7, or 3 to 6.

In one embodiment, the target-editing guide RNA may be an oligonucleotide, or a pharmaceutically acceptable salt thereof, including a first oligonucleotide identifying a target RNA, a second oligonucleotide linked to the 5'-side of the first oligonucleotide, and a linking portion linking the first oligonucleotide and the second oligonucleotide and containing an alkyleneoxy unit, wherein the first oligonucleotide is composed of a target-corresponding nucleotide residue corresponding to an adenosine residue in the target RNA, an oligonucleotide of 10 to 24 residues, linked to the 3'-side of the target-corresponding nucleotide residue and having a base sequence complementary to the target RNA, and an oligonucleotide of 3 to 6 residues, linked to the 5'-side of the target-corresponding nucleotide residue and having a base sequence complementary to the target RNA, the number of residues in the second oligonucleotide is 2 to 10 and the second oligonucleotide has no nucleotide residue corresponding to a nucleotide residue of the target RNA or has a nucleotide residue which does not form a complementary pair with a nucleotide residue of the target RNA, at the 3'-end thereof, at least the nucleotide residues at other than the 3'-end form a double-stranded structure complementary to the target RNA, or has a base sequence complementary to the target RNA to form a double-stranded structure complementary to the target RNA, at least one residue selected from a counter region composed of the target-corresponding nucleotide residue and each one residue at the 3'-side and the 5'-side thereof is a nucleotide residue other than a natural ribonucleotide residue, and the oligonucleotide, or a pharmaceutically acceptable salt thereof, induces site-specific editing for the target RNA. The first oligonucleotide and the second oligonucleotide constituting the target-editing guide RNA containing the linking portion may be configured from any modified nucleotide residue described above.

In one embodiment, the target-editing guide RNA may be an oligonucleotide, or a pharmaceutically acceptable salt thereof, including a first oligonucleotide identifying a target RNA, a second oligonucleotide linked to the 5'-side of the first oligonucleotide, and a linking portion linking the first oligonucleotide and the second oligonucleotide and containing an alkyleneoxy unit, wherein the first oligonucleotide is composed of a target-corresponding nucleotide residue corresponding to an adenosine residue in the target RNA, an oligonucleotide of 10 to 24 residues, linked to the 3'-side of the target-corresponding nucleotide residue and having a base sequence complementary to the target RNA, and an oligonucleotide of 3 to 6 residues, linked to the 5'-side of the target-corresponding nucleotide residue and having a base sequence complementary to the target RNA, the second oligonucleotide has a number of residues of 2 to 10 and has a base sequence complementary to the target RNA to form a double-stranded structure complementary to the target RNA, at least one residue selected from a counter region composed of the target-corresponding nucleotide residue and each one residue at the 3'-side and the 5'-side thereof is a nucleotide residue other than a natural ribonucleotide residue, and the oligonucleotide, or a pharmaceutically acceptable salt thereof, induces site-specific editing for the target RNA. The first oligonucleotide and the second oligonucleotide constituting the target-editing guide RNA containing the linking portion may be configured from any modified nucleotide residue described above.

In one embodiment, the target-editing guide RNA may be an oligonucleotide represented by any formula selected from the group consisting of the following
(AD1_ASS1.39), (AD1_PANK2.39), (AD1_NPHS2.39), (AD1_GRIA2.39), (AD1_ASS1.52), (AD1_GRIA2.39e), (AD1_ASS1.39e), (AD1_PANK2.39e), (AD1_NPHS2.39e), (AD1_GRIA2.52e), (AD1_ASS1.52e), (AD1_PANK2.52e), (AD1_NPHS2.52e) and (AD1_A1AT.39), or a pharmaceutically acceptable salt thereof.
(AD1_ASS1.39) 
U(M)^T(L)^G(M)^A(L)^U(M)^G(F)^A(F)^C(M)^C(M)^c^i^G(M)^U(F)^G(M)^G(

F)^C(M)^A(F)^U(M)^C(F)^A(M)^G(L)^U(M)^T(L)^G(M)
(AD1_PANK2.39) 
A(M)^A(L)^G(M)^A(L)^A(M)^A(F)^U(F)^U(M)^C(M)^c^a^A(M)^C(F)^A(M)^A(
F)^A(M)^U(F)^A(M)^C(F)^C(M)^A(L)^C(M)^C(L)^U(M)
(AD1_NPHS2.39) 
A(M)^T(L)^G(M)^T(L)^C(M)^C(F)^A(F)^G(M)^U(M)^c^i^G(M)^A(F)^A(M)^U(
F)^A(M)^U(F)^A(M)^A(F)^U(M)^T(L)^A(M)^C(L)^U(M)
(AD1_GRIA2.39) 
G(M)^C(L)^A(M)^T(L)^C(M)^C(F)^U(F)^G(M)^C(M)^c^i^C(M)^A(F)^U(M)^A(
F)^A(M)^A(F)^G(M)^G(F)^C(M)^A(L)^C(M)^C(L)^C(M)
(AD1_ASS1.52) 
U(M)^G(L)^A(M)^U(L)^G(F)^A(F)^C(M)^C(M)^c^i^G(M)^U(F)^G(M)^G(F)^C(

M)^A(F)^U(M)^C(F)^A(L)^G(M)^U(L)^U(M)
(AD1_GRIA2.39e) 
G(M)^C(E)^A(M)^T(E)^C(M)^C(F)^U(F)^G(M)^C(M)^c^i^C(M)^A(F)^U(M)^A(
F)^A(M)^A(F)^G(M)^G(F)^C(M)^A(E)^C(M)^C(E)^C(M)
(AD1_ASS1.39e) 
U(M)^T(E)^G(M)^A(E)^U(M)^G(F)^A(F)^C(M)^C(M)^c^i^G(M)^U(F)^G(M)^G(
F)^C(M)^A(F)^U(M)^C(F)^A(M)^G(E)^U(M)^T(E)^G(M)
(AD1_PANK2.39e) 
A(M)^A(E)^G(M)^A(E)^A(M)^A(F)^U(F)^U(M)^C(M)^c^a^A(M)^C(F)^A(M)^A(
F)^A(M)^U(F)^A(M)^C(F)^C(M)^A(E)^C(M)^C(E)^U(M)
(AD1_NPHS2.39e) 
A(M)^T(E)^G(M)^T(E)^C(M)^C(F)^A(F)^G(M)^U(M)^c^i^G(M)^A(F)^A(M)^U(
F)^A(M)^U(F)^A(M)^A(F)^U(M)^T(E)^A(M)^C(E)^U(M)
(AD1_GRIA2.52e) 
C(M)^A(E)^U(M)^C(E)^C(F)^U(F)^G(M)^C(M)^c^i^C(M)^A(F)^U(M)^A(F)^A(
M)^A(F)^G(M)^G(F)^C(E)^A(M)^C(E)^C(M)
(AD1_ASS1.52e) 
U(M)^G(E)^A(M)^T(E)^G(F)^A(F)^C(M)^C(M)^c^i^G(M)^U(F)^G(M)^G(F)^C(
M)^A(F)^U(M)^C(F)^A(E)^G(M)^T(E)^U(M)
(AD1_PANK2.52e) 
A(M)^G(E)^A(M)^A(E)^A(F)^U(F)^U(M)^C(M)^c^a^A(M)^C(F)^A(M)^A(F)^A(
M)^U(F)^A(M)^C(F)^C(E)^A(M)^C(E)^C(M)
(AD1_NPHS2.52e) 
U(M)^G(E)^U(M)^C(E)^C(F)^A(F)^G(M)^U(M)^c^i^G(M)^A(F)^A(M)^U(F)^A(
M)^U(F)^A(M)^A(F)^T(E)^U(M)^A(E)^C(M)
(AD1_A1AT.39)

G(M)^T(L)^C(M)^C(L)^C(M)^U(F)^U(F)^C(M)^U(M)^c^i^U(M)^C(F)^G(M)^A(
F)^U(M)^G(F)^G(M)^U(F)^C(M)^A(L)^G(M)^C(L)^A(M)

In the formulae, a capital letter represents a ribonucleotide residue (RNA). A small letter represents a 2'-deoxyribonucleotide residue (DNA). N(M) represents a 2'-O-methylated ribonucleotide residue of D-ribofuranose. N(F) represents a 2'-fluoro-2'-deoxyribonucleotide residue of D-ribofuranose. N(L) represents a 2'-O,4'-C-methylene-bridged ribonucleotide residue of D-ribofuranose. N(E) represents a 2'-O,4'-C-ethylene-bridged ribonucleotide residue of D-ribofuranose. "^" represents that nucleoside units are linked by -P(=S)(OH)-, namely, represents a phosphorothioate bond.

The target-editing guide RNA may be synthesized according to a method described in a known document (see, for example, Nucleic Acids Research, 12, 4539 (1984)), by use of a commercially available synthesizer (for example, model 392 manufactured by PerkinElmer Inc., by a phosphoramidite method). The phosphoramidite reagent to be used may be a commercially available reagent, or may be a reagent appropriately synthesized according to a method described in a known document. After coupling of the phosphoramidite reagent, reaction with a reagent such as sulfur, tetraethylthiuram disulfide (TETD, Applied Biosystems), Beaucage reagent (Glen Research), or xanthan hydride may be made to thereby introduce a phosphorothioate bond (see, for example, Tetrahedron Letters, 32, 3005(1991), J. Am. Chem. Soc., 112, 1253(1990), PCT/WO98/54198).

The oligonucleotide (target-editing guide RNA) may be used in the form of a pharmaceutically acceptable salt. The "pharmaceutically acceptable salt" refers to a salt of an oligonucleotide. Examples of such a salt may include metal salts, for example, alkali metal salts such as a sodium salt, a potassium salt, and a lithium salt, alkali earth metal salts such as a calcium salt and a magnesium salt, an aluminum salt, an iron salt, a zinc salt, a copper salt, a nickel salt, and a cobalt salt; amine salts, for example, inorganic salts such as ammonium salts;, and organic salts such as a t-octylamine salt, a dibenzylamine salt, a morpholine salt, a glucosamine salt, a phenylglycine alkyl ester salt, an ethylenediamine salt, an *N*-methylglucamine salt, a guanidine salt, a diethylamine salt, a triethylamine salt, a dicyclohexylamine salt, an *N*,*N*'-dibenzylethylenediamine salt, a chloroprocaine salt, a procaine salt, a diethanolamine salt, an *N*-benzyl-phenethylamine salt, a piperazine salt, a tetramethylammonium salt, and a tris(hydroxymethyl)aminomethane salt; inorganic acid salts, for example, hydrohalic acid salts such as hydrofluoride, hydrochloride, hydrobromide and hydroiodide, nitrate, perchlorate, sulfate, and phosphate; organic acid salts, for example, lower alkane sulfonates such as methane sulfonate, trifluoromethane sulfonate, and ethane sulfonate, aryl sulfonates such as benzene sulfonate and p-toluene sulfonate, acetate, malate, fumarate, succinate, citrate, tartrate, oxalate, and maleate; and amino acid salts such as a glycine salt, a lysine salt, an arginine salt, an ornitine salts, glutamate, and aspartate. The form of the pharmaceutically acceptable salt of the oligonucleotide is preferably an alkali metal salt of the oligonucleotide, more preferably a sodium salt thereof. Such a salt may be produced by a known method.

The oligonucleotide and the pharmaceutically acceptable salt thereof may also be present as solvates (for example, hydrates), and may be such solvates.

An optically active substance due to asymmetry of a phosphorus atom may be present in the oligonucleotide and the pharmaceutically acceptable salt thereof, and the oligonucleotide of the present invention also encompasses such an optically active substance. Such an optically active substance may be synthesized by a known method (for example, Org. Lett., 114, 967 (2009), Bioorganic & Medicinal Chemistry Letters, 8, 2359(1998)).

A delivery molecule of GalNAc, cholesterol, fatty acid, and the like may be bound via a linker or a phosphodiester bond (including a phosphorothioate bond) at the 5'-end or the 3'-end of the oligonucleotide and the pharmaceutically acceptable salt thereof. Examples of such delivery molecule and linker may include those described in Bioconjugate Chem. 2019, 30, 366. The oligonucleotide and the pharmaceutically acceptable salt thereof, where the delivery molecule is bound, may be produced by a known production method. The oligonucleotide and the pharmaceutically acceptable salt thereof, herein described, encompass a form where the delivery molecule is bound.

When the oligonucleotide, or the pharmaceutically acceptable salt or solvate thereof is used for the treatment of a disease, the oligonucleotide, or the pharmaceutically acceptable salt or solvate thereof, by itself, or a mixture thereof with an appropriately pharmaceutically acceptable excipient, diluent, and/or the like may be orally administered as a tablet, a capsule, a granule, a powder, a syrup, or the like, or may be parenterally administered as an injection, a suppository, a patch, a topical product, or the like.

Such a formulation is produced by a well-known method by use of additive(s) such as an excipient (examples include organic excipients: for example, sugar derivatives such as lactose, sucrose, dextrose, mannitol, and sorbitol; starch derivatives such as corn starch, potato starch, α starch, and dextrin; cellulose derivatives such as crystalline cellulose; gum arabic; dextran; and pullulan; and inorganic excipients: for example, silicate derivatives such as light anhydrous silicic acid, synthetic aluminum silicate, calcium silicate, and magnesium aluminometasilicate; phosphates such as calcium hydrogen phosphate; carbonates such as calcium carbonate; and sulfates such as calcium sulfate), a lubricant (examples include stearic acid; stearic acid metal salts such as calcium stearate and magnesium stearate; talc; colloidal silica; wax compounds such as bead wax and gay wax; boric acid; adipic acid; sulfates such as sodium sulfate; glycol; fumaric acid; sodium benzoate; DL leucine; lauryl sulfates such as sodium lauryl sulfate and magnesium lauryl sulfate: silicic acid compounds such as anhydrous silicia acid and silicia acid hydrate; and the above starch derivatives), a binder (examples include hydroxypropyl cellulose, hydroxypropylmethyl cellulose, polyvinylpyrrolidone, macrogol, and the same compounds as in the excipient), a disintegrant (examples include cellulose derivatives such as low-substituted hydroxypropyl cellulose, carboxymethyl cellulose, carboxymethyl cellulose calcium, and internally crosslinked carboxymethyl cellulose sodium; carboxymethyl starch, carboxymethyl starch sodium, and chemically modified starch/cellulose compounds such as crosslinked polyvinylpyrrolidone), an emulsifier (examples include colloidal clay such as bentonite and veegum; metal hydroxides such as magnesium hydroxide and aluminum hydroxide; anionic surfactants such as sodium lauryl sulfate and calcium stearate; cationic surfactants such as benzalkonium chloride; and non-ionic surfactants such as polyoxyethylene alkyl ether, polyoxyethylene sorbitan fatty acid ester, and sucrose fatty acid ester), a stabilizer (examples include paraoxybenzoate compounds such as methylparaben and propylparaben; alcohol compounds such as chlorobutanol, benzyl alcohol, and phenyl ethyl alcohol; benzalkonium chloride; phenol compounds such as phenol and cresol; thimerosal; dehydroacetic acid; and sorbic acid), a flavoring agent (examples include sweeteners, acidulants, and flavors, which are commonly used), and/or a diluent.

A therapeutic agent containing the oligonucleotide, or the pharmaceutically acceptable salt or solvate thereof may contain 0.1 to 250 µmoles/ml, preferably 1 to 50 µmoles/ml of the oligonucleotide. The therapeutic agent may also be formed by containing a predetermined amount of the oligonucleotide, or the pharmaceutically acceptable salt or solvate thereof, 0.02 to 10%w/v of carbohydrate or polyhydric alcohol, and 0.01 to 0.4%w/v of a pharmaceutically acceptable surfactant.

The carbohydrate is particularly preferably at least one of monosaccharide and disaccharide. Examples of such carbohydrate and polyhydric alcohol include glucose, galactose, mannose, lactose, maltose, mannitol, and sorbitol. These may be used singly or in combination thereof.

Preferable examples of the surfactant include polyoxyethylene sorbitan mono- to tri-esters, alkylphenyl polyoxyethylene, sodium taurocholate, sodium collate, and polyhydric alcohol ester. Among them, polyoxyethylene sorbitan mono- to tri-esters are particularly preferable, and particularly preferable esters are oleate, laurate, stearate, and palmitate. These may be used singly or in combination thereof.

The therapeutic agent containing the oligonucleotide, or the pharmaceutically acceptable salt or solvate thereof may further preferably contain 0.03 M to 0.09 M of a pharmaceutically acceptable neutral salt, for example, sodium chloride, potassium chloride and/or calcium chloride.

The therapeutic agent containing the oligonucleotide, or the pharmaceutically acceptable salt or solvate thereof may further preferably contain 0.002 to 0.05 M of a pharmaceutically acceptable buffer. Examples of a preferable buffer include sodium citrate, sodium glycinate, sodium phosphate, and tris(hydroxymethyl)aminomethane. These buffers may be used singly or in combination thereof.

The therapeutic agent described above may be supplied in a solution state. However, for the case where storage for a certain period of time is needed, the therapeutic agent is preferably usually freeze-dried for the purpose of stabilization of the oligonucleotide and prevention of a decrease in effect of treatment, and in such a case, the therapeutic agent, when used, may be reconstructed with a liquid for dissolution (such as distilled water for injection), namely, may be formed into a liquid to be administered. Accordingly, the therapeutic agent of the present invention also includes a freeze-dried product to be used after reconstruction with a liquid for dissolution so that each component has a concentration in a predetermined range. Any amino acid such as albumin and/or glycine may be further contained for the purpose of promotion of solubility of a freeze-dried product.

The oligonucleotide, or the pharmaceutically acceptable salt or solvate thereof, when administered to human, may be administered in one or several doses at, for example, a dosage of about 0.01 mg/kg to 100 mg/kg (body weight), preferably 0.1 mg/kg to 20 mg/kg (body weight) per day for an adult by subcutaneous injection, intravenous drip injection, or intravenous injection, and the dosage and the number of doses may be appropriately changed depending on the type and symptom of a disease, the age, the administration method, and the like.

### Treatment method of disease

A treatment method of a disease includes a step of administering the therapeutic agent to a subject. The treatment method of a disease may include a step of administering a pharmacologically effective amount of the therapeutic agent, to a subject. The "treatment" herein used may be any treatment applied to a disease, and examples thereof include therapy, amelioration, suppression of progression (prevention of exacerbation), and prevention (suitably therapy or prevention) of a disease. The subject to be treated may be a warm-blooded animal including human, or may be a non-human warm-blooded animal or human.

### Site-specific editing method for target RNA

A site-specific editing method for a target RNA includes a step of bringing the target RNA into contact with a target-editing guide RNA as an oligonucleotide inducing site-specific editing for the target RNA, in the presence of adenosine deaminase. The target-editing guide RNA may be taken with the target RNA to partially form a double strand and recruit adenosine deaminase, and thus site-specifically convert an adenosine residue contained in the target RNA into an inosine residue. The site-specific editing method for the target RNA may further include a step of preparing the target-editing guide RNA.

The site-specific editing method for the target RNA may be performed by, for example, introducing the above target-editing guide RNA into eukaryotic cells having the target RNA. A procedure appropriately selected from various procedures used in nucleic acid medicines may be applied to a method for introducing the target-editing guide RNA into the eukaryotic cells. The site-specific editing method for the target RNA may be performed *in vitro* or *in vivo.*

Other embodiments of the present invention also encompass use of the target-editing guide RNA in the production of a pharmaceutical composition for use in the treatment of a disease (for example, hereditary disease), use of the target-editing guide RNA in the treatment of a disease (for example, hereditary disease), and the target-editing guide RNA for use in the treatment of a disease (for example, hereditary disease).

### Examples

Hereinafter, the present invention is more specifically described with reference to Examples, but the present invention is not limited to these Examples.

### (Reference Example 1)

An oligonucleotide having a sequence (SEQ ID NO: 1) shown in Table 1 and fully composed of natural RNA residues (hereinafter, also referred to as "AD1-gRNA03") was synthesized with a phosphoramidite method (see, for example, Nucleic Acids Research, 12, 4539 (1984), Nature Communications 6, Article number: 6317 (2015)). The resulting compound was identified by negative-ion ESI mass spectrometry (measured value: 7714.8).

In Table 1, the underlined part corresponds to the first oligonucleotide (ASR), and RNA as a target is Rluc_sRNA described below. The oligonucleotide of Reference Example 1 is considered to be capable of taking, for example, the following structure together with such a target RNA.

**[Table 1]**

| | Sequence | SEQ ID NO: |
|---|---|---|
| AD1-gRNA03 | CAGCUCAACCAAGCGGUGAGGUAC | 1 |
| Rluc_sRNA target part | GUACCUCACCGCUUAGUUCGAGCUG | 2 |

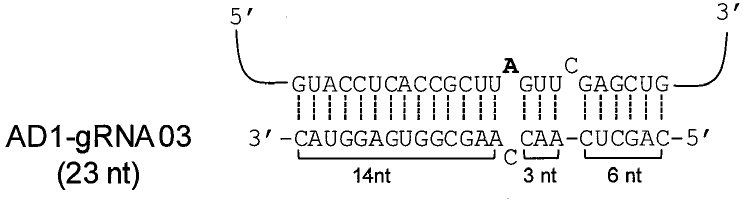

### (Reference Example 2)

hGAPDH_sRNA02 (SEQ ID NO: 3) having the following sequence was prepared as a model target RNA, in the same manner as in Rluc_sRNA described below. An oligoribonucleotide hGAPDH_AD1 (SEQ ID NO: 4) having the following sequence was prepared as a target-editing guide RNA to the model target RNA, by a phosphoramidite method. The underline in the Table indicates the adenosine residue to be edited.

**[Table 2]**

| | Sequence | SEQ ID NO: |
|---|---|---|
| hGAPDH_sRNA | GGGCCUCUUCGCUAUUACGCCAGCUGG CGAAAGGGGGAUGUGCUGCAAGGCGAU UAAGUUGGGUAACGCCAGGGUUUUCCC AGUCACGACGUUGUAAAACGACGGCCA GUGAAUUCUCUCCCCUCCUCACAGUUGC CAUGUAGACCCCUUGAAGAGGGGAGGG GCCUAAGCUUGGCGUAAUCAUGGUCAUA GCUGUUUCC | 3 |
| hGAPDH_AD | UCAAGGGUCCACAUGGCAACUGUG | 4 |

### (Examples 1 to 32)

Oligonucleotides of Examples 1 to 16 were obtained based on the sequence of the oligonucleotide of Reference Example 1, by introduction of modified nucleotides as shown in Table 3. Oligonucleotides of Examples 17 to 32 were obtained based on the sequence of the oligonucleotide of Reference Example 2, by introduction of modified nucleotides as shown in Table 4. These oligonucleotide compounds were synthesized by the phosphoramidite method in the same manner as in Reference Example 1. In the sequences, when an "18" section was synthesized, DMT Hexaethylene Glycol phosphoramidite (ChemGenes, catalog number: CLP-9765) was used. When a "9" section was synthesized, DMT-Triethoxy-glycol phosphoramidite (ChemGenes, catalog number: CLP-1113) was used. When a "6" section was synthesized, DMT-hexane-Diol phosphoramidite (ChemGenes, catalog number: CLP-1120) was used. When a "3" section was synthesized, DMT-propane-Diol phosphoramidite (ChemGenes, catalog number: CLP-9908) was used. The "2'-O-methyl nucleoside" section was synthesized with a phosphoramidite form described in Nucleic Acids Research 17, 3373 (1989). The "DNA" section was synthesized with a phosphoramidite form described in Nucleic Acids Research 11, 4539 (1984). The "2'-O, 4'-C-methylene nucleoside" section was synthesized with a phosphoramidite form described in WO99/14226. The "2'-deoxy-2'-fluoro nucleoside" section was synthesized with a phosphoramidite form described in J. Med. Chem., 36, 831(1993).

**[Table 3]**

| Example | Sequece name | Sequence (5'-3') | Molecular weight |
|---|---|---|---|
| 1 | AD1-gRNA03_1 | C(M)^A(M)^G(M)^C(M)^U(M)^C(M)^A(M)^A(M)^C(M)^C^A(M)^A(M)^G(M) ^C(M)^G(M)^G(M)^U(M)^G(M)^A(M)^G(M)^G(M)^U(M)^A(M)^C(M) | 8406.10 |
| 2 | AD1-gRNA03_2 | C(M)^A(F)^G(M)^C(F)^U(M)^C(F)^A(F)^A(M)^C(M)^C^A(M)^A(M)^G(M)^ C(M)^G(M)^G(M)^U(M)^G(M)^A(M)^G(M)^G(M)^U(M)^A(M)^C(M) | 8357.90 |
| 3 | AD1-gRNA03_3 | C(M)^A(F)^G(M)^C(F)^U(M)^C(F)^A(F)^A(M)^C(M)^C^A(M)^A(F)^G(M)^C (F)^G(M)^G(F)^U(M)^G(F)^A(M)^G(F)^G(M)^U(F)^A(M)^C(F) | 8273.70 |
| 4 | AD1-gRNA03_4 | C(M)^A(F)^G(M)^C(F)^U(M)^C(F)^A(F)^A(M)^C(M)^C^A(M)^A(M)^G(F)^C (M)^G(F)^G(M)^U(F)^G(M)^A(F)^G(M)^G(F)^U(M)^A(F)^C(M) | 8285.80 |
| 5 | AD1-gRNA03_5 | CAGCUC3AACCAAGCGGUGAGGUAC | 7852.60 |
| 6 | AD1-gRNA03_6 | CAGCUCG3AACCAAGCGGUGAGGUAC | 8198.10 |
| 7 | AD1-gRNA03_7 | CAGCUC9AACCAAGCGGUGAGGUAC | 7927.00 |
| 8 | AD1-gRNA03_8 | CAGCUCG9AACCAAGCGGUGAGGUAC | 8272.20 |
| 9 | AD1-gRNA03_9 | C(M)^A(L)^G(M)^C(L)^U(M)^C(L)^A(L)^A(M)^C(M)^C^A(M)^A(M)^G(F)^C( M)^G(F)^G(M)^U(F)^G(M)^A(F)^G(M)^G(F)^U(M)^A(F)^C(M) | 8352.82 |
| 10 | AD1-gRNA03_10 | C(M)^A(F)^G(M)^C(F)^U(M)^C(F)^A(F)^A(M)^C(M)^C^A(M)^A(M)^G(L)^C (M)^G(L)^G(M)^U(L)^G(M)^A(L)^G(M)^G(L)^U(M)^A(L) | 8024.57 |
| 11 | AD1-gRNA03_11 | C(M)^A(L)^G(M)^C(L)^U(M)^C(L)^A(L)^A(M)^C(M)^C^A(M)^A(M)^G(L)^C( M)^G(L)^G(M)^U(L)^G(M)^A(L)^G(M)^G(L)^U(M)^A(L) | 8091.75 |
| 12 | AD1-gRNA03_12 | C(M)^A(F)^G(M)^C(F)^U(M)^C(F)^G(M)^18^A(F)^A(M)^C(M)^C^A(M)^A( M)^G(F)^C(M)^G(F)^G(M)^U(F)^G(M)^A(F)^G(M)^G(F)^U(M)^A(F)^C(M) | 9025.15 |
| 13 | AD1-gRNA03_13 | C(M)^A(F)^G(M)^C(F)^U(M)^C(F)^G(M)^9^A(F)^A(M)^C(M)^C^A(M)^A(M) ^G(F)^C(M)^G(F)^G(M)^U(F)^G(M)^A(F)^G(M)^G(F)^U(M)^A(F)^C(M) | 8889.46 |
| 14 | AD1-gRNA03_14 | C(M)^A(F)^G(M)^C(F)^U(M)^C(F)^G(M)^6^A(F)^A(M)^C(M)^C^A(M)^A(M) ^G(F)^C(M)^G(F)^G(M)^U(F)^G(M)^A(F)^G(M)^G(F)^U(M)^A(F)^C(M) | 8857.34 |
| 15 | AD1-gRNA03_15 | C(M)^A(F)^G(M)^C(F)^U(M)^C(F)^G(M)^3^A(F)^A(M)^C(M)^C^A(M)^A(M) ^G(F)^C(M)^G(F)^G(M)^U(F)^G(M)^A(F)^G(M)^G(F)^U(M)^A(F)^C(M) | 8815.39 |
| 16 | AD1-gRNA03_16 | C(M)^A(L)^G(M)^C(L)^U(M)^C(L)^G(M)^9^A(L)^A(M)^C(M)^C^A(M)^A(M)^ G(L)^C(M)^G(L)^G(M)^U(L)^G(M)^A(L)^G(M)^G(L)^U(M)^A(L) | 8695.38 |

**[Table 4]**

| Example | Sequece name | Sequence (5'-3') | Molecular weight |
|---|---|---|---|
| 17 | hGAPDH_AD1_4 | U(M)^C(F)^A(M)^A(F)^G(M)^G(F)^G(F)^U(M)^C(M)^C^A(M)^C(M)^A(F)^U (M)^G(F)^G(M)^C(F)^A(M)^A(F)^C(M)^U(F)^G(M)^U(F)^G(M) | 8267.04 |
| 18 | hGAPDH_AD1_17 | U(M)^C(F)^A(M)^A(F)^G(M)^G(F)^G(F)^U(M)^C(M)^C^A(M)^C(M)^A(F)^U (M)^G(F)^G(M)^C(F)^A(M)^A(F)^C(M)^U(F)^G(M)^U(F)^G(M)^A(F)^G(M) | 8989.72 |
| 19 | hGAPDH_AD1_18 | U(M)^C(F)^A(M)^A(F)^G(M)^G(F)^G(F)^U(M)^C(M)^C^A(M)^C(M)^A(F)^U (M)^G(F)^G(M)^C(F)^A(M)^A(F)^C(M)^U(F)^G(M)^U(F)^G(M)^A(F)^G(M)^ G(F)^A(M) | 9711.77 |
| 20 | hGAPDH_AD1_19 | U(M)^C(F)^A(M)^A(F)^G(M)^G(F)^G(F)^U(M)^C(M)^C^A(M)^C(M)^A(F)^U (M)^G(F)^G(M)^C(F)^A(M)^A(F)^C(M)^U(F)^G(M)^U(F)^G(M)^A(F)^G(M)^ G(F)^A(M)^G(F)^G(M) | 10453.16 |
| 21 | hGAPDH_AD1_20 | T(L)^C(F)^A(L)^A(F)^G(M)^G(F)^G(F)^U(M)^C(M)^C^A(M)^C(M)^A(F)^U( M)^G(F)^G(M)^C(F)^A(M)^A(F)^C(M)^U(F)^G(M)^U(F)^G(M) | 8274.31 |
| 22 | hGAPDH_AD1_21 | T(L)^C(F)^A(L)^A(F)^G(L)^G(F)^G(F)^U(M)^C(M)^C^A(M)^C(M)^A(F)^U(M )^G(F)^G(M)^C(F)^A(M)^A(F)^C(M)^U(F)^G(M)^U(F)^G(M) | 8272.55 |
| 23 | hGAPDH_AD1_22 | U(M)^C(F)^A(M)^A(F)^G(M)^G(F)^G(F)^U(M)^C(M)^C^A(M)^C(M)^A(F)^T (L)^G(F)^G(L)^C(F)^A(L)^A(F)^C(M)^U(F)^G(M)^U(F)^G(M) | 8272.27 |
| 24 | hGAPDH_AD1_23 | U(M)^C(F)^A(M)^A(F)^G(M)^G(F)^G(F)^U(M)^C(M)^C^A(M)^C(M)^A(F)^U (M)^G(F)^G(M)^C(F)^A(M)^A(F)^C(L)^U(F)^G(L)^U(F)^G(L) | 8273.09 |
| 25 | hGAPDH_AD1_24 | U(M)^C(F)^A(M)^A(F)^G(M)^G(F)^G(F)^U(M)^C(M)^C^A(M)^C(M)^A(F)^T (L)^G(F)^G(L)^C(F)^A(L)^A(F)^C(L)^U(F)^G(L)^U(F)^G(L) | 8280.49 |
| 26 | hGAPDH_AD1_25 | T(L)^C(F)^A(L)^A(F)^G(M)^G(F)^G(F)^U(M)^C(M)^C^A(M)^C(M)^A(F)^U( M)^G(F)^G(M)^C(F)^A(M)^A(F)^C(L)^U(F)^G(L)^U(F)^G(L) | 8279.42 |
| 27 | hGAPDH_AD1_26 | T(L)^C(F)^A(L)^A(F)^G(L)^G(F)^G(F)^U(M)^C(M)^C^A(M)^C(M)^A(F)^U(M )^G(F)^G(M)^C(F)^A(M)^A(F)^C(L)^U(F)^G(L)^U(F)^G(L) | 8282.49 |
| 28 | hGAPDH_AD1_27 | T(L)^C(F)^A(L)^A(F)^G(M)^G(F)^G(F)^U(M)^C(M)^C^A(M)^C(M)^A(F)^T(L )^G(F)^G(L)^C(F)^A(L)^A(F)^C(L)^U(F)^G(L)^U(F)^G(L) | 8290.76 |
| 29 | hGAPDH_AD1_28 | T(L)^C(F)^A(L)^A(F)^G(L)^G(F)^G(F)^U(M)^C(M)^C^A(M)^C(M)^A(F)^T(L) ^G(F)^G(L)^C(F)^A(L)^A(F)^C(L)^U(F)^G(L)^U(F)^G(L) | 8290.60 |
| 30 | hGAPDH_AD1_29 | U(M)^C(F)^A(M)^A(F)^G(M)^G(F)^G(F)^U(M)^C(M)^C^a^C(M)^A(F)^U(M) ^G(F)^G(M)^C(F)^A(M)^A(F)^C(M)^U(F)^G(M)^U(F)^G(M) | 8232.90 |
| 31 | hGAPDH_AD1_31 | U(M)^C(F)^A(M)^A(F)^G(M)^G(F)^G(F)^U(M)^C(M)^c^a^C(M)^A(F)^U(M)^ G(F)^G(M)^C(F)^A(M)^A(F)^C(M)^U(F)^G(M)^U(F)^G(M) | 8217.93 |
| 32 | hGAPDH_AD1_32 | U(M)^C(F)^A(M)^A(F)^G(M)^G(F)^G(F)^U(M)^C(M)^5c^a^C(M)^A(F)^U(M )^G(F)^G(M)^C(F)^A(M)^A(F)^C(M)^U(F)^G(M)^U(F)^G(M) | 8231.97 |

The "Molecular weight" in the Tables represents a measured value by negative-ion ESI mass spectrometry. In the "Sequence" in the Tables, a capital letter represents an RNA, a small letter represents a DNA, N(M) represents 2'-O-methylation of D-ribofuranose, N(F) represents 2'-deoxy-2'-fluorination of D-ribofuranose, N(L) represents 2'-O,4'-C-methylenation of D-ribofuranose, and N(E) represents 2'-O,4'-C-ethylenation of D-ribofuranose. "3" represents a linker represented by -O(CH₂)₃O-, "6" represents a linker represented by -O(CH₂)₆O-, "9" represents a linker represented by -O(CH₂CH₂O)₃-, and "18" represents a linker represented by -O(CH₂CH₂O)₆-. "^" represents that nucleoside units are linked by - P(=S)(OH)-. Unless particularly noted, it represents that nucleoside units, or a nucleoside unit and a linker are linked by -P(=O)(OH)-. The 5'-end and the 3'-end of each oligonucleotide correspond to a hydroxyl group formed by linking of a hydrogen atom with an oxygen atom in each chemical formula. The structures of the nucleoside units are shown below. Each broken line in chemical formulae indicates a linking site.

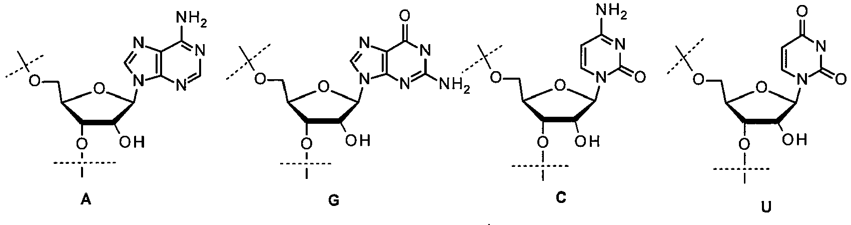

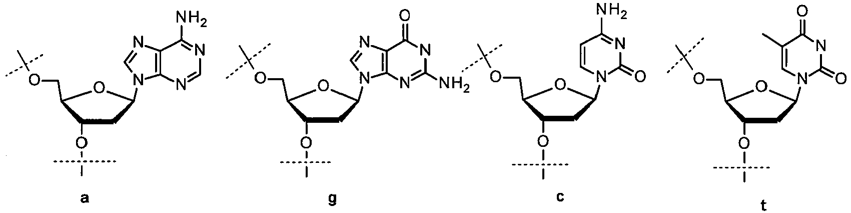

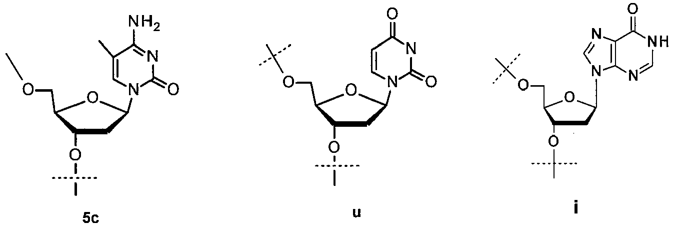

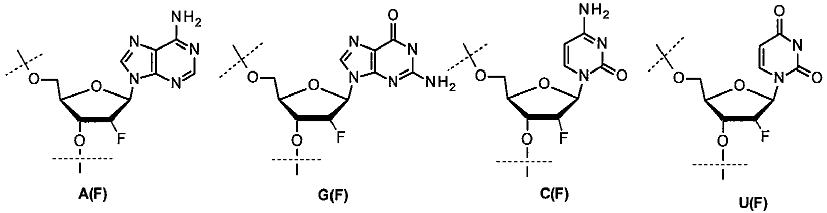

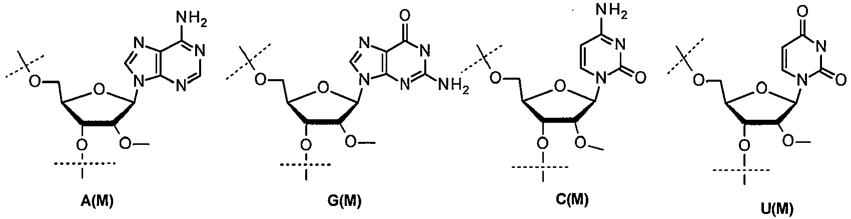

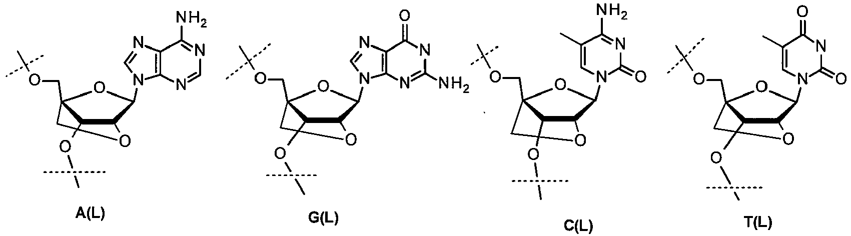

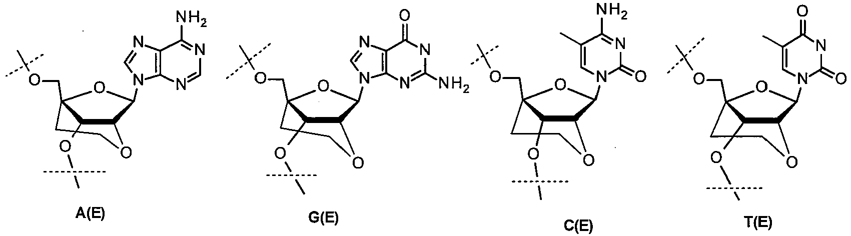

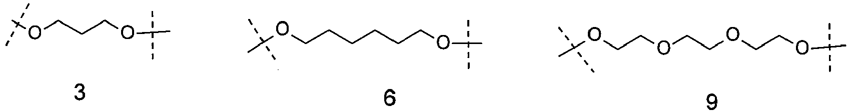

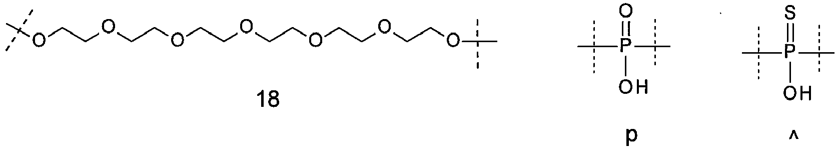

### (Test Example 1)

### Preparation of adenosine deaminase

Recombinant hADAR2 and hADAR1p110, as adenosine deaminase, were prepared by synthesis/purification with a yeast expression system, according to the descriptions of documents (Macbeth, MR. and Bass, BL. Methods Enzymol. 424, 319-331 (2007), Fukuda, M. et al. Sci. Rep. srep41478 (2017)). Plasmids (pcDNA3.1(-)Hygro_ADAR2, pcDNA3.1(-)Hygro_ADAR1p110, pcDNA3.1(-)Hygro_ADAR1p150) expressing hADAR2, hADAR1p110 and hADAR1p150 were prepared by ordinary methods.

### Preparation of model target RNA: Synthesis of Rluc_sRNA

Rluc_WT RNA (SEQ ID NO: 5) prepared from psiCHECK^{™}-2 Vector (Promega) by transcription according to an ordinary method was prepared so as to be at 0.2 nM. The editing reaction *in vitro* was performed by addition of recombinant hADAR2 at a final concentration of 1 µM and incubation in an editing reaction buffer (20 mM HEPES-KOH (pH 7.5), 2 mM MgCl₂, 100 mM NaCl, 0.5 mM DTT, 0.01% TritonX-100, 5% glycerol) at 37°C for 2 hours.

Rluc_WT RNA after the editing reaction was purified by phenol/chloroform extraction and ethanol precipitation. Next, cDNA was synthesized by Rluc_WT_BamR01 primer (SEQ ID NO: 6) by use of Primescript Reverse Transcriptase II (TaKaRa). Thereafter, the cDNA was amplified by PCR (30 cycles, denaturation: 98°C, 10 seconds, annealing: 55°C, 15 seconds, extension: 68°C, 60 seconds), by use of Rluc WT EcoF01 primer (SEQ ID NO: 7) and Rluc_WT_BamR01 primer (SEQ ID NO: 6), PrimeStar GXL DNA Polymerase (TaKaRa). Next, the obtained cDNA was closed as insert DNA into a pUC19 plasmid. The insert DNA and the pUC19 plasmid were subjected to restriction enzyme digestion at 37°C for 1 hour by use of EcoRI (TaKaRa) and BamHI (TaKaRa), and then each DNA was purified by phenol/chloroform extraction and ethanol precipitation. The pUC19 plasmid and the insert DNA after the restriction enzyme digestion were mixed at a molar ratio of 1:3, and ligation reaction was performed by use of DNA Ligation Kit <Mighty Mix> (TaKaRa). The obtained ligation sample was transformed into DH5α, and cultured with an LB agar plate at 37°C overnight, and then a plasmid was extracted with QIAprep Spin Miniprep Kit (QIAGEN). The obtained plasmid DNA was subjected to base sequence analysis, and plasmid DNA (pUC19-Rluc_K41R) was obtained which had a sequence (Rluc_K41R) with A122 of Rluc_WT mutated by G and which had Rluc_K41R cloned.

With pUC19-Rluc_K41R used as a template, 1st PCR (30 cycles, denaturation: 98°C, 10 seconds, annealing: 55°C, 15 seconds, extension: 68°C, 40 seconds) was performed with PrimeStar GXL DNA Polymerase (TaKaRa), by use of Rluc_NheF01 primer (SEQ ID NO: 8) and RL_W104X_RV primer (SEQ ID NO: 9) for the 5'-side fragment, and Rluc_XhoR01 primer (SEQ ID NO: 10) and RL W104X FW primer (SEQ ID NO: 11) for the 3'-side fragment. Next, each PCR product was diluted 100-fold, subjected to 2nd PCR (30 cycles, denaturation: 98°C, 10 seconds, annealing: 55°C, 15 seconds, extension: 68°C, 60 seconds) with PrimeStar GXL DNA Polymerase (TaKaRa), by use of Rluc NheF01 primer and Rluc_XhoR01 primer, and purified by phenol/chloroform extraction and ethanol precipitation, and thus DNA was obtained which had a sequence where G311 of Rluc_K41R was mutated to A (Rluc_K41R_W104X) (SEQ ID NO: 14).

The obtained DNA (Rluc_K41R_W104X) and psiCHECK^{™}-2 Vector (promega) were subjected to restriction enzyme digestion using NheI (TaKaRa) and XhoI (TaKaRa) at 37°C for 1 hour, and then each DNA was purified by phenol/chloroform extraction and ethanol precipitation. Rluc_K41R_W104X and psiCHECK^{™}-2 Vector after the restriction enzyme digestion were mixed at a molar ratio of 3:1, and ligation reaction was performed by use of DNA Ligation Kit <Mighty Mix> (TaKaRa). The obtained ligation sample was transformed into DH5α, and cultured with an LB agar plate at 37°C overnight. Colonies selected were cultured in an LB liquid medium at 37°C overnight, and a plasmid was extracted with QIAprep Spin Miniprep Kit (QIAGEN). Thereafter, the sequence of the plasmid, obtained by base sequence analysis, was confirmed.

With the sequence-confirmed plasmid used as a template, a template DNA for *in vitro* transcription reaction was amplified by PCR (30 cycles (denaturation: 98°C, 10 seconds, annealing: 55°C, 15 seconds, extension: 68°C, 20 seconds)), by use of T7_Rluc_sRNA_F01 primer (SEQ ID NO: 12) and Rluc_sRNA_R01 primer (SEQ ID NO: 13), and PrimeStar GXL DNA Polymerase (TaKaRa), and purified by phenol/chloroform extraction and ethanol precipitation. Rluc_sRNA (SEQ ID NO: 15) was obtained by *in vitro* transcription with AmpliScribe T7 Kit (Epicentre Biotechnologies). Rluc_sRNA synthesized was subjected to cutout and purification with a 5% polyacrylamide gel containing 8 M urea, and used for the subsequent tests.

The sequences of Rluc_WT_RNA, Rluc_K41R_W104X and Rluc_sRNA, and the sequences of the primers used above are listed below. The underlines in the Tables indicate the adenosine residues to be edited.

**[Table 5]**

| | Sequence | SEQ ID NO: |
|---|---|---|
| Rluc_WT_RNA | ACCAUGGCUUCCAAGGUGUACGACCCCGAGCAACGCAAACGCAUGAUCACUGG GCCUCAGUGGUGGGCUCGCUGCAAGCAAAUGAACGUGCUGGACUCCUUCAUCA ACUACUAUGAUUCCGAGAAGCACGCCGAGAACGCCGUGAUUUUUCUGCAUGGU AACGCUGCCUCCAGCUACCUGUGGAGGCACGUCGUGCCUCACAUCGAGCCCGU GGCUAGAUGCAUCAUCCCUGAUCUGAUCGGAAUGGGUAAGUCCGGCAAGAGCG GGAAUGGCUCAUAUCGCCUCCUGGAUCACUACAAGUACCUCACCGCUUGGUUC GAGCUGCUGAACCUUCCAAAGAAAAUCAUCUUUGUGGGCCACGACUGGGGGGC UUGUCUGGCCUUUCACUACUCCUACGAGCACCAAGACAAGAUCAAGGCCAUCG UCCAUGCUGAGAGUGUCGUGGACGUGAUCGAGUCCUGGGACGAGUGGCCUGA CAUCGAGGAGGAUAUCGCCCUGAUCAAGAGCGAAGAGGGCGAGAAAAUGGUG CUUGAGAAUAACUUCUUCGUCGAGACCAUGCUCCCAAGCAAGAUCAUGCGGAA ACUGGAGCCUGAGGAGUUCGCUGCCUACCUGGAGCCAUUCAAGGAGAAGGGC GAGGUUAGACGGCCUACCCUCUCCUGGCCUCGCGAGAUCCCUCUCGUUAAGGG AGGCAAGCCCGACGUCGUCCAGAUUGUCCGCAACUACAACGCCUACCUUCGGG CCAGCGACGAUCUGCCUAAGAUGUUCAUCGAGUCCGACCCUGGGUUCUUUUCC AACGCUAUUGUCGAGGGAGCUAAGAAGUUCCCUAACACCGAGUUCGUGAAGGU GAAGGGCCUCCACUUCAGCCAGGAGGACGCUCCAGAUGAAAUGGGUAAGUACA UCAAGAGCUUCG | 5 |

**[Table 6]**

| Primer | Sequence | SEQ ID NO: |
|---|---|---|
| Rluc_WT_BamR01 | GAAGGATCCTTACAGGTCCTCCTCTGAGATC AGCTTCTGCTC | 6 |
| Rluc_WT_EcoF01 | GCAGAATTCACCATGGCTTCCAAGGTGTACG AC | 7 |
| Rluc_NheF01 | GACGCTAGCACCATGGCTTCCAAGGTGTAC GAC | 8 |
| RL_W104X_RV | CAGCTCGAACTAAGCGGTGAG | 9 |
| Rluc_XhoR01 | GAACTCGAGTTACAGGTCCTCCTCTGAGATC AGCTTCTGCTC | 10 |
| RL_W104X_FW | CTCACCGCTTAGTTCGAGCTG | 11 |
| T7_Rluc_sRNA_F01 | CTAATACGACTCACTATAGGGCTGGACTCCT TCATCAAC | 12 |
| Rluc_sRNA_R01 | CCAGTCGTGGCCCACAAAG | 13 |

**[Table 7]**

| | Sequence | SEQ ID NO: |
|---|---|---|
| Rluc_K41R_W104X | ATGGCTTCCAAGGTGTACGACCCCGAGCAACGCAAACGCATGATCACTGGGC CTCAGTGGTGGGCTCGCTGCAAGCAAATGAACGTGCTGGACTCCTTCATCAAC TACTATGATTCCGAGAGGCACGCCGAGAACGCCGTGATTTTTCTGCATGGTAA CGCTGCCTCCAGCTACCTGTGGAGGCACGTCGTGCCTCACATCGAGCCCGTG GCTAGATGCATCATCCCTGATCTGATCGGAATGGGTAAGTCCGGCAAGAGCG GGAATGGCTCATATCGCCTCCTGGATCACTACAAGTACCTCACCGCTTAGTTC GAGCTGCTGAACCTTCCAAAGAAAATCATCTTTGTGGGCCACGACTGGGGGG CTTGTCTGGCCTTTCACTACTCCTACGAGCACCAAGACAAGATCAAGGCCATC GTCCATGCTGAGAGTGTCGTGGACGTGATCGAGTCCTGGGACGAGTGGCCTG ACATCGAGGAGGATATCGCCCTGATCAAGAGCGAAGAGGGCGAGAAAATGG TGCTTGAGAATAACTTCTTCGTCGAGACCATGCTCCCAAGCAAGATCATGCGG AAACTGGAGCCTGAGGAGTTCGCTGCCTACCTGGAGCCATTCAAGGAGAAGG GCGAGGTTAGACGGCCTACCCTCTCCTGGCCTCGCGAGATCCCTCTCGTTAAG GGAGGCAAGCCCGACGTCGTCCAGATTGTCCGCAACTACAACGCCTACCTTC GGGCCAGCGACGATCTGCCTAAGATGTTCATCGAGTCCGACCCTGGGTTCTTT TCCAACGCTATTGTCGAGGGAGCTAAGAAGTTCCCTAACACCGAGTTCGTGAA GGTGAAGGGCCTCCACTTCAGCCAGGAGGACGCTCCAGATGAAATGGGTAA GTACATCAAGAGCTTCGTGGAGCGCGTGCTGAAGAACGAGCAGTAA | 14 |
| Rluc_sRNA | GGGCUGGACUCCUUCAUCAACUACUAUGAUUCCGAGAGGCACGCCGAGAA CGCCGUGAUUUUUCUGCAUGGUAACGCUGCCUCCAGCUACCUGUGGAGGC ACGUCGUGCCUCACAUCGAGCCCGUGGCUAGAUGCAUCAUCCCUGAUCUG AUCGGAAUGGGUAAGUCCGGCAAGAGCGGGAAUGGCUCAUAUCGCCUCCU GGAUCACUACAAGUACCUCACCGCUUAGUUCGAGCUGCUGAACCUUCCAAA GAAAAUCAUCUUUGUGGGCCACGACUGG | 15 |

### Cell culture

HeLa cells were subcultured on a 24-well plate at 5.0 × 10⁴ cells/well, and cultured for 48 hours. Lipofectamine 3000 (Thermo) was used for transfecting 50 ng of a plasmid (psiCHECK2_Rluc_K41R_W104X) expressing the target RNA, 350 ng of a plasmid (pcDNA3.1(-)Hygro_ADAR2, pcDNA3.1(-)Hygro_ADAR1p110 or pcDNA3.1(-)Hygro_ADAR1p150) expressing ADAR, and 10 nM of gRNA as each of the oligonucleotides of Examples 1 to 16. After the transfection, the cells were culture for 48 hours, and then recovered.

### Editing analysis method

Total RNA was extracted from the cells cultured in a 24-well plate, by use of Sepasol RNA I Super G (NACALAI TESQUE, INC.), subjected to DNase treatment with Recombinant DNase I (TaKaRa), and then purified by phenol/chloroform extraction and ethanol precipitation. cDNA was amplified by performing reverse transcription reaction using PrimeScript II Reverse Transcriptase (TaKaRa), 0.5 µg of Total RNA, and 0.25 µM Oligo(dT)17 (SEQ ID NO: 16). PrimeStar GXL DNA polymerase (TaKaRa), Rluc_F01 primer (SEQ ID NO: 17), and 3'-Adp primer (SEQ ID NO: 18) were used to perform 1st PCR (30 cycles (denaturation: 98°C, 10 seconds, annealing: 55°C, 15 seconds, extension: 68°C, 60 seconds)). With cDNA obtained by dilution of the 1st PCR product at 200-fold, used as a template, the Rluc fragment was amplified by 2nd PCR (30 cycles (denaturation: 98°C, 10 seconds, annealing: 55°C, 15 seconds, extension: 68°C, 60 seconds)), by use of PrimeStar GXL DNA polymerase (TaKaRa), Rluc_F01 primer (SEQ ID NO: 17), and Rluc_R01 primer (SEQ ID NO: 19). Big Dye Terminator v3.1 Cycle Sequence Kit (Thermo Fisher Scientific) and 0.165 µM Rluc_sRNA_F01 primer (SEQ ID NO: 20) were used to perform sequencing reaction, and analysis was performed by Applied Biosystems 3500 Genetic Analyzer (Thermo Fisher Scientific). The chromatographic chart obtained by the sequencing was adopted and thus the editing rate (%) was calculated from the peak height ratio (G/(G+A)) of the target site (A311).

**[Table 8]**

| Primer | Sequence | SEQ ID NO: |
|---|---|---|
| Oligo(dT)17 | GGCCACGCGTCGACTAGTACTTTTTTT TTTTTTTTTT | 16 |
| Rluc_F01 | CTAATACGACTCACTATAGGGACCATG GCTTCCAAGGTGTAC | 17 |
| 3'-Adp | GGCCACGCGTCGACTAGTAC | 18 |
| Rluc_R01 | TTACTGCTCGTTCTTCAGCACG | 19 |
| Rluc_sRNA_F01 | GCTGGACTCCTTCATCAAC | 20 |

### Luciferase reporter assay method

Dual-Luciferase Reporter Assay System (Promega) was used. A cell extract was obtained by use of 100 µL of Passive Lysis Buffer (Promega) for the cells cultured on the 24-well plate. One hundred µE of LARII was added to 20 µL of the obtained cell extract, and the emission intensity of Firefly luciferase (Flue) was measured after 60 seconds with GloMax(R)20/20 Luminometer (Promega). One hundred µE of Stop&Glo Reagent was added immediately thereafter, and the emission intensity of Renilla luciferase (Rluc) was measured after 60 seconds. The emission intensity was normalized by Flue.

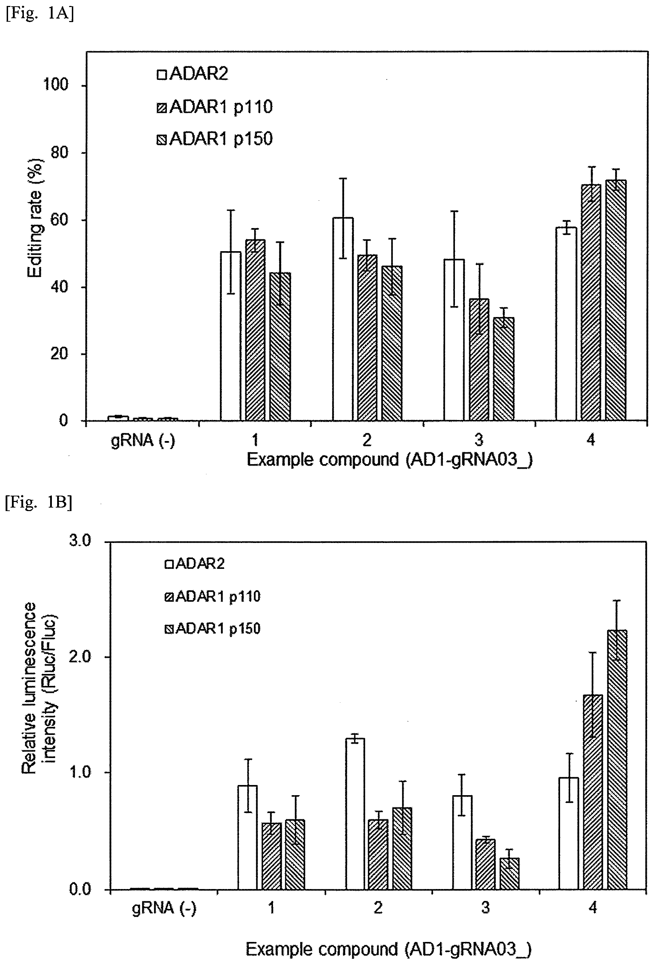

The editing rates (%) in the case of use of each of the compounds of Examples (AD1-gRNA03_1 to AD1-gRNA03_4) are shown in Fig. 1A. In the case where the plasmid expressing ADAR1 was transfected, an editing rate of about 30% to 70% was exhibited as compared with the case where no gRNA was used (gRNA(-)). In the case where the plasmid expressing ADAR2 was transfected, an editing rate of about 40% to 60% was exhibited as compared with the case where no gRNA was used (gRNA(-)). The results of luciferase reporter assay in the case of use of each of the compounds of Examples (AD1-gRNA03_1 to AD1-gRNA03_4) are shown in Fig. 1B. In the case where the compounds of Examples (AD1-gRNA03_1 to AD1-gRNA03_4) were each used, a high luciferase activity was observed as compared with the case where no gRNA was used (gRNA(-)).

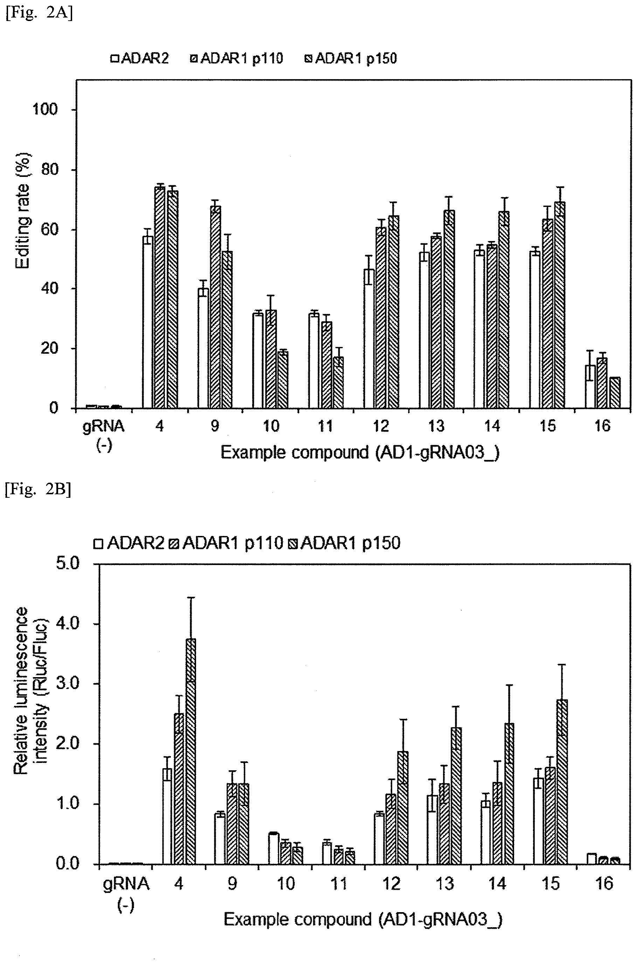

The editing rates (%) in the case of use of each of the compounds of Examples (AD1-gRNA03_4, AD1-gRNA03_9 to AD1-gRNA03_16) are shown in Fig. 2A. In the case where the plasmid expressing ADAR was transfected, AD1-gRNA03_4, AD1-gRNA03_9 to AD1-gRNA03_16 each exhibited a high editing rate as compared with the case where no gRNA was used (gRNA(-)). In the case where the compounds of Examples (AD1-gRNA03_4, AD1-gRNA03_9 to AD1-gRNA03_16) were each used, a high luciferase activity was observed as compared with the case where no gRNA was used (gRNA(-)).

### (Test Example 2)

### Evaluation of editing inducibility in cells, with endogenous ADAR

The editing inducibility in cells, of each of the compounds of Examples (AD1-gRNA03_4, AD1-gRNA03_9 to AD1-gRNA03_16), was evaluated in the same manner as described above except that no plasmid expressing ADAR was used and only 25 ng of a reporter plasmid and 50 nM of gRNA as the compound of Example were transfected.

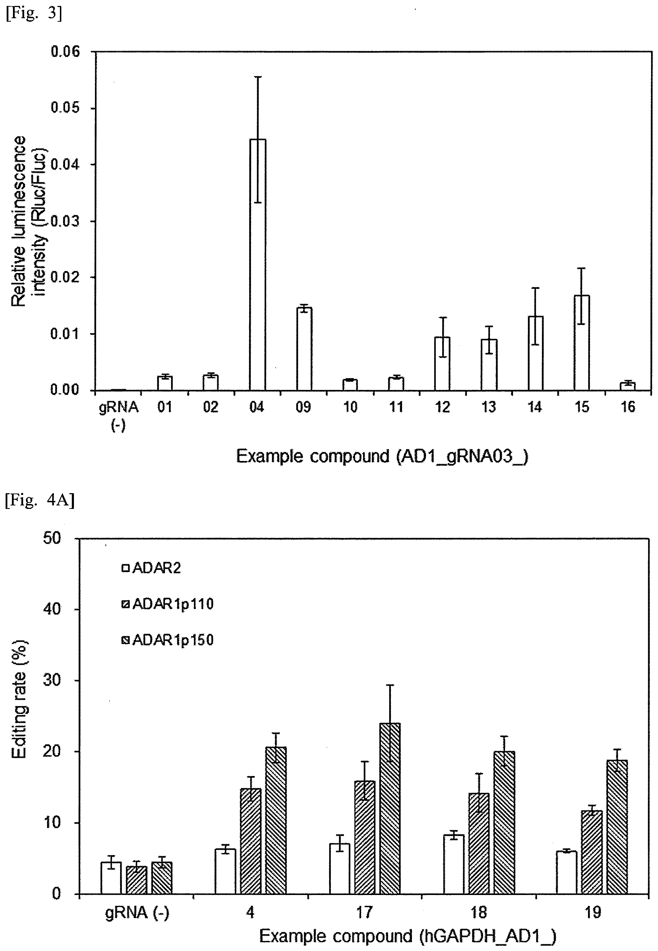

The results of luciferase reporter assay in the case of use of each of the compounds of Examples (AD1-gRNA03_4, AD1-gRNA03_9 to AD1-gRNA03_16) are shown in Fig. 3. In the case where the compounds of Examples (AD1-gRNA03_4, AD1-gRNA03_9 to AD1-gRNA03_16) were each used, an enhancement in luciferase activity was observed as compared with the case where no gRNA was added (gRNA(-)). The editing rate was also examined, and the cases of use of the compounds of Examples (AD1-gRNA03_4, AD1-gRNA03_15) were found to respectively indicate editing rates (± S.D.%) of 9.0 ± 0.1% and 4.1 ± 2.3%, and indicate higher values than the background (respectively 1.3 ± 1.4% and 0.9 ± 0.4%).

**[Table 9]**

| | Target site | | Background | |
|---|---|---|---|---|
| | ave. | S. D. | ave. | S. D. |
| gRNA(-) | 0.5 | 0.2 | 0.8 | 0.4 |
| AD1-gRNA03_4 | 9.0 | 0.1 | 1.3 | 1.4 |
| AD1-gRNA03_15 | 4.1 | 2.3 | 0.9 | 0.4 |

### (Test Example 3)

### Evaluation of editing inducibility in cells, of GAPDH gene sequence, with gRNA as compound of Example

HEK293 cells were subcultured on a 24-well plate at 5.0 × 10⁴ cells/well, and cultured for 48 hours. Lipofectamine 3000 (Thermo) was used for transfecting 500 ng of a plasmid (pcDNA3.1(-)Hygro_ADAR2,
pcDNA3.1(-)Hygro_ADAR1p110 or pcDNA3.1(-)Hygro_ADAR1p150) expressing ADAR, and 50 nM of gRNA as the compound of Example. After the transfection, the cells were cultured for 48 hours, and then recovered.

### Editing analysis method

cDNA was amplified by the same manner as in the method described as the editing analysis method of Test Example 1. The GAPDH fragment was amplified by PCR (30 cycles (denaturation: 98°C, 10 seconds, annealing: 55°C, 15 seconds, extension: 68°C, 60 seconds)), by use of PrimeStar GXL DNA polymerase (TaKaRa), GAPDH_FW primer (SEQ ID NO: 21), and GAPDH RV primer (SEQ ID NO: 22). Big Dye Terminator v3.1 Cycle Sequence Kit (Thermo Fisher Scientific) and 0.165 µM GAPDH_seqF01 primer (SEQ ID NO: 23) were used to perform sequencing reaction, and analysis was performed by Applied Biosystems 3500 Genetic Analyzer (Thermo Fisher Scientific). The chromatographic chart obtained by the sequencing was adopted and thus the editing rate (%) was calculated from the peak height ratio (G/(G+A)) of the target site.

**[Table 10]**

| Primer | Sequence | SEQ ID NO: |
|---|---|---|
| GAPDH_FW | CTCAAGATCATCAGCAATGCCTCCTGC | 21 |
| GAPDH_RV | GAGCACAGGGTACTTTATTGATGGTACATG ACAAGG | 22 |
| GAPDH_seqF01 | GTATGACAACGAATTTGGCTAC | 23 |

The editing rates (%) in the case of use of each of the compounds of Examples (hGAPDH_AD1_4, hGAPDH_AD1_17 to hGAPDH_AD1_19) are shown in Fig. 4A. In the case where the plasmid expressing ADAR1 was transfected, a high editing rate of about 15% to 25% was exhibited as compared with the case where no gRNA was used (gRNA(-)). In the case where the plasmid expressing ADAR2 was transfected, a slightly high editing rate was exhibited as compared with the case where no gRNA was used (gRNA(-)).

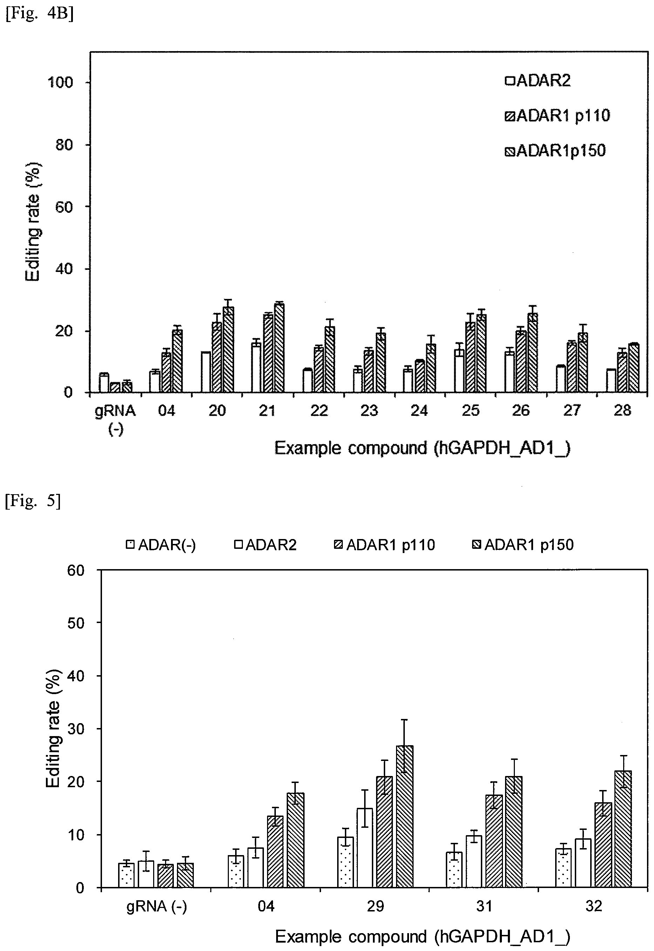

The editing rates (%) in the case of use of each of the compounds of Examples (hGAPDH_AD1_4, hGAPDH_AD1_20 to hGAPDH_AD1_28) are shown in Fig. 4B. In the case where the plasmid expressing ADAR1 was transfected, a high editing rate of about 10% to 30% was exhibited as compared with the case where no gRNA was used (gRNA(-)). In the case where the plasmid expressing ADAR2 was transfected, a high editing rate was exhibited as compared with the case where no gRNA was used.

The editing rates (%) in the case of use of each of the compounds of Examples (hGAPDH_AD1_4, hGAPDH_AD1_29, hGAPDH_AD1_31 and hGAPDH_AD1_32) are shown in Fig. 5. In the case where the plasmid expressing ADAR1 was transfected, a high editing rate of about 15% to 30% was exhibited as compared with the case where no gRNA was used (gRNA(-)). In the case where the plasmid expressing ADAR2 was transfected, a high editing rate was exhibited as compared with the case where no gRNA was used (gRNA(-)).

### (Examples 33 to 49)

Oligonucleotides of Examples 33 to 49 were obtained based on the sequence of the oligonucleotide of Reference Example 2, by introduction of modified nucleotides as shown in Table 11. These oligonucleotides were synthesized by the phosphoramidite method in the same manner as in Reference Example 1. In the sequences, when an "mU" section was synthesized, N3-Methyl Uridine CED phosphoramidite (ChemGenes, catalog number: ANP-7451) was used, when a "d" section was synthesized, dSpacer CE Phosphoramidite (Glen Research, catalog number: 10-1914) was used, and when an "me" section was synthesized, N3-Methyl deoxy Cytidine CED phosphoramidite (ChemGenes, catalog number: ANP-3851) was used. When an "mt" section was synthesized, N3-Methyl Thymidine CED phosphoramidite (ChemGenes, catalog number: ANP-6153) was used. The details of other modified nucleotides are as described above.

**[Table 11]**

| Example | Sequece name | Sequence (5'-3') | Molecular weight |
|---|---|---|---|
| 33 | hGAPDH_AD1_33 | U(M)^C(F)^A(M)^A(F)^G(M)^G(F)^G(F)^U(M)^C(M)^mU^a^C(M)^A(F)^U(M)^ G(F)^G(M)^C(F)^A(M)^A(F)^C(M)^U(F)^G(M)^U(F)^G(M) | 8250.85 |
| 34 | hGAPDH_AD1_34 | U(M)^C(F)^A(M)^A(F)^G(M)^G(F)^G(F)^U(M)^C(M)^d^a^C(M)^A(F)^U(M)^G( F)^G(M)^C(F)^A(M)^A(F)^C(M)^U(F)^G(M)^U(F)^G(M) | 8104.30 |
| 35 | hGAPDH_AD1_35 | U(M)^C(F)^A(M)^A(F)^G(M)^G(F)^G(F)^U(M)^C(M)^a^a^C(M)^A(F)^U(M)^G( F)^G(M)^C(F)^A(M)^A(F)^C(M)^U(F)^G(M)^U(F)^G(M) | 8242.77 |
| 36 | hGAPDH_AD1_36 | U(M)^C(F)^A(M)^A(F)^G(M)^G(F)^G(F)^U(M)^C(M)^g^a^C(M)^A(F)^U(M)^G( F)^G(M)^C(F)^A(M)^A(F)^C(M)^U(F)^G(M)^U(F)^G(M) | 8255.99 |
| 37 | hGAPDH_AD1_37 | U(M)^C(F)^A(M)^A(F)^G(M)^G(F)^G(F)^U(M)^C(M)^t^a^C(M)^A(F)^U(M)^G( F)^G(M)^C(F)^A(M)^A(F)^C(M)^U(F)^G(M)^U(F)^G(M) | 8232.05 |
| 38 | hGAPDH_AD1_38 | U(M)^C(M)^A(M)^A(M)^G(M)^G(F)^G(F)^U(M)^C(M)^c^a^C(M)^A(F)^U(M)^ G(F)^G(M)^C(F)^A(M)^A(F)^C(M)^U(M)^G(M)^U(M)^G(M) | 8264.13 |
| 39 | hGAPDH_AD1_39 | U(M)^C(L)^A(M)^A(L)^G(M)^G(F)^G(F)^U(M)^C(M)^c^a^C(M)^A(F)^U(M)^G( F)^G(M)^C(F)^A(M)^A(F)^C(M)^T(L)^G(M)^T(L)^G(M) | 8294.71 |
| 40 | hGAPDH_AD1_40 | T(L)^C(M)^A(L)^A(M)^G(L)^G(F)^G(F)^U(M)^C(M)^c^a^C(M)^A(F)^U(M)^G(F )^G(M)^C(F)^A(M)^A(F)^C(L)^U(M)^G(L)^U(M)^G(L) | 8276.42 |
| 41 | hGAPDH AD1_41 | U(M)^C(F)^A(M)^A(F)^G(M)^G(F)^G(F)^U(M)^C(M)^mc^a^C(M)^A(F)^U(M)^ G(F)^G(M)^C(F)^A(M)^A(F)^C(M)^U(F)^G(M)^U(F)^G(M) | 8231.57 |
| 42 | hGAPDH_AD1_43 | U(M)^C(F)^A(M)^A(F)^G(M)^G(F)^G(F)^U(M)^C(M)^mt^a^C(M)^A(F)^U(M)^ G(F)^G(M)^C(F)^A(M)^A(F)^C(M)^U(F)^G(M)^U(F)^G(M) | 8241.94 |
| 43 | hGAPDH_AD1_44 | U(M)^C(L)^A(M)^A(L)^G(M)^G(F)^G(F)^U(M)^C(M)^mc^a^C(M)^A(F)^U(M)^ G(F)^G(M)^C(F)^A(M)^A(F)^C(M)^T(L)^G(M)^T(L)^G(M) | 8315.83 |
| 44 | hGAPDH_AD1_46 | U(M)^C(L)^A(M)^A(L)^G(M)^G(F)^G(F)^U(M)^C(M)^mt^a^C(M)^A(F)^U(M)^ G(F)^G(M)^C(F)^A(M)^A(F)^C(M)^T(L)^G(M)^T(L)^G(M) | 8329.59 |
| 45 | hGAPDH_AD1_47 | U(M)^C(L)^A(M)^A(L)^G(M)^G(F)^G(F)^U(M)^C(M)^c^a^C(M)^A(F)^U(M)^G( F)^G(M)^C(F)^A(M)^A(L)^C(M)^T(L)^G(M)^T(L)^G(M) | 8306.03 |
| 46 | hGAPDH_AD1_48 | U(M)^C(L)^A(M)^A(L)^G(M)^G(L)^G(F)^U(M)^C(M)^c^a^C(M)^A(F)^U(M)^G( F)^G(M)^C(F)^A(M)^A(L)^C(M)^T(L)^G(M)^T(L)^G(M) | 8317.56 |
| 47 | hGAPDH_AD1_49 | U(M)^C(L)^A(M)^A(L)^G(M)^G(F)^G(F)^U(M)^C(M)^c^a^C(M)^A(F)^U(M)^G( F)^G(M)^C(L)^A(M)^A(L)^C(M)^T(L)^G(M)^T(L)^G(M) | 8331.43 |
| 48 | hGAPDH_AD1_50 | U(M)^C(L)^A(M)^A(L)^G(M)^G(L)^G(F)^U(M)^C(M)^c^a^C(M)^A(F)^U(M)^G( F)^G(M)^C(L)^A(M)^A(L)^C(M)^T(L)^G(M)^T(L)^G(M) | 8341.79 |
| 49 | hGAPDH_AD1_51 | U(M)^C(L)^A(M)^A(L)^G(M)^G(L)^G(L)^U(M)^C(M)^c^a^C(M)^A(F)^U(M)^G( F)^G(M)^C(L)^A(M)^A(L)^C(M)^T(L)^G(M)^T(L)^G(M) | 8351.90 |

In the "Sequence" in the Table, "mU" represents N3-methyluridine, "d" represents 1, 2-dideoxyribose, "me" represents N3-methyl-2'-deoxycytidine, and "mt" represents N3-methylthymidine. Other abbreviations are as described above. The 5'-end and the 3'-end of each oligonucleotide correspond to a hydroxyl group formed by linking of a hydrogen atom with an oxygen atom in the formulae.

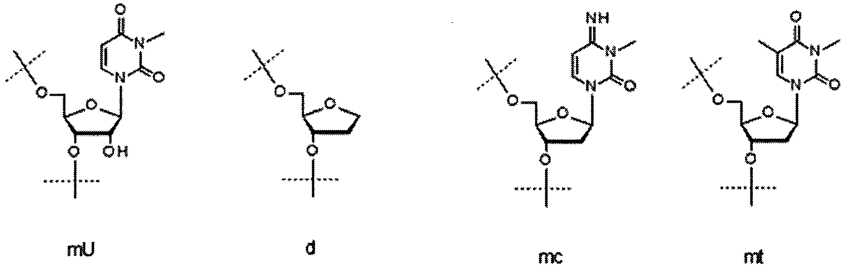

### (Reference Examples 3 to 7)

Each oligonucleotide having the following sequence and fully composed of natural RNA residues was prepared as a target-editing guide RNA targeting a disease sequence, by a phosphoramidite method. The resulting compound was identified by negative-ion ESI mass spectrometry.

**[Table 12]**

| Reference Example | Sequece name | Sequence (5'-3') | Molecular weight | SEQ ID NO: |
|---|---|---|---|---|
| 3 | AD1_AGXT | CAGAGUCCCCGAAGCCAUCAAGGG | 7707.99 | 24 |
| 4 | AD1_GNE | GCCCUUUUCCGCAUCACUCGAACC | 7478.55 | 25 |
| 5 | AD1_EYS | UGAUCCUUCCACCCAGUGGUACAA | 7568.23 | 26 |
| 6 | AD1_HFE | CUCCACUGGCACGUAUAUCUCUGC | 7522.82 | 27 |
| 7 | AD1_UGT1A1 | UAAAAUCUCCGUCUCUGAUGUACA | 7557.97 | 28 |

### (Examples 50 to 64)

Oligonucleotides of Examples 50 to 64 were obtained based on the sequences of the oligonucleotides of Reference Examples 3 to 7, by introduction of modified nucleotides as shown in Table 13. These oligonucleotides were synthesized by the phosphoramidite method in the same manner as in Reference Example 1. The details of the modified nucleotides are as described above.

**[Table 13]**

| Example | Sequece name | Sequence (5'-3') | Molecular weight |
|---|---|---|---|
| 50 | AD1_AGXT.2 | CAGAGUCCCCgAAGCCAUCAAGGG | 7699.20 |
| 51 | AD1_GNE.2 | GCCCUUUUCCgCAUCACUCGAACC | 7470.60 |
| 52 | AD1_EYS.2 | UGAUCCUUCCaCCCAGUGGUACAA | 7558.50 |
| 53 | AD1_HFE.2 | CUCCACUGGCaCGUAUAUCUCUGC | 7511.60 |
| 54 | AD1_UGT1A 1.2 | UAAAAUCUCCgUCUCUGAUGUACA | 7544.50 |
| 55 | AD1_AGXT.3 | CAGAGUCCCCiAAGCCAUCAAGGG | 7684.00 |
| 56 | AD1_GNE.3 | GCCCUUUUCCiCAUCACUCGAACC | 7455.40 |
| 57 | AD1_EYS.3 | UGAUCCUUCCiCCCAGUGGUACAA | 7559.50 |
| 58 | AD1_HFE.3 | CUCCACUGGCiCGUAUAUCUCUGC | 7512.40 |
| 59 | AD1_UGT1A1.3 | UAAAAUCUCCiUCUCUGAUGUACA | 7529.30 |
| 60 | AD1_AGXT_30 | C(M)^A(F)^G(M)^A(F)^G(M)^U(F)^C(F)^C(M)^C(M)^C^i^A(M)^A(F)^G(M)^C( F)^C(M)^A(F)^U(M)^C(F)^A(M)^A(F)^G(M)^G(F)^G(M) | 8242.29 |
| 61 | AD1_GNE_30 | G(M)^C(F)^C(M)^C(F)^U(M)^U(F)^U(F)^U(M)^C(M)^C^i^C(M)^A(F)^U(M)^C( F)^A(M)^C(F)^U(M)^C(F)^G(M)^A(F)^A(M)^C(F)^C(M) | 8010.77 |
| 62 | AD1_EYS_29 | U(M)^G(F)^A(M)^U(F)^C(M)^C(F)^U(F)^U(M)^C(M)^C^a^C(M)^C(F)^C(M)^A( F)^G(M)^U(F)^G(M)^G(F)^U(M)^A(F)^C(M)^A(F)^A(M) | 8116.86 |
| 63 | AD1_HFE_29 | C(M)^U(F)^C(M)^C(F)^A(M)^C(F)^U(F)^G(M)^G(M)^C^a^C(M)^G(F)^U(M)^A( F)^U(M)^A(F)^U(M)^C(F)^U(M)^C(F)^U(M)^G(F)^C(M) | 8067.40 |
| 64 | AD1_UGT1A1_30 | U(M)^A(F)^A(M)^A(F)^A(M)^U(F)^C(F)^U(M)^C(M)^C^i^U(M)^C(F)^U(M)^C( F)^U(M)^G(F)^A(M)^U(F)^G(M)^U(F)^A(M)^C(F)^A(M) | 8080.60 |

Abbreviations in the "Sequence" in the Table are as described above. The 5'-end and the 3'-end of each oligonucleotide correspond to a hydroxyl group formed by linking of a hydrogen atom with an oxygen atom in the formulae.

### (Test Example 4)

The compounds obtained in Examples 33 to 49 were each evaluated with respect to the editing induction activity in cells, of the hGAPDH gene sequence, with the oligonucleotide, in the same manner as in Test Example 3.

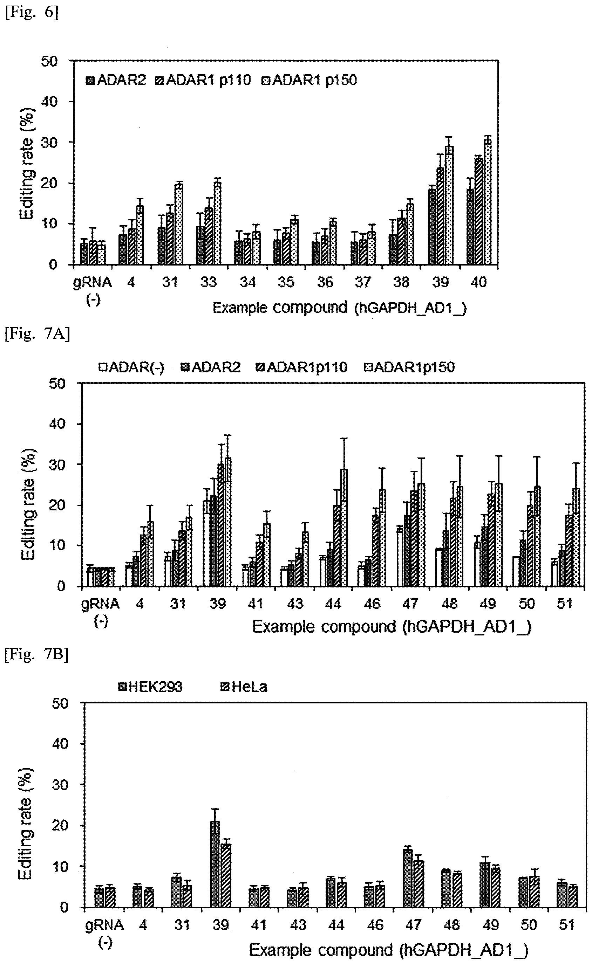

The editing rates (%) in the case of use of each of the compounds of Examples (hGAPDH_AD1_4, HGAPDH_AD1_31, hGAPDH_AD1_33 and hGAPDH_AD1_40) are shown in Fig. 6. In the case where the plasmid expressing ADAR1 was transfected, a high editing rate was exhibited as compared with the case where no gRNA was used (in the drawing, gRNA(-)). In the case where the plasmid expressing ADAR2 was transfected, a high editing rate was exhibited as compared with the case where no gRNA was used (in the drawing, gRNA(-)). In particular, hGAPDH_AD1_33 of Example 33, in which the target-corresponding nucleotide residue was mU, exhibited a high editing rate. Example 39 and Example 40 (hGAPDH_AD1_39, hGAPDH_AD1_40), in which LNA was introduced at four or six positions to the linking site with the target RNA, each exhibited the highest editing rate.

Furthermore, the editing induction in cells in the case of no transfection of any plasmid expressing ADAR1 or ADAR2 and transfection of only gRNA as the compound of Example at a concentration of 50 nM was evaluated, and the cases of use of the compounds of Examples (hGAPDH_AD1_39 and hGAPDH_AD1_40) were found to respectively indicate editing rates (± S.D.%) of 13.6 ± 1.7% and 13.9±1.6% in HeLa cells, and indicate clearly higher editing rates than the background (5.0 ± 0.9%). The cases of use of the compounds of Examples (hGAPDH_AD1_39 and hGAPDH_AD1_40) were found to respectively indicate editing rates (± S.D.%) of 16.1 ± 3.9% and 16.2 ± 1.0% in HEK293 cells, and indicate clearly higher editing rates than the background (4.4 ± 0.7%).

The editing rates (%) in the case of use of each of the compounds of Examples (hGAPDH_AD1_4, hGAPDH_AD1_31, hGAPDH_AD1_39, hGAPDH_AD1_41, hGAPDH_AD1_43, hGAPDH_AD1_44, hGAPDH_AD1_46, and hGAPDH_AD1_51) are shown in Fig. 7A. In the case where the plasmid expressing ADAR1 was transfected, a high editing rate was exhibited as compared with the case where no gRNA was used (in the drawing, gRNA(-)). In the case where the plasmid expressing ADAR2 was transfected, a high editing rate was exhibited as compared with the case where no gRNA was used (in the drawing, gRNA(-)). In particular, Examples 39 and 43 to 49(hGAPDH_AD1_39, hGAPDH_AD1_44, and hGAPDH_ADl_46 to hGAPDH_AD1_51) where LNA was introduced to the linking site with the target RNA exhibited a high editing rate.

Furthermore, the editing rates (%) in evaluation of the editing induction in cells in the case of no transfection of any plasmid expressing ADAR1 or ADAR2 and transfection of only gRNA as the compound of Example at a concentration of 50 nM are shown in Fig. 7B. The cases of use of the compounds of Examples (hGAPDH_AD1_31, hGAPDH_AD1_39, hGAPDH_AD1_44, HGAPDH_AD1_47 to hGAPDH_AD1_51) indicated clearly higher editing rates in HEK293 cells or HeLa cells, than the background.

### (Test Example 5)

### Evaluation of editing inducibility of model target RNA having AGXT gene sequence

### (A) Synthesis of AGXT_RNA

Oligo DNAs of AGXT_FW (SEQ ID NO: 29) and AGXT_RV (SEQ ID NO: 30) and NEBuffer2 (NEB) were used to perform an annealing reaction (heating at 80°C for 3 min and cooling to 25°C over 15 min), and thus an insert was prepared. pUC19 was subjected to reaction by use of EcoRI (TaKaRa) and HindIII (TaKaRa) at 37°C for 1 hour, and then purified by phenol/chloroform extraction and ethanol precipitation. The insert and plasmid were added at a molar ratio of 3:1, and ligation reaction was performed by use of DNA Ligation Kit<Mighty Mix> (TaKaRa). The obtained ligation sample was transformed into DH5α, and cultured with an LB agar plate at 37°C overnight. Colonies selected were cultured in an LB liquid medium at 37°C overnight, and the plasmid was extracted with QIAprep Spin Miniprep Kit (QIAGEN). One hundred ng of the obtained plasmid was subjected to sequencing reaction using Big Dye Terminator v3.1 Cycle Sequence Kit (Thermo Fisher Scientific), 0.165 µM of pUC19_seqFW primer (SEQ ID NO: 31) and 0.165 µM of pUC19_seqRV primer (SEQ ID NO: 32), and it was confirmed by sequence analysis with Applied Biosystems 3500 Genetic Analyzer (Thermo Fisher Scientific) that a pUC19_AGXT plasmid was synthesized. With pUC19_AGXT used as a template, a template DNA was produced by PCR (denaturation: 98°C, 10 seconds, annealing: 55°C, 15 seconds, extension: 68°C, 20 seconds), by use of T7_pUC19_FW (SEQ ID NO: 33) and M13_RV primer (SEQ ID NO: 34), and PrimeStar GXL DNA Polymerase (TaKaRa), and purified by phenol/chloroform extraction and ethanol precipitation. AGXT_RNA was synthesized as a model target RNA by *in vitro* transcription with AmpliScribe T7 Kit (Epicentre Biotechnologies) and cutout and purification with a 5% polyacrylamide gel containing 8 M urea.

**[Table 14]**

| Primer | Sequence | SEQ ID NO: |
|---|---|---|
| AGXT_FW | AATTCCGTGCTGCAGCCCCTTGATGGCTTC AGGGAACTCTGCCACAGGTGAGCCTGA | 29 |
| AGXT_RV | AGCTTCAGGCTCACCTGTGGCAGAGTTCCC TGAAGCCATCAAGGGGCTGCAGCACGG | 30 |
| pUC19_seqFW | CAACTGTTGGGAAGGGCGATC | 31 |
| pUC19_seqRV | CGACAGGTTTCCCGACTGGAAAG | 32 |
| T7_pUC19_FW | CTAATACGACTCACTATAGGGCCTCTTCGC TATTACGCCAG | 33 |
| M13_RV | GGAAACAGCTATGACCATGATTAC | 34 |
| T7proGGG | CTAATACGACTCACTATAGGG | 35 |

### (B) Annealing reaction

In an annealing buffer (10 mM Tri-HCl (pH 7.6), 150 mM NaCl), 0.3 µM of AGXT_RNA, and 0.9 µM of the compounds of Examples 50 and 55 (AD1_AGXT.2, AD1_AGXT.3) and Reference Example 3 (AD1_AGXT) (hereinafter, sometimes simply designated as "gRNA") were heated at 80°C for 3 min, and cooled to 25°C over 15 min.

### (C) In vitro editing reaction

Under the assumption that AGXT_RNA was taken with gRNA to completely form a complex by the annealing reaction, the following concentration of the AGXT_RNA-gRNA complex was calculated. The editing reaction using ADAR2 was performed by addition of recombinant hADAR2 at a final concentration of 12.5 nM and incubation at 37°C for 30 minutes, in an editing reaction buffer (20 mM HEPES-KOH (pH 7.5), 2 mM MgCl₂, 100 mM NaCl, 0.5 mM DTT, 0.01% TritonX-100, 5% glycerol), to a 5 nM AGXT_RNA-gRNA complex. The editing reaction using ADAR1 was performed by addition of recombinant hADAR1p110 at a final concentration of 250 nM to a 5 nM AGXT_RNA-gRNA complex and incubation at 37°C for 30 minutes, in an editing reaction buffer (20 mM HEPES-KOH (pH 7.5), 2 mM MgCl₂, 100 mM NaCl, 0.5 mM DTT, 0.01% TritonX-100, 5% glycerol).

### (D) Editing analysis method

AGXT_RNA after the editing reaction was purified by phenol/chloroform extraction and ethanol precipitation, and cDNA was synthesized by M13_RV primer (SEQ ID NO: 34) by use of Primescript Reverse Transcriptase II (TaKaRa). Thereafter, the cDNA was amplified by PCR (denaturation: 98°C, 10 seconds, annealing: 55°C, 15 seconds, extension: 68°C, 20 seconds), by use of T7_pUC19_FW primer (SEQ ID NO: 33) and M13_RV primer (SEQ ID NO: 34), and PrimeStar GXL DNA Polymerase (TaKaRa). The sequence analysis of the obtained cDNA was conducted by sequencing reaction using T7proGGG primer (SEQ ID NO: 35) and Big Dye Terminator v3.1 Cycle Sequence Kit, and analysis by Applied Biosystem 3500 Genetic Analyzer (Thermo Fisher Scientific). The chromatographic chart obtained by the sequencing was adopted and thus the editing rate (%) was calculated from the peak height ratio (G/(G+A)) of the target site.

### (E) Results of editing analysis

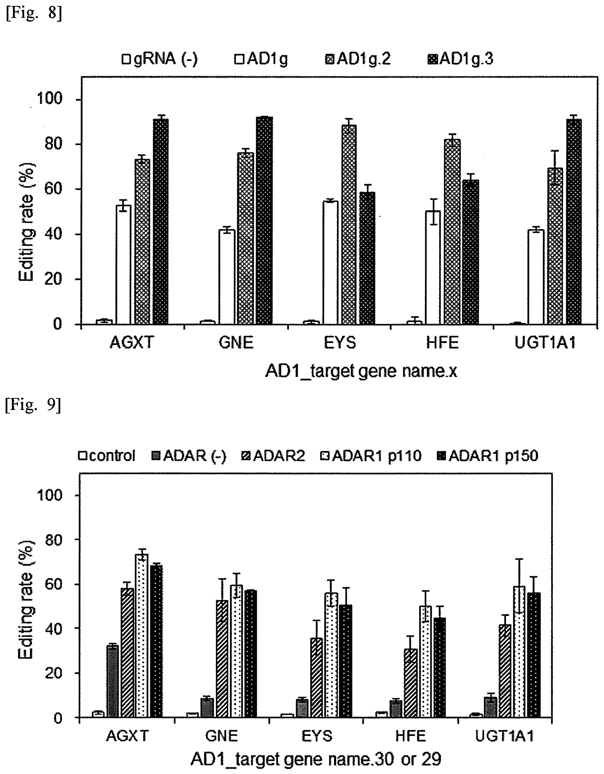

The results of the editing rate are shown in Fig. 8. The editing efficiency of RNA with ADAR1p110 was exhibited as a high editing rate in Example 50 (AD1_AGXT.2, in the drawing, AD1g.2 of AGXT) in which guanosine at the 3'-side of the target-corresponding nucleotide was 2'-deoxyguanosine in gRNA, as compared with Reference Example 3 (AD1_AGXT, in the drawing, ADlg of AGXT) in which the entire was composed of natural RNA. Example 55 (AD1_AGXT.3, in the drawing, AD1g.3 of AGXT) in which guanosine at the 3'-side of the target-corresponding nucleotide was 2'-deoxyinosine exhibited a higher editing rate. This indicates that, in the case where the 5'-side of the adenosine residue to be edited in the target RNA corresponds to cytidine, a high editing rate is exhibited by use of 2'-deoxyinosine as the nucleotide residue at the 3'-side of the target-corresponding nucleotide in gRNA.

### (Test Example 6)

### Evaluation of editing inducibility of model target RNA having GNE gene sequence

### (A) Synthesis of GNE_RNA

pUC_GNE plasmid expressing a model target RNA was synthesized in the same manner as in the method described in (A) Synthesis of AGXT RNA in Test Example 5 except that the following GNE_FW (SEQ ID NO: 36) and GNE_RV (SEQ ID NO: 37) were used as oligo DNAs instead of AGXT_FW and AGXT_RV, and the plasmid was used to synthesize GNE_RNA as a model target RNA.

**[Table 15]**

| | Sequence | SEQ ID NO: |
|---|---|---|
| GNE_FW | AATTCAGCAAAGAGATGGTTCGAGTGATG CAGAAGAAGGGCATTGAGCATCATCCCA | 36 |
| GNE_RV | AGCTTGGGATGATGCTCAATGCCCTTCTTCT GCATCACTCGAACCATCTCTTTGCTG | 37 |

### (B) Evaluation of editing inducibility and results thereof

The editing inducibility was evaluated with the compounds of Examples 51 and 56 (AD1_GNE.2, AD1_GNE.3) and Reference Example 4 (AD1_GNE), by the same method as in (B) to (D) of Test Example 5.

### (C) Results of editing analysis

The results of the editing rate are shown in Fig. 8. The editing efficiency of RNA with ADAR1p110 was exhibited as a high editing rate in Example 51 (AD1_GNE.2, in the drawing, AD1g.2 of GNE) in which guanosine at the 3'-side of the target-corresponding nucleotide was 2'-deoxyguanosine in gRNA, as compared with Reference Example 4 (AD1_GNE, in the drawing, AD1g of GNE) in which the entire was composed of natural RNA. Example 56 (AD1_GNE.3, in the drawing, AD1g.3 of GNE) in which guanosine at the 3'-side of the target-corresponding nucleotide position was 2'-deoxyinosine exhibited a higher editing rate. This indicates that, in the case where the 5'-side of the adenosine residue to be edited in the target RNA corresponds to cytidine, a high editing rate is exhibited by use of 2'-deoxyinosine as the nucleotide residue at the 3'-side of the target-corresponding nucleotide in gRNA.

### (Test Example 7)

### Evaluation of editing inducibility of model target RNA having EYS gene sequence

### (A) Synthesis of EYS_RNA

A pUC_EYS plasmid expressing a model target RNA was synthesized in the same manner as in the method described in (A) Synthesis of AGXT_RNA in Test Example 5 except that the following EYS_FW (SEQ ID NO: 38) and EYS_RV (SEQ ID NO: 39) were used as oligo DNAs instead of AGXT_FW and AGXT_RV, and the plasmid was used to synthesize EYS_RNA as a model target RNA.

**[Table16]**

| | Sequence | SEQ ID NO: |
|---|---|---|
| EYS_FW | AATTCCACAGCTGCAATTGTACCACTGGGT AGAAAGGATCATTCTGCACAGAGCACA | 38 |
| EYS_RV | AGCTTGTGCTCTGTGCAGAATGATCCTTTCT ACCCAGTGGTACAATTGCAGCTGTGG | 39 |

### (B) Evaluation of editing inducibility and results thereof

The editing inducibility was evaluated with the compounds of Examples 52 and 57 (AD1_EYS.2, AD1_EYS.3) and Reference Example 5 (AD1_EYS), by the same method as in (B) to (D) of Test Example 5.

### (C) Results of editing analysis

The results of the editing rate are shown in Fig. 8. The editing efficiency of RNA with ADAR1p110 was exhibited as a high editing rate in Example 52 (AD1_EYS.2, in the drawing, AD1g.2 of EYS) in which guanosine at the 3'-side of the target-corresponding nucleotide was 2'-deoxyguanosine in gRNA, as compared with Reference Example 5 (AD1_EYS, in the drawing, AD1g of EYS) in which the entire was composed of natural ga-type RNA. Example 57 (AD1_EYS.3, in the drawing, AD1g.3 of EYS) in which guanosine at the 3'-side of the target-corresponding nucleotide position was 2'-deoxyinosine exhibited a higher editing rate. This indicates that, in the case where the 5'-side of the adenosine residue to be edited in the target RNA corresponds to cytidine, a high editing rate is exhibited by use of 2'-deoxyinosine as the nucleotide residue at the 3'-side of the target-corresponding nucleotide in gRNA.

### (Test Example 8)

### Evaluation of editing inducibility of model target RNA having HFE gene sequence

### (A) Synthesis of HFE_RNA

A pUC_HFE plasmid expressing a model target RNA was synthesized in the same manner as in the method described in (A) Synthesis of AGXT_RNA in Test Example 5 except that the following HFE_FW (SEQ ID NO: 40) and HFE_RV (SEQ ID NO: 41) were used as oligo DNAs instead of AGXT_FW and AGXT_RV, and the plasmid was used to synthesize HFE_RNA as a model target RNA.

**[Table 17]**

| | Sequence | SEQ ID NO: |
|---|---|---|
| HFE_FW | AATTCCCTGGGGAAGAGCAGAGATATACG TACCAGGTGGAGCACCCAGGCCTGGATA | 40 |
| HFE_RV | AGCTTATCCAGGCCTGGGTGCTCCACCTG GTACGTATATCTCTGCTCTTCCCCAGGG | 41 |

### (B) Evaluation of editing inducibility and results thereof

The editing inducibility was evaluated with the compounds of Examples 53 and 58 (AD1_HFE.2, AD1_HFE.3) and Reference Example 6 (AD1_HFE), by the same method as in (B) to (D) of Test Example 5.

### (C) Results of editing analysis

The results of the editing rate are shown in Fig. 8. The editing efficiency of RNA with ADAR1p110 was exhibited as a high editing rate in Example 53 (AD1_HFE.2, in the drawing, AD1g.2 of HFE) in which guanosine at the 3'-side of the target-corresponding nucleotide was 2'-deoxyguanosine in gRNA, as compared with Reference Example 6 (AD1_HFE, in the drawing, ADlg of HFE) in which the entire was composed of natural RNA. Example 58 (AD1_HFE.3, in the drawing, AD1g.3 of HFE) in which guanosine at the 3'-side of the target-corresponding nucleotide position was 2'-deoxyinosine exhibited a higher editing rate. This indicates that, in the case where the 5'-side of the adenosine residue to be edited in the target RNA corresponds to cytidine, a high editing rate is exhibited by use of 2'-deoxyinosine as the nucleotide residue at the 3'-side of the target-corresponding nucleotide in gRNA.

### (Test Example 9)

### Evaluation of editing inducibility of model target RNA having UGT1A1 gene sequence

(A) Synthesis of UGT1A1_RNA

A pUC_UGT1A1 plasmid expressing a model target RNA was synthesized in the same manner as in the method described in (A) Synthesis of AGXT_RNA in Test Example 5 except that the following UGT1A1_FW (SEQ ID NO: 42) and UGT1A1_RV (SEQ ID NO: 43) were used as oligo DNAs instead of AGXT_FW and AGXT_RV, and the plasmid was used to synthesize UGT1A1_RNA as a model target RNA.

**[Table 18]**

| | Sequence | SEQ ID NO: |
|---|---|---|
| UGT1A1_FW | AATTCTGACGCCTCGTTGTACATCAGAGAC AGAGCATTTTACACCTTGAAGACGTAA | 42 |
| UGT1A1_RV | AGCTTTACGTCTTCAAGGTGTAAAATGCTCT GTCTCTGATGTACAACGAGGCGTCAG | 43 |

### (B) Evaluation of editing inducibility and results thereof

The editing inducibility was evaluated with the compounds of Examples 54 and 59 (AD1_UGT1A1.2, AD1_UGT1A1.3) and Reference Example 7 (AD1_UGT1A1), by the same method as in (B) to (D) of Test Example 5.

### (C) Results of editing analysis

The results of the editing rate are shown in Fig. 8. The editing efficiency of RNA with ADAR1p110 was exhibited as a high editing rate in Example 54 (AD1_UGT1A1.2, in the drawing, AD1g.2 of UGT1A1) in which guanosine at the 3'-side of the target-corresponding nucleotide was 2'-deoxyguanosine in gRNA, as compared with Reference Example 7 (AD1_UGT1A1, in the drawing, ADlg of UGT1A1) in which the entire was composed of natural RNA. Example 59 (AD1_UGT1A1.3, in the drawing, AD1g.3 of UGT1A1) in which guanosine at the 3'-side of the target-corresponding nucleotide position was 2'-deoxyinosine exhibited a higher editing rate. This indicates that, in the case where the 5'-side of the adenosine residue to be edited in the target RNA corresponds to cytidine, a high editing rate is exhibited by use of 2'-deoxyinosine as the nucleotide residue at the 3'-side of the target-corresponding nucleotide in gRNA.

### (Test Example 10)

### Evaluation of editing inducibility of model target RNA having AGXT gene sequence, in cultured cells, with gRNA

### (A) Synthesis of target RNA expression plasmid

With pUC19_AGXT obtained in (A) of Test Example 5, used as a template, an insert DNA was produced by PCR (30 cycles, denaturation: 98°C, 10 seconds, annealing: 55°C, 15 seconds, extension: 68°C, 20 seconds) by use of DS_disease_XhoF01 primer (SEQ ID NO: 44) and DS_disease_KpnR01 primer (SEQ ID NO: 45), and PrimeStar GXL DNA Polymerase (TaKaRa), and purified by phenol/chloroform extraction and ethanol precipitation. The insert DNA and pcDNA3.1(-)Hygro were subjected to reaction by use of XhoI (TaKaRa) and KpnI (TaKaRa) at 37°C for 1 hour, and then purified by phenol/chloroform extraction and ethanol precipitation. The insert and plasmid were added at a molar ratio of 3:1, and ligation reaction was performed by use of DNA Ligation Kit<Mighty Mix> (TaKaRa). The obtained ligation sample was transformed into DH5α, and cultured with an LB agar plate at 37°C overnight. Colonies selected were cultured in an LB liquid medium at 37°C overnight, and the plasmid was extracted with QIAprep Spin Miniprep Kit (QIAGEN). One hundred ng of the obtained plasmid was subjected to sequencing reaction using Big Dye Terminator v3.1 Cycle Sequence Kit (Thermo Fisher Scientific), 0.165 µM of pcDNA3_1pro_F01 primer (SEQ ID NO: 46) and 0.165 µM of pcDNA31-seqR01 primer (SEQ ID NO: 47), and it was confirmed by sequence analysis with Applied Biosystems 3500 Genetic Analyzer (Thermo Fisher Scientific) that pcDNA3.1(-)Hygro_AGXT was synthesized.

**[Table 19]**

| Primer | Sequence | SEQ ID NO: |
|---|---|---|
| DS_disease_XhoF01 | GTACTCGAGGGGCCTCTTCGCTATTACGCC AG | 44 |
| DS_disease_KpnR01 | GATGGTACCGGAAACAGCTATGACCATG | 45 |
| pcONA3_1pro_F01 | TGGCACCAAAATCAACGGG | 46 |
| pcDNA31-seqR01 | GCTATTGTCTTCCCAATCCTCC | 47 |
| 3-Adp | GGCCACGCGTCGACTAGTAC | 48 |

### (B) Cell culture

HEK293 cells were seeded on a 24-well plate at 5.0 × 10⁴ cells/well, and cultured for 48 hours. Lipofectamine 3000 (Thermo) was used for transfecting 10 ng of pcDNA3.1 (-)Hygro_AGXT, 500 ng of a plasmid (pcDNA3.1(-)Hygro_ADAR2, pcDNA3.1(-)Hygro_ADAR1p110, or pcDNA3.1(-)Hygro_ADAR1p150) expressing ADAR, and 50 nM gRNA(Example 60; AD1_AGXT.30), and culture was conducted for 48 hours.

### (C) Editing analysis

Total RNA was extracted from the cells cultured in a 24-well plate, by use of Sepasol RNA I Super G (NACALAI TESQUE, INC.), subjected to DNase treatment with Recombinant DNase I (TaKaRa), and then purified by phenol/chloroform extraction and ethanol precipitation. cDNA was amplified by performing reverse transcription reaction using PrimeScript II Reverse Transcriptase (TaKaRa), 0.5 µg of Total RNA, and 0.25 µM Oligo(dT)17. PrimeStar GXL DNA polymerase (TaKaRa), T7proGGG primer (SEQ ID NO: 35), and 3'-Adp primer (SEQ ID NO: 48) were used to perform 1st PCR. With cDNA obtained by dilution of the 1st PCR product at 200-fold, used as a template, the AGXT fragment was amplified by 2nd PCR by use of PrimeStar GXL DNA polymerase (TaKaRa), DS_disease_XhoF01 primer (SEQ ID NO: 44), and DS_disease_KpnR01 primer (SEQ ID NO: 45). Big Dye Terminator v3.1 Cycle Sequence Kit, and 0.165 µM DS_disease_XhoF01 primer were used to perform sequencing reaction, and analysis was performed by Applied Biosystem 3500 Genetic Analyzer. The chromatographic chart obtained by the sequencing was adopted and thus the editing rate was calculated from the peak height ratio (G/(G+A)) of the target site.

### (D) Results of editing analysis

The editing rates (%) in the case of use of the compound of Example 60 (AD1_AGXT_30) are shown in Fig. 9. In the case where the target RNA expression plasmid and gRNA as the compound of Example were transfected, a high editing rate of about 30% was exhibited. In the case where the target RNA expression plasmid and gRNA as the compound of Example were transfected together with the plasmid expressing ADAR2, a high editing rate of about 60% was exhibited. In the case where the target RNA expression plasmid and gRNA as the compound of Example were transfected together with the plasmid expressing ADAR1p110 or ADAR1p150, a high editing rate of about 70% was exhibited.

### (Test Example 11)

### Evaluation of editing inducibility of model target RNA having GNE gene sequence, in cultured cells, with gRNA

The editing inducibility of the GNE gene sequence, in cultured cells, with gRNA, was evaluated according to (A) to (C) of Test Example 10 except that the target RNA expression plasmid was constructed with pUC19_GNE obtained in (A) of Test Example 6, used as a template instead of pUC19_AGXT, and AD1_GNE_30 of Example 61 was used as gRNA.

The editing rates (%) in the case of use of the compound of Example 61 (AD1_GNE_30) are shown in Fig. 9. In the case where the target RNA expression plasmid and gRNA as the compound of Example were transfected, an editing rate of about 10% was exhibited. In the case where the target RNA expression plasmid and gRNA as the compound of Example were transfected together with the plasmid expressing ADAR2, a high editing rate of about 50% was exhibited. In the case where the target RNA expression plasmid and gRNA as the compound of Example were transfected together with the plasmid expressing ADAR1p110 or ADAR1p150, a high editing rate of about 60% was exhibited.

### (Test Example 12)

### Evaluation of editing inducibility of model target RNA having EYS gene sequence, in cultured cells, with gRNA

The editing inducibility of the EYS gene sequence, in cultured cells, with gRNA, was evaluated according to (A) to (C) of Test Example 10 except that the target RNA expression plasmid was constructed with pUC19_EYS obtained in (A) of Test Example 7, used as a template instead of pUC19_AGXT, and AD1_EYS_29 of Example 62 was used as gRNA.

The editing rates (%) in the case of use of the compound of Example 62 (AD1_EYS_29) are shown in Fig. 9. In the case where the target RNA expression plasmid and gRNA as the compound of Example were transfected, an editing rate of about 10% was exhibited. In the case where the target RNA expression plasmid and gRNA as the compound of Example were transfected together with the plasmid expressing ADAR2, a high editing rate of about 40% was exhibited. In the case where the target RNA expression plasmid and gRNA as the compound of Example were transfected together with the plasmid expressing ADAR1p110 or ADAR1p150, a high editing rate of about 50% to 60% was exhibited.

### (Test Example 13)

### Evaluation of editing inducibility of model target RNA having HFE gene sequence, in cultured cells, with gRNA

The editing inducibility of the HFE gene sequence, in cultured cells, with gRNA, was evaluated according to (A) to (C) of Test Example 10 except that the target RNA expression plasmid was constructed with pUC19_HFE obtained in (A) of Test Example 8, used as a template instead of pUC19_AGXT, and AD1_HFE_29 of Example 63 was used as gRNA.

The editing rates (%) in the case of use of the compound of Example 63 (AD1_HFE_29) are shown in Fig. 9. In the case where the target RNA expression plasmid and gRNA as the compound of Example were transfected, an editing rate of about 10% was exhibited. In the case where the target RNA expression plasmid and gRNA as the compound of Example were transfected together with the plasmid expressing ADAR2, a high editing rate of about 40% was exhibited. In the case where the target RNA expression plasmid and gRNA as the compound of Example were transfected together with the plasmid expressing ADAR1p110 or ADAR1p150, a high editing rate of about 50% was exhibited.

### (Test Example 14)

### Evaluation of editing inducibility of model target RNA having UGT1A1 gene sequence, in cultured cells, with gRNA

The editing inducibility of the UGT1A1 gene sequence, in cultured cells, with gRNA, was evaluated according to (A) to (C) of Test Example 10 except that the target RNA expression plasmid was constructed with pUC19_UGT1A1 obtained in (A) of Test Example 9, used as a template instead of pUC19_AGXT, and AD1_UGT1A1_30 of Example 64 was used as gRNA.

The editing rates (%) in the case of use of the compound of Example 64 (AD1_ UGT1A1 _30) are shown in Fig. 9. In the case where the target RNA expression plasmid and gRNA as the compound of Example were transfected, an editing rate of about 10% was exhibited. In the case where the target RNA expression plasmid and gRNA as the compound of Example were transfected together with the plasmid expressing ADAR2, a high editing rate of about 40% was exhibited. In the case where the target RNA expression plasmid and gRNA as the compound of Example were transfected together with the plasmid expressing ADAR1p110 or ADAR1p150, a high editing rate of about 60% was exhibited.

### (Reference Examples 8 to 22)

Each oligonucleotide having the following sequence and entirely composed of natural ribonucleotide residues was synthesized as a target-editing guide RNA targeting a disease sequence, by a phosphoramidite method as in Reference Example 1. In the Table, the "Molecular weight" represents a measured value by negative-ion ESI mass spectrometry and "-" represents no measurement.

**[Table 20]**

| Reference Example | Sequece name | Sequence (5'-3) | Molecular weight | SEQ ID NO: |
|---|---|---|---|---|
| 8 | AD1_Factor V | CUGUAUCCUCGCCUGUCCAGGGAU | 7576.65 | 49 |
| 9 | AD1_PAH.1 | GUGGAAACUCGGAAGGCCAGGCCA | 7788.98 | 50 |
| 10 | AD1_ASS1 | UUGAUGACCCGGUGGCAUCAGUUG | 7680.78 | 51 |
| 11 | AD1_GFAP | AUACUGGUGCGGAUCUCUUUCAGG | 7642.68 | 52 |
| 12 | AD1_CBS | GGCGCCGGUCGAAGCCCUUCUCCC | 7596.00 | 53 |
| 13 | AD1_GRIA2 | GCAUCCUGCCGCAUAAAGGCACCC | 7611.00 | 54 |
| 14 | AD1_SLCO2A1 | GGGUCAUUACGUUUAAUGAAAUCC | 7640.00 | 55 |
| 15 | AD1g_RL_A287_GC | CUUGUAUGACCCAGGAGGCGAUAU | 7688.30 | 56 |
| 16 | AD1g_RL_A287_GG | CUUGUAUGACGCAGGAGGCGAUAU | 7733.63 | 57 |
| 17 | AD1g_RL_A287_U | CUUGUAUGACUCAGGAGGCGAUAU | 7689.64 | 58 |
| 18 | AD1g_RL_A287_A | CUUGUAUGACACAGGAGGCGAUAU | 7715.62 | 59 |
| 19 | AD1_PAH.2 | CUGUAGCCCCAAGUGAAAAGUUAU | 7661.70 | 60 |
| 20 | AD1_PANK2 | AAGAAAUUCCAACAAAUACCACCU | - | 61 |
| 21 | AD1_NPHS2 | AUGUCCAGUCGGAAUAUAAUUACU | 7624.00 | 62 |
| 22 | AD1_A1AT | GUCCCUUCUCGUCGAUGGUCAGCA | - | 63 |

In the "Sequence" in the Table, a capital letter represents an RNA. The 5'-end and the 3'-end of each of the oligonucleotides of Reference Examples 8 to 22 correspond to a hydroxyl group formed by linking of a hydrogen atom with an oxygen atom.

### (Examples 65 to 136)

Compounds of Examples 65 to 136 were obtained based on the sequences of the oligonucleotides of Reference Example 2, and Reference Examples 8 to 22, by introduction of modified nucleotides as shown in Table 21 to Table 24. These oligonucleotides were synthesized by the phosphoramidite method in the same manner as in Reference Example 1. In the sequences, when an "ec" section was synthesized, N3-Ethyl deoxy Cytidine CED phosphoramidite (ChemGene, catalog number: ANP-3856) was used. When a "T" section was synthesized, Ribo Thymidine CED phosphoramidite (ChemGene, catalog number: ANP-7511) was used.

**[Table 21]**

| Example | Sequece name | Sequence (5'-3') | Molecular weight |
|---|---|---|---|
| 65 | AD1_Factor V.3 | CUGUAUCCUCiCCUGUCCAGGGAU | 7551.0 |
| 66 | AD1_PAH.1.3 | GUGGAAACUCIGAAGGCCAGGCCA | 7762.0 |
| 67 | AD1_ASS1.3 | UUGAUGACCCiGUGGCAUCAGUUG | 7656.0 |
| 68 | AD1_GFAP.3 | AUACUGGUGCiGAUCUCUUUCAGG | 7616.0 |
| 69 | AD1_CBS.3 | GGCGCCGGUCiAAGCCCUUCUCCC | 7564.0 |
| 70 | AD1_GRIA2.3 | GCAUCCUGCCiCAUAAAGGCACCC | 7580.0 |
| 71 | AD1_SLCO2A1.3 | GGGUCAUUACiUUUAAUGAAAUCC | 7608.0 |
| 72 | AD1_PAH.2.29 | C(M)^U(F)^G(M)^U(F)^A(M)^G(F)^C(F)^C(M)^C(M)^C^a^A(M)^G(F)^U(M)^G( F)^A(M)^A(F)^A(M)^A(F)^G(M)^U(F)^U(M)^A(F)^U(M) | 8198.39 |
| 73 | AD1_PAH.2.41 | C(M)^U(F)^G(M)^U(F)^A(M)^G(F)^C(F)^C(M)^C(M)^mc^a^A(M)^G(F)^U(M)^ G(F)^A(M)^A(F)^A(M)^A(F)^G(M)^U(F)^U(M)^A(F)^U(M) | 8202.26 |
| 74 | AD1_PAH.2.43 | C(M)^U(F)^G(M)^U(F)^A(M)^G(F)^C(F)^C(M)^C(M)^mt^a^A(M)^G(F)^U(M)^ G(F)^A(M)^A(F)^A(M)^A(F)^G(M)^U(F)^U(M)^A(F)^U(M) | 8211.25 |
| 75 | AD1_ASS1.39 | U(M)^T(L)^G(M)^A(L)^U(M)^G(F)^A(F)^C(M)^C(M)^c^i^G(M)^U(F)^G(M)^G(F )^C(M)^A(F)^U(M)^C(F)^A(M)^G(L)^U(M)^T(L)^G(M) | 8261.77 |
| 76 | AD1_PANK2.39 | A(M)^A(L)^G(M)^A(L)^A(M)^A(F)^U(F)^U(M)^C(M)^c^a^A(M)^C(F)^A(M)^A( F)^A(M)^U(F)^A(M)^C(F)^C(M)^A(L)^C(M)^C(L)^U(M) | 8171.84 |
| 77 | AD1_NPHS2.39 | A(M)^T(L)^G(M)^T(L)^C(M)^C(F)^A(F)^G(M)^U(M)^c^i^G(M)^A(F)^A(M)^U(F) ^A(M)^U(F)^A(M)^A(F)^U(M)^T(L)^A(M)^C(L)^U(M) | 8226.35 |
| 78 | AD1_GRIA2.39 | G(M)^C(L)^A(M)^T(L)^C(M)^C(F)^U(F)^G(M)^C(M)^c^i^C(M)^A(F)^U(M)^A(F) ^A(M)^A(F)^G(M)^G(F)^C(M)^A(L)^C(M)^C(L)^C(M) | 8204.44 |
| 79 | AD1_ASS1.52 | U(M)^G(L)^A(M)^U(L)^G(F)^A(F)^C(M)^C(M)^c^i^G(M)^U(F)^G(M)^G(F)^C( M)^A(F)^U(M)^C(F)^A(L)^G(M)^U(L)^U(M) | 7552.82 |
| 80 | AD1_GRIA2.39e | G(M)^C(E)^A(M)^T(E)^C(M)^C(F)^U(F)^G(M)^C(M)^c^i^C(M)^A(F)^U(M)^A(F )^A(M)^A(F)^G(M)^G(F)^C(M)^A(E)^C(M)^C(E)^C(M) | 8255.48 |
| 81 | AD1_ASS1.39e | U(M)^T(E)^G(M)^A(E)^U(M)^G(F)^A(F)^C(M)^C(M)^c^i^G(M)^U(F)^G(M)^G( F)^C(M)^A(F)^U(M)^C(F)^A(M)^G(E)^U(M)^T(E)^G(M) | 8318.39 |
| 82 | AD1_PANK2.39e | A(M)^A(E)^G(M)^A(E)^A(M)^A(F)^U(F)^U(M)^C(M)^c^a^A(M)^C(F)^A(M)^A( F)^A(M)^U(F)^A(M)^C(F)^C(M)^A(E)^C(M)^C(E)^U(M) | 8227.84 |
| 83 | AD1_NPHS2.39e | A(M)^T(E)^G(M)^T(E)^C(M)^C(F)^A(F)^G(M)^U(M)^c^i^G(M)^A(F)^A(M)^U(F )^A(M)^U(F)^A(M)^A(F)^U(M)^T(E)^A(M)^C(E)^U(M) | 8283.79 |
| 84 | AD1_GRIA2.52e | C(M)^A(E)^U(M)^C(E)^C(F)^U(F)^G(M)^C(M)^c^i^C(M)^A(F)^U(M)^A(F)^A( M)^A(F)^G(M)^G(F)^C(E)^A(M)^C(E)^C(M) | 7547.10 |
| 85 | AD1_ASS1.52e | U(M)^G(E)^A(M)^T(E)^G(F)^A(F)^C(M)^C(M)^c^i^G(M)^U(F)^G(M)^G(F)^C( M)^A(F)^U(M)^C(F)^A(E)^G(M)^T(E)^U(M) | 7606.87 |
| 86 | AD1_PANK2.52e | A(M)^G(E)^A(M)^A(E)^A(F)^U(F)^U(M)^C(M)^c^a^A(M)^C(F)^A(M)^A(F)^A( M)^U(F)^A(M)^C(F)^C(E)^A(M)^C(E)^C(M) | 7548.20 |
| 87 | AD1_NPHS2.52e | U(M)^G(E)^U(M)^C(E)^C(F)^A(F)^G(M)^U(M)^c^i^G(M)^A(F)^A(M)^U(F)^A( M)^U(F)^A(M)^A(F)^T(E)^U(M)^A(E)^C(M) | 7561.07 |

**[Table 22]**

| Example | Sequece name | Sequence (5'-3) | Molecular weight |
|---|---|---|---|
| 88 | AD1g_RL_A287_Gc | CUUGUAUGACcCAGGAGGCGAUAU | 7676.38 |
| 89 | AD1g_RL_A287_Gg | CUUGUAUGACgCAGGAGGCGAUAU | 7715.88 |
| 90 | AD1g_RL_A287_Gi | CUUGUAUGACiCAGGAGGCGAUAU | 7701.23 |
| 91 | AD1g_RL_A287_Gt | CUUGUAUGACtCAGGAGGCGAUAU | 7690.51 |
| 92 | AD1g_RL_A287_Gd | CUUGUAUGACdCAGGAGGCGAUAU | 7566.57 |
| 93 | AD1g_RL_A287_u | CUUGUAUGACuCAGGAGGCGAUAU | 7674.20 |
| 94 | AD1g_RL_A287_T | CUUGUAUGACTCAGGAGGCGAUAU | 7708.24 |
| 95 | AD1 g_RL_A287 _5mc | CUUGUAUGAC5cCAGGAGGCGAUAU | 7688.22 |
| 96 | AD1g_RL_A287_a | CUUGUAUGACaCAGGAGGCGAUAU | 7700.80 |
| 97 | AD1g_RL_A287_Gi.39 | C(M)^T(L)^U(M)^G(L)^U(M)^A(F)^U(F)^G(M)^A(M)^c^i^C(M)^A(F)^G(M)^G(F )^A(M)^G(F)^G(M)^C(F)^G(M)^A(L)^U(M)^A(L)^U(M) | 8299.67 |
| 98 | AD1g_RL_A287_Gu.39 | C(M)^T(L)^U(M)^G(L)^U(M)^A(F)^U(F)^G(M)^A(M)^c^u^C(M)^A(F)^G(M)^G( F)^A(M)^G(F)^G(M)^C(F)^G(M)^A(L)^U(M)^A(L)^U(M) | 8274.00 |
| 99 | AD1g_RL_A287_Gt.39 | C(M)^T(L)^U(M)^G(L)^U(M)^A(F)^U(F)^G(M)^A(M)^c^t^C(M)^A(F)^G(M)^G(F )^A(M)^G(F)^G(M)^C(F)^G(M)^A(L)^U(M)^A(L)^U(M) | 8288.39 |

**[Table23]**

| Example | Sequece name | Sequence (5'-3) | Molecular weight |
|---|---|---|---|
| 100 | hGAPDH_AD1_42 | U(M)^C(F)^A(M)^A(F)^G(M)^G(F)^G(F)^U(M)^C(M)^ec^a^C(M)^A(F)^U(M)^ G(F)^G(M)^C(F)^A(M)^A(F)^C(M)^U(F)^G(M)^U(F)^G(M) | 8242.45 |
| 101 | hGAPDH_AD1_45 | U(M)^C(L)^A(M)^A(L)^G(M)^G(F)^G(F)^U(M)^C(M)^ec^a^C(M)^A(F)^U(M)^G (F)^G(M)^C(F)^A(M)^A(F)^C(M)^T(L)^G(M)^T(L)^G(M) | 8325.88 |
| 102 | hGAPDH_AD1_52 | C(M)^A(L)^A(M)^G(L)^G(F)^G(F)^U(M)^C(M)^c^a^C(M)^A(F)^U(M)^G(F)^G( M)^C(F)^A(M)^A(F)^C(L)^U(M)^G(L)^U(M) | 7558.92 |
| 103 | hGAPDH_AD1_53 | A(L)^A(M)^G(L)^G(F)^G(F)^U(M)^C(M)^c^a^C(M)^A(F)^U(M)^G(F)^G(M)^C( F)^A(M)^A(F)^C(L)^U(M)^G(L) | 6887.29 |
| 104 | hGAPDH_AD1_54 | T(L)^C(L)^A(L)^A(L)^G(L)^G(F)^G(F)^U(M)^C(M)^c^a^C(M)^A(F)^U(M)^G(F)^ G(M)^C(F)^A(M)^A(F)^C(L)^T(L)^G(L)^T(L)^G(L) | 8315.67 |
| 105 | hGAPDH_AD1_55 | C(L)^A(L)^A(L)^G(L)^G(F)^G(F)^U(M)^C(M)^c^a^C(M)^A(F)^U(M)^G(F)^G(M )^C(F)^A(M)^A(F)^C(L)^T(L)^G(L)^T(L) | 7593.58 |
| 106 | hGAPDH_AD1_56 | A(L)^A(L)^G(L)^G(F)^G(F)^U(M)^C(M)^c^a^C(M)^A(F)^U(M)^G(F)^G(M)^C(F )^A(M)^A(F)^C(L)^T(L)^G(L) | 6897.00 |
| 107 | hGAPDH_AD1_57 | C(L)^A(L)^A(L)^G(L)^G(F)^G(F)^U(M)^C(M)^c^a^C(M)^A(F)^U(M)^G(F)^G(M )^C(F)^A(M)^A(F)^C(L)^T(L)^G(L) | 7242.41 |
| 108 | hGAPDH_AD1_58 | C(L)^A(L)^A(L)^G(L)^G(F)^G(F)^U(M)^C(M)^c^a^C(M)^A(F)^U(M)^G(F)^G(M )^C(F)^A(M)^A(F)^C(L)^T(L) | 6870.50 |
| 109 | hGAPDH_AD1_59 | C(L)^A(L)^A(L)^G(L)^G(F)^G(F)^U(M)^C(M)^c^a^C(M)^A(F)^U(M)^G(F)^G(M )^C(F)^A(M)^A(F)^C(L) | 6522.69 |
| 110 | hGAPDH_AD1_60 | A(L)^A(L)^G(L)^G(F)^G(F)^U(M)^C(M)^c^a^C(M)^A(F)^U(M)^G(F)^G(M)^C(F )^A(M)^A(F)^C(L)^T(L)^G(L)^T(L) | 7244.05 |
| 111 | hGAPDH_AD1_61 | A(L)^G(L)^G(F)^G(F)^U(M)^C(M)^c^a^C(M)^A(F)^U(M)^G(F)^G(M)^C(F)^A( M)^A(F)^C(L)^T(L)^G(L)^T(L) | 6889.36 |
| 112 | hGAPDH_AD1_62 | U(M)^C(L)^A(M)^A(L)^G(M)^G(F)^G(F)^U(M)^C(F)^c^a^C(M)^A(F)^U(M)^G( F)^G(M)^C(F)^A(M)^A(F)^C(M)^T(L)^G(M)^T(L)^G(M) | 8284.98 |
| 113 | hGAPDH_AD1_63 | U(M)^C(L)^A(M)^A(L)^G(M)^G(F)^G(F)^U(M)^c^c^a^C(M)^A(F)^U(M)^G(F)^ G(M)^C(F)^A(M)^A(F)^C(M)^T(L)^G(M)^T(L)^G(M) | 8268.23 |
| 114 | hGAPDH_AD1_64 | U(M)^C(L)^A(M)^A(L)^G(M)^G(F)^G(F)^U(M)^C(L)^c^a^C(M)^A(F)^U(M)^G(F )^G(M)^C(F)^A(M)^A(F)^C(M)^T(L)^G(M)^T(L)^G(M) | 8309.97 |
| 115 | hGAPDH_AD1_65 | U(M)^C(L)^A(M)^A(L)^G(M)^G(F)^G(F)^U(M)^C^c^a^C(M)^A(F)^U(M)^G(F)^ G(M)^C(F)^A(M)^A(F)^C(M)^T(L)^G(M)^T(L)^G(M) | 8285.46 |
| 116 | hGAPDH_AD1_66 | U(M)^C(L)^A(M)^A(L)^G(M)^G(F)^G(F)^U(M)^C(M)^C^a^C(M)^A(F)^U(M)^G( F)^G(M)^C(F)^A(M)^A(F)^C(M)^T(L)^G(M)^T(L)^G(M) | 8315.67 |
| 117 | hGAPDH_AD1_67 | U(M)^C(L)^A(M)^A(L)^G(M)^G(F)^G(F)^U(M)^C(F)^C^a^C(M)^A(F)^U(M)^G( F)^G(M)^C(F)^A(M)^A(F)^C(M)^T(L)^G(M)^T(L)^G(M) | 8302.40 |

**[table 24]**

| Example | Sequece name | Sequence (5'-3') | Molecular weight |
|---|---|---|---|
| 118 | hGAPDH_AD1_68 | J(M)^C(L)^A(M)^A(L)^G(M)^G(F)^G(F)^U(M)^c^C^a^C(M)^A(F)^U(M)^G(F)^ G(M)^C(F)^A(M)^A(F)^C(M)^T(L)^G(M)^T(L)^G(M) | 8287.43 |
| 119 | hGAPDH_AD1_69 | J(M)^C(L)^A(M)^A(L)^G(M)^G(F)^G(F)^U(M)^C(L)^C^a^C(M)^A(F)^U(M)^G( F)^G(M)^C(F)^A(M)^A(F)^C(M)^T(L)^G(M)^T(L)^G(M) | 8327.86 |
| 120 | hGAPDH_AD1_70 | J(M)^C(L)^A(M)^A(L)^G(M)^G(F)^G(F)^U(M)^C^C^a^C(M)^A(F)^U(M)^G(F)^ G(M)^C(F)^A(M)^A(F)^C(M)^T(L)^G(M)^T(L)^G(M) | 8301.99 |
| 121 | hGAPDH_AD1_71 | J(M)^C(L)^A(M)^A(L)^G(M)^G(F)^G(F)^U(M)^C(M)^C(F)^a^C(M)^A(F)^U(M)^ G(F)^G(M)^C(F)^A(M)^A(F)^C(M)^T(L)^G(M)^T(L)^G(M) | 8314.44 |
| 122 | hGAPDH_AD1_72 | U(M)^C(L)^A(M)^A(L)^G(M)^G(F)^G(F)^U(M)^C(F)^C(F)^a^C(M)^A(F)^U(M)^ G(F)^G(M)^C(F)^A(M)^A(F)^C(M)^T(L)^G(M)^T(L)^G(M) | 8302.34 |
| 123 | hGAPDH_AD1_73 | U(M)^C(L)^A(M)^A(L)^G(M)^G(F)^G(F)^U(M)^c^C(F)^a^C(M)^A(F)^U(M)^G( F)^G(M)^C(F)^A(M)^A(F)^C(M)^T(L)^G(M)^T(L)^G(M) | 8284.99 |
| 124 | hGAPDH_AD1_74 | U(M)^C(L)^A(M)^A(L)^G(M)^G(F)^G(F)^U(M)^C(L)^C(F)^a^C(M)^A(F)^U(M)^ G(F)^G(M)^C(F)^A(M)^A(F)^C(M)^T(L)^G(M)^T(L)^G(M) | 8325.69 |
| 125 | hGAPDH_AD1_75 | J(M)^C(L)^A(M)^A(L)^G(M)^G(F)^G(F)^U(M)^C^C(F)^a^C(M)^A(F)^U(M)^G( F)^G(M)^C(F)^A(M)^A(F)^C(M)^T(L)^G(M)^T(L)^G(M) | 8302.12 |
| 126 | hGAPDH_AD1_76 | J(M)^C(L)^A(M)^A(L)^G(M)^G(F)^G(F)^U(M)^C(M)^C(L)^a^C(M)^A(F)^U(M)^ G(F)^G(M)^C(F)^A(M)^A(F)^C(M)^T(L)^G(M)^T(L)^G(M) | 8338.32 |
| 127 | hGAPDH_AD1_77 | U(M)^C(L)^A(M)^A(L)^G(M)^G(F)^G(F)^U(M)^C(F)^C(L)^a^C(M)^A(F)^U(M)^ G(F)^G(M)^C(F)^A(M)^A(F)^C(M)^T(L)^G(M)^T(L)^G(M) | 8326.27 |
| 128 | hGAPDH_AD1_78 | J(M)^C(L)^A(M)^A(L)^G(M)^G(F)^G(F)^U(M)^c^C(L)^a^C(M)^A(F)^U(M)^G(F ^G(M)^C(F)^A(M)^A(F)^C(M)^T(L)^G(M)^T(L)^G(M) | 8308.04 |
| 129 | hGAPDH_AD1_79 | U(M)^C(L)^A(M)^A(L)^G(M)^G(F)^G(F)^U(M)^C(L)^C(L)^a^C(M)^A(F)^U(M)^ G(F)^G(M)^C(F)^A(M)^A(F)^C(M)^T(L)^G(M)^T(L)^G(M) | 8351.75 |
| 130 | hGAPDH_AD1_80 | U(M)^C(L)^A(M)^A(L)^G(M)^G(F)^G(F)^U(M)^C^C(L)^a^C(M)^A(F)^U(M)^G( F)^G(M)^C(F)^A(M)^A(F)^C(M)^T(L)^G(M)^T(L)^G(M) | 8325.16 |
| 131 | hGAPDH_AD1_81 | U(M)^C(L)^A(M)^A(L)^G(M)^G(F)^G(F)^U(M)^C(M)^C(M)^a^C(M)^A(F)^U(M) ^G(F)^G(M)^C(F)^A(M)^A(F)^C(M)^T(L)^G(M)^T(L)^G(M) | 8328.78 |
| 132 | hGAPDH_AD1_82 | U(M)^C(L)^A(M)^A(L)^G(M)^G(F)^G(F)^U(M)^C(F)^C(M)^a^C(M)^A(F)^U(M)^ G(F)^G(M)^C(F)^A(M)^A(F)^C(M)^T(L)^G(M)^T(L)^G(M) | 8318.58 |
| 133 | hGAPDH_AD1_83 | U(M)^C(L)^A(M)^A(L)^G(M)^G(F)^G(F)^U(M)^c^C(M)^a^C(M)^A(F)^U(M)^G( F)^G(M)^C(F)^A(M)^A(F)^C(M)^T(L)^G(M)^T(L)^G(M) | 8298.95 |
| 134 | hGAPDH_AD1_84 | U(M)^C(L)^A(M)^A(L)^G(M)^G(F)^G(F)^U(M)^C(L)^C(M)^a^C(M)^A(F)^U(M)^ G(F)^G(M)^C(F)^A(M)^A(F)^C(M)^T(L)^G(M)^T(L)^G(M) | 8342.90 |
| 135 | hGAPDH_AD1_85 | U(M)^C(L)^A(M)^A(L)^G(M)^G(F)^G(F)^U(M)^C^C(M)^a^C(M)^A(F)^U(M)^G( F)^G(M)^C(F)^A(M)^A(F)^C(M)^T(L)^G(M)^T(L)^G(M) | 8315.71 |
| 136 | AD1_A1AT.39 | G(M)^T(L)^C(M)^C(L)^C(M)^U(F)^U(F)^C(M)^U(M)^c^i^U(M)^C(F)^G(M)^A(F) ^U(M)^G(F)^G(M)^U(F)^C(M)^A(L)^G(M)^C(L)^A(M) | 8171.07 |

The "Molecular weight" in the Tables represents a measured value by negative-ion ESI mass spectrometry. In the "Sequence" in the Tables, "T" represents ribothymidine, and "ec" represents N3-ethyl-2'-deoxycytidine. Other abbreviations are as described above. The 5'-end and the 3'-end of each oligonucleotide correspond to a hydroxyl group formed by linking of a hydrogen atom with an oxygen atom.

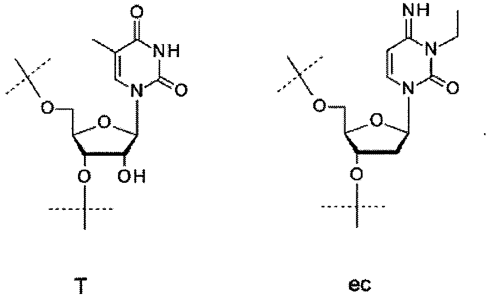

### (Test Example 15)

### Evaluation (3) of editing inducibility of GAPDH gene sequence, in cultured cells, with gRNA as compound of Example

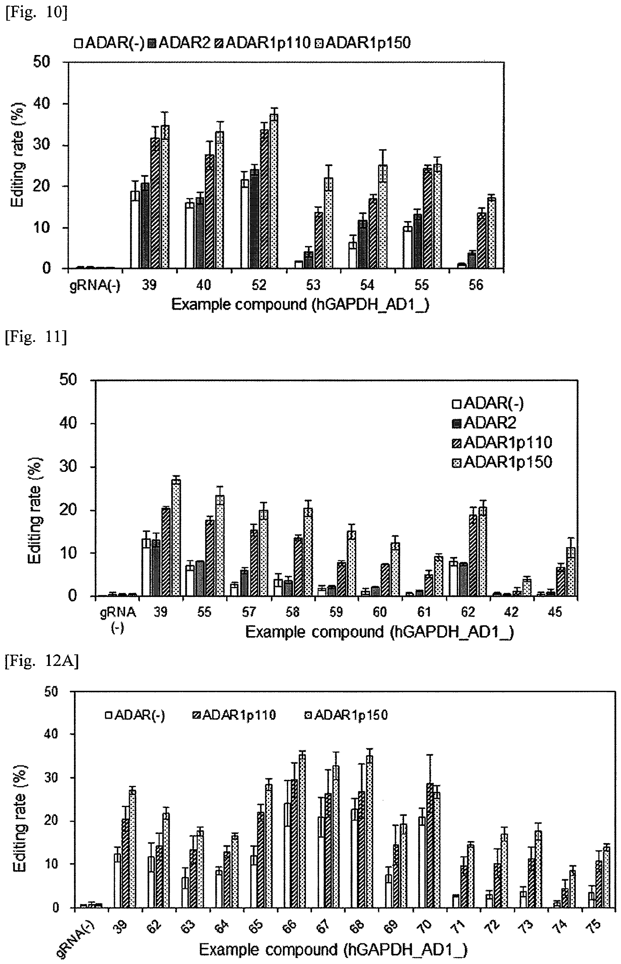

The method of evaluation of editing inducibility of each of the compounds of Examples was the same method in Test Example 3. The editing rates (%) in the case of use of each of the compounds of Examples 39, 40, and 102 to 106 (hGAPDH_AD1_39, hGAPDH_AD1_40, hGAPDH_AD1_52, to hGAPDH_AD1_56) are shown in Fig. 10.

In the case where the plasmid expressing ADAR1p110 or ADAR1p150 was transfected, a high editing rate was exhibited as compared with the case where no gRNA was used (in the drawing, gRNA(-)). In particular, the compounds of Examples 39, 40, and 102 (hGAPDH_AD1_39, hGAPDH_AD1_40, hGAPDH_ADl_52) where LNA was introduced to the linking site with the target RNA exhibited the highest editing rate. Furthermore, the editing induction in cells, in the case of transfection of only gRNA as the compound of Example at a concentration of 50 nM, was evaluated, and then a high editing rate was exhibited as compared with gRNA(-).

The editing rates (%) in the case of use of each of the compounds of Examples 39, 100, 101, 105, and 107 to 112 (hGAPDH_AD1_39, hGAPDH_AD1_42, hGAPDH_AD1_45, hGAPDH_AD1_55, hGAPDH_AD1_57 to hGAPDH_AD1_62) are shown in Fig. 11.

In the case where the plasmid expressing ADAR1p110 or ADAR1p150 was transfected, a high editing rate was exhibited as compared with the case where no gRNA was used (in the drawing, gRNA(-)). In particular, the compounds of Examples 39, 105, and 112 (hGAPDH_AD1_39, hGAPDH_AD1_55, hGAPDH_AD1_62) where LNA was introduced to the linking site with the target RNA exhibited the highest editing rate. Furthermore, the editing induction in cells, in the case of transfection of only gRNA as the compound of Example at a concentration of 50 nM, was evaluated, and then a high editing rate was exhibited as compared with gRNA(-).

The editing rates (%) in the case of use of each of the compounds of Examples (hGAPDH_AD1_39, hGAPDH_AD1_62 to hGAPDH_AD1_75) are shown in Fig. 12A.

In the case where the plasmid expressing ADAR1p110 or ADAR1p150 was transfected, a high editing rate was exhibited as compared with the case where no gRNA was used (in the drawing, gRNA(-)). In particular, the compounds of Examples 39, 112, 115 to 118, and 120 (hGAPDH_AD1_39, hGAPDH_AD1_62, hGAPDH_AD1_65 to hGAPDH_AD1_68, hGAPDH_AD1_70), in which a deoxynucleotide residue or a ribonucleotide residue was introduced as the target-corresponding nucleotide residue, each exhibited a particularly high editing rate. Furthermore, the editing induction in cells, in the case of transfection of only gRNA as the compound of Example at a concentration of 50 nM, was evaluated, and then a high editing rate was exhibited as compared with gRNA(-).

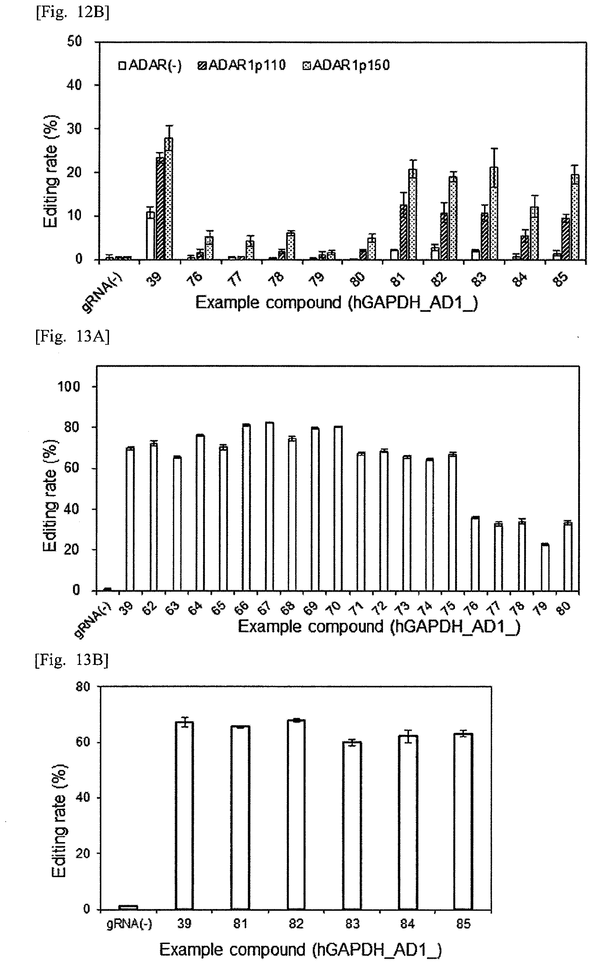

The editing rates (%) in the case of use of each of the compounds of Examples (hGAPDH_AD1_39, hGAPDH_AD1_76 to hGAPDH_AD1_85) are shown in Fig. 12B.

In the case where the plasmid expressing ADAR1p110 or ADAR1p150 was transfected, a high editing rate was exhibited as compared with the case where no gRNA was used (in the drawing, gRNA(-)). In particular, the compounds of Examples 131 to 133, and 135 (hGAPDH_AD1_81 to hGAPDH_AD1_83, hGAPDH_AD1_85), in which a 2'-O-Me- ribonucleotide residue was introduced as the target-corresponding nucleotide residue, each exhibited a particularly high editing rate.

### (Test Example 16)

### Evaluation of editing inducibility of RNA having hGAPDH gene sequence

### (A) Synthesis of target RNA

The same operation as in the method described in (A) Synthesis of AGXT_RNA in Test Example 5 was performed. Here, the following GAPDH_FW (SEQ ID NO: 64) and GAPDH_RV (SEQ ID NO: 65) were used as oligo DNAs.

**[Table 25]**

| | Sequence | SEQ ID NO: |
|---|---|---|
| GAPDH_FW | AATTCTCTCCCCTCCTCACAGTTGCCATGTAG ACCCCTTGAAGAGGGGAGGGGCCTA | 64 |
| GAPDH_RV | AGCTTAGGCCCCTCCCCTCTTCAAGGGGTCT ACATGGCAACTGTGAGGAGGGGAGAG | 65 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by ADAR1p110 are shown in Fig. 13A and Fig. 13B. The editing efficiency of RNA with ADAR1p110 was exhibited as a high editing rate with the compounds of Examples 39 and 112 to 135 (hGAPDH_AD1_39, hGAPDH_AD1_62 to 85), as compared with the case of no addition of gRNA. In particular, it was indicated that one in which a deoxyribonucleotide residue, a ribonucleotide residue, a 2'-F- ribonucleotide residue, or a 2'-O-Me- ribonucleotide residue was used as the target-corresponding nucleotide residue exhibited a high editing rate.

### (Test Example 17) Evaluation of editing inducibility of RNA having Factor V gene sequence

### (A) Synthesis of target RNA

The same operation as in the method described in (A) Synthesis of AGXT_RNA in Test Example 5 was performed. Here, one having a Factor V gene sequence, described below, was used as oligo DNA. The Factor V gene sequence here used contains the 1682-th to 1706-th base sequences of GenBank accession no. NM_000130.45, and furthermore contains the mutation (rs6025) of nucleoside, called c.1601G>A.

**[Table 26]**

| | Sequence | SEQ ID NO: |
|---|---|---|
| Factor V_FW | AATTCTGTAAGAGCAGATCCCTGGACAGGCA AGGAATACAGGTATTTTGTCCTTGAA | 95 |
| Factor _RV | AGCTTTCAAGGACAAAATACCTGTATTCCTTG CCTGTCCAGGGATCTGCTCTTACAG | 96 |

### (B) Evaluation of editing inducibility and results thereof

The method of evaluation of editing inducibility of each of the compounds of Examples was the same method as in (B) and (D) of Test Example 5.

### (C) Results of editing analysis

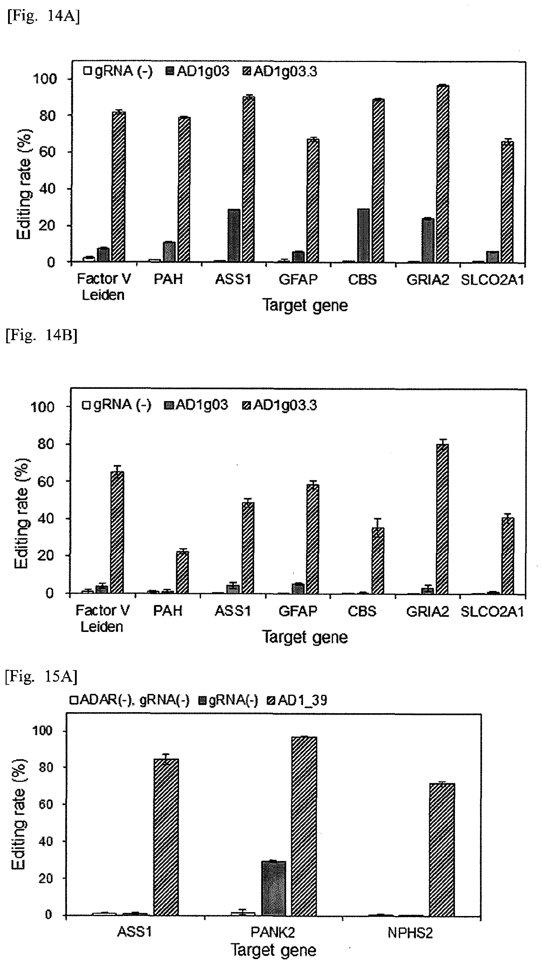

The results of the editing rate by ADAR1p110 are shown in Fig. 14A. The editing efficiency of RNA with ADAR1p110 was exhibited as a high editing rate with the compound of Example 65 (AD1_Factor V.3, in the drawing, AD1g03.3 of Factor V) in which guanosine at the 3'-side of the target-corresponding nucleotide residue was 2'-deoxyinosine in gRNA, as compared with the compound of Reference Example 8 (AD1_Factor V, in the drawing, AD1g03 of Factor V), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

The results of the editing rate by ADAR2 are shown in Fig. 14B. The editing efficiency of RNA with ADAR2 was exhibited as a high editing rate in Example 65 (AD1_Factor V.3, in the drawing, AD1g03.3 of Factor V) in which guanosine at the 3'-side of the target-corresponding nucleotide residue was 2'-deoxyinosine in gRNA, as compared with the compound of Reference Example 8 (AD1_Factor V, in the drawing, AD1g03 of Factor V), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

### (Test Example 18) Evaluation of editing inducibility of RNA having CBS gene sequence

### (A) Synthesis of target RNA

The same operation as in the method described in (A) Synthesis of AGXT_RNA in Test Example 5 was performed. Here, one having a CBS gene sequence, described below, was used as oligo DNA. The CBS gene sequence here used contains the 1561-th to 1585-th base sequences of GenBank accession no. NM_000071.2, and furthermore contains the mutation (rs28934891) of nucleoside, called c.1330G>A.

**[Table 27]**

| | Sequence | SEQ ID NO: |
|---|---|---|
| CBS_FW | AATTCCGAGATCCTCCGGGAGAAGGGCTTCA ACCAGGCGCCCGTGGTGGATGAGGCA | 97 |
| CBS_RV | AGCTTGCCTCATCCACCACGGGCGCCTGGTT GAAGCCCTTCTCCCGGAGGATCTCGG | 98 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by ADAR1p110 are shown in Fig. 14A.
The editing efficiency of RNA with ADAR1p110 was exhibited as a high editing rate with the compound of Example 69 (AD1_CBS.3, in the drawing, AD1g03.3 of CBS) in which guanosine at the 3'-side of the target-corresponding nucleotide residue was 2'-deoxyinosine in gRNA, as compared with the compound of Reference Example 12 (AD1_CBS, in the drawing, AD1g03 of CBS), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

The results of the editing rate by ADAR2 are shown in Fig. 14B. The editing efficiency of RNA with ADAR2 was exhibited as a high editing rate with the compound of Example 69 (AD1_CBS.3, in the drawing, AD1g03.3 of CBS) in which guanosine at the 3'-side of the target-corresponding nucleotide residue was 2'-deoxyinosine in gRNA, as compared with the compound of Reference Example 12 (AD1_CBS, in the drawing, AD1g03 of CBS), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

### (Test Example 19) Evaluation of editing inducibility of RNA having PAH gene sequence

### (A) Synthesis of target RNA

The same operation as in the method described in (A) Synthesis of AGXT_RNA in Test Example 5 was performed. Here, one having a PAH gene sequence was used as oligo DNA. The PAH gene sequence here used contains the 882-th to 906-th base sequences of GenBank accession no. NM_000277.3, and furthermore contains the mutation (rs5030849) of nucleoside, called c.782G>A.

**[Table 28]**

| | Sequence | SEQ ID NO: |
|---|---|---|
| PAH.1_FW | AATTCTAACAGTGATAATAACTTTTCACTTAG GGCCTACAGTACTGCTTATCAGAGA | 99 |
| PAH.1_RV | AGCTTCTCTGATAAGCAGTACTGTAGGCCCT AAGTGAAAAGTTATTATCACTGTTAG | 100 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by ADAR1p110 are shown in Fig. 14A. The editing efficiency of RNA with ADAR1p110 was exhibited as a high editing rate with the compound of Example 66 (AD1_PAH.1.3, in the drawing, AD1g03.3 of PAH) in which guanosine at the 3'-side of the target-corresponding nucleotide residue was 2'-deoxyinosine in gRNA, as compared with the compound of Reference Example 9 (AD1_PAH.1, in the drawing, AD1g03 of PAH), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

The results of the editing rate by ADAR2 are shown in Fig. 14B. The editing efficiency of RNA with ADAR2 was exhibited as a high editing rate with the compound of Example 66 (AD1_PAH.1.3, in the drawing, AD1g03.3 of PAH) in which guanosine at the 3'-side of the target-corresponding nucleotide residue was 2'-deoxyinosine in gRNA, as compared with the compound of Reference Example 9 (AD1_PAH.1, in the drawing, AD1g03 of PAH), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

### (Test Example 20) Evaluation of editing inducibility of RNA having ASS1 gene sequence

### (A) Synthesis of target RNA

The same operation as in the method described in (A) Synthesis of AGXT_RNA in Test Example 5 was performed. Here, one having an ASS1 gene sequence, described below, was used as oligo DNA. The ASS1 gene sequence here used contains the 1510-th to 1534-th base sequences of GenBank accession no. NM_000050.4, and furthermore contains the mutation (rs121908641) of nucleoside, called c.1168G>A.

**[Table 29]**

| | Sequence | SEQ ID NO: |
|---|---|---|
| ASS1_FW | AATTCTGATTATGAGCCAACTGATGCCACCA GGTTCATCAACATCAATTCCCTCAGA | 101 |
| ASS1_RV | AGCTTCTGAGGGAATTGATGTTGATGAACCT GGTGGCATCAGTTGGCTCATAATCAG | 102 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by ADAR1p110 are shown in Fig. 14A. The editing efficiency of RNA with ADAR1p110 was exhibited as a high editing rate with the compound of Example 67 (AD1_ASS1.3, in the drawing, AD1g03.3 of ASS1) in which guanosine at the 3'-side of the target-corresponding nucleotide residue was 2'-deoxyinosine in gRNA, as compared with the compound of Reference Example 10 (AD1_ASS1, in the drawing, AD1g03 of ASS1), entirely composed of natural ribonucleotide residues. This indicates that, in the case where the 5'-side of the editing site corresponds to cytidine, a high editing rate is exhibited by use of 2'-deoxyinosine as the nucleotide at the 3'-side of the target-corresponding nucleotide residue in gRNA.

The results of the editing rate by ADAR2 are shown in Fig. 14B. The editing efficiency of RNA with ADAR2 was exhibited as a high editing rate with the compound of Example 67 (AD1_ASS1.3, in the drawing, AD1g03.3 of ASS1) in which guanosine at the 3'-side of the target-corresponding nucleotide residue was 2'-deoxyinosine in gRNA, as compared with the compound of Reference Example 10 (AD1_ASS1, in the drawing, AD1g03 of ASS1), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

### (Test Example 21) Evaluation of editing inducibility of RNA having GRIA2 gene sequence

### (A) Synthesis of target RNA

The same operation as in the method described in (A) Synthesis of AGXT_RNA in Test Example 5 was performed. Here, one having a GRIA2 gene sequence, described below, was used as oligo DNA. The GRIA2 gene sequence here used contains the 2121-th to 2145-th base sequences of GenBank accession no. NM_000826.4, and furthermore the 2135-th adenosine corresponds to a site at which the editing efficiency to inosine is to be reduced in the case of a reduction in expression of ADAR2 in ALS disease.

**[Table 30]**

| | Sequence | SEQ ID NO: |
|---|---|---|
| GRIA2_FW | AATTCTGGTTTTCCTTGGGTGCCTTTATGCAG CAAGGATGCGATATTTCGCCAAGAA | 103 |
| GRIA2_RV | AGCTTTCTTGGCGAAATATCGCATCCTTGCTG CATAAAGGCACCCAAGGAAAACCAG | 104 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by ADAR1p110 are shown in Fig. 14A. The editing efficiency of RNA with ADAR1p110 was exhibited as a high editing rate with the compound of Example 70 (AD1_GRIA2.3, in the drawing, AD1g03.3 of GRIA2) in which guanosine at the 3'-side of the target-corresponding nucleotide residue was 2'-deoxyinosine in gRNA, as compared with the compound of Reference Example 13 (AD1_GRIA2, in the drawing, AD1g03 of GRIA2), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

The results of the editing rate by ADAR2 are shown in Fig. 14B. The editing efficiency of RNA with ADAR2 was exhibited as a high editing rate with the compound of Example 70 (AD1_GRIA2.3, in the drawing, AD1g03.3 of GRIA2) in which guanosine at the 3'-side of the target-corresponding nucleotide residue was 2'-deoxyinosine in gRNA, as compared with the compound of Reference Example 13 (AD1_GRIA2, in the drawing, AD1g03 of GRIA2), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

### (Test Example 22) Evaluation of editing inducibility of RNA having SLCO2A1 gene sequence

### (A) Synthesis of target RNA

The same operation as in the method described in (A) Synthesis of AGXT_RNA in Test Example 5 was performed. Here, one having an SLCO2A1 gene sequence, described below, was used as oligo DNA. The SLCO2A1 gene sequence here used contains the 81020-th to 81044-th base sequences of GenBank accession no. NM_005630.3, and furthermore contains the mutation (rs765249238) of nucleoside, called c.940+1G>A.

**[Table 31]**

| | Sequence | SEQ ID NO: |
|---|---|---|
| SLCO2A1_FW | AATTCGGCTCCCTGGTGGATTTCATTAAACAT AACTGACCCTACAGCCCTGGGTGGA | 105 |
| SLCO2A1_RV | AGCTTCCACCCAGGGCTGTAGGGTCAGTTAT GTTTAATGAAATCCACCAGGGAGCCG | 106 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by ADAR1p110 are shown in Fig. 14A. The editing efficiency of RNA with ADAR1p110 was exhibited as a high editing rate with the compound of Example 71 (AD1_ SLCO2A1.3, in the drawing, AD1g03.3 of SLCO2A1) in which guanosine at the 3'-side of the target-corresponding nucleotide residue was 2'-deoxyinosine in gRNA, as compared with the compound of Reference Example 14 (AD1_SLCO2A1, in the drawing, AD1g03 of SLCO2A1), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

The results of the editing rate by ADAR2 are shown in Fig. 14B. The editing efficiency of RNA with ADAR2 was exhibited as a high editing rate with the compound of Example 71 (AD1_SLCO2A1.3, in the drawing, AD1g03.3 of SLCO2A1) in which guanosine at the 3'-side of the target-corresponding nucleotide residue was 2'-deoxyinosine in gRNA, as compared with the compound of Reference Example 14 (AD1_SLCO2A1, in the drawing, AD1g03 of SLCO2A1), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

### (Test Example 23) Evaluation of editing inducibility of RNA having GFAP gene sequence

### (A) Synthesis of target RNA

The same operation as in the method described in (A) Synthesis of AGXT_RNA in Test Example 5 was performed. Here, one having a GFAP gene sequence, described below, was used as oligo DNA. The GFAP gene sequence here used contains the 2154-th to 2178-th base sequences of GenBank accession no. NM_002055.5, and furthermore contains the mutation (rs59565950) of nucleoside, called c.716G>A.

**[Table 32]**

| | Sequence | SEQ ID NO: |
|---|---|---|
| GFAP_FW | AATTCCTCACCGCAGCCCTGAAAGAGATCCA CACGCAGTATGAGGCAATGGCGTCCA | 107 |
| GFAP_RV | AGCTTGGACGCCATTGCCTCATACTGCGTGT GGATCTCTTTCAGGGCTGCGGTGAGG | 108 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by ADAR1p110 are shown in Fig. 14A.
The editing efficiency of RNA with ADAR1p110 was exhibited as a high editing rate with the compound of Example 68 (AD1_GFAP.3, in the drawing, AD1g03.3 of GFAP) in which guanosine at the 3'-side of the target-corresponding nucleotide residue was 2'-deoxyinosine in gRNA, as compared with the compound of Reference Example 11 (AD1_GFAP, in the drawing, AD1g03 of GFAP), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

The results of the editing rate by ADAR2 are shown in Fig. 14B. The editing efficiency of RNA with ADAR2 was exhibited as a high editing rate with the compound of Example 68 (AD1_GFAP, in the drawing, AD1g03.3 of GFAP) in which guanosine at the 3'-side of the target-corresponding nucleotide residue was 2'-deoxyinosine in gRNA, as compared with the compound of Reference Example 11 (AD1_GFAP, in the drawing, AD1g03 of GFAP), entirely composed of natural ribonucleotide residues. This indicates that, in the case where the nucleoside at the 5'-side of the editing site is cytidine, a high editing rate is exhibited by use of 2'-deoxyinosine as the nucleotide at the 3'-side of the target-corresponding nucleotide residue in gRNA.

### (Test Example 24) Evaluation of editing inducibility of RNA having ASS1 gene sequence

### (A) Synthesis of target RNA

The same operation as in the method described in (A) Synthesis of AGXT_RNA in Test Example 5 was performed. Here, one having an ASS 1 gene sequence, described in Test Example 20, was used as oligo DNA. The ASS1 gene sequence here used contains the 1510-th to 1534-th base sequences of GenBank accession no. NM_000050.4, and furthermore contains the mutation (rs121908641) of nucleoside, called c.1168G>A.

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by use of Example 76 (AD1 _ASS1.39) are shown in Fig. 15A. The RNA editing efficiency of the chemically modified compound of Example 76 (AD1_ASS1.39), with ADAR1p110, was exhibited as a high editing rate as compared with one where no gRNA was added. The compound of the present Example is indicated to be able to repair the ASS1 gene sequence mutated.

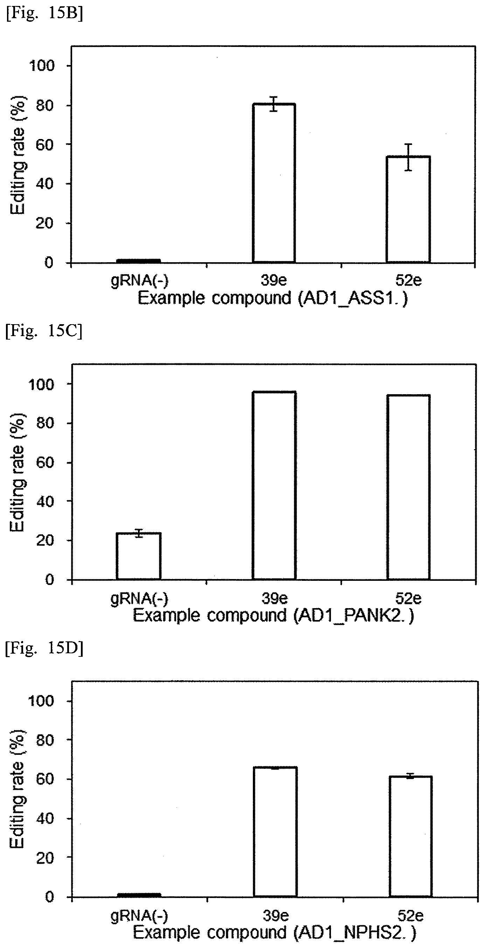

The results of the editing rates by use of the compound of Example 81 (AD1_ASS1.39e) and the compound of Example 85 (AD1_ASS1.52e) are shown in Fig. 15B. The RNA editing efficiency of each of the chemically modified compound of Example 81 (AD1_ASS1.39e) and compound of Example 85 (AD1_ASS1.52e), with ADAR1p110, was exhibited as a high editing rate as compared with one where no gRNA was added. The compounds of the present Examples are indicated to be able to repair the ASS1 gene sequence mutated.

### (Test Example 25) Evaluation of editing inducibility of RNA having PANK2 gene sequence

### (A) Synthesis of target RNA

The same operation as in the method described in (A) Synthesis of AGXT_RNA in Test Example 5 was performed. Here, one having a PANK2 gene sequence, described below, was used as oligo DNA. The PANK2 gene sequence here used contains 1714-th to 1738-th base sequences of GenBank accession no. NM_153638.3, and furthermore contains the mutation (rs137852959) of nucleoside, called c.1561G>A.

**[Table 33]**

| | Sequence | SEQ ID NO: |
|---|---|---|
| PANK2_FW | AATTCAAACATTAACCAGGTGGTATTTGTTAG AAATTTCTTGAGAATTAATACGATA | 109 |
| PANK2_RV | AGCTTATCGTATTAATTCTCAAGAAATTTCTAA CAAATACCACCTGGTTAATGTTTG | 110 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by use of the compound of Example 76 (AD1_PANK2.39) are shown in Fig. 15A. The RNA editing efficiency of the chemically modified compound of Example 76 (AD1_PANK2.39), with ADAR1p110, was exhibited as a high editing rate as compared with one where no gRNA was added. The compound of the present Example is indicated to be able to repair the PANK2 gene sequence mutated.

The results of the editing rates by use of the compound of Example 82 (AD1_PANK2.39e) and the compound of Example 86 (AD1_PANK2.52e) are shown in Fig. 15C. The RNA editing efficiency of the chemically modified compound of Example 76 (AD1_PANK2.39), with ADAR1p110, was exhibited as a high editing rate as compared with one where no gRNA was added. The compounds of the present Examples are indicated to be able to repair the PANK2 gene sequence mutated.

### (Test Example 26) Evaluation of editing inducibility of RNA having NPHS2 gene sequence

### (A) Synthesis of target RNA

The same operation as in the method described in (A) Synthesis of AGXT_RNA in Test Example 5 was performed. Here, one having an NPHS2 gene sequence, described below, was used as oligo DNA. The NPHS2 gene sequence here used contains the 483-th to 501-th base sequences of GenBank accession no. NM_014625.4, and furthermore contains the mutation (rs74315342) of nucleoside, called c.413G>A.

**[Table 34]**

| | Sequence | SEQ ID NO: |
|---|---|---|
| NPHS2_FW | AATTCGAGTATGAAAGAGTAATTATATTCCAA CTGGGACATCTGCTTCCTGGAAGAA | 111 |
| NPHS2_RV | AGCTTTCTTCCAGGAAGCAGATGTCCCAGTT GGAATATAATTACTCTTTCATACTCG | 112 |

### (B) Evaluation of editing inducibility and results thereof

The method of evaluation of editing inducibility of each of the compounds of Examples was the same method as in (B) to (D) of Test Example 5.

### (C) Results of editing analysis

The results of the editing rate by use of Example 77 (AD1_NPHS2.39) are shown in Fig. 15A. The RNA editing efficiency of the chemically modified compound of Example 77 (AD1_NPHS2.39), with ADAR1p110, was exhibited as a high editing rate as compared with one where no gRNA was added. The compound of the present Example is indicated to be able to repair the NPHS2 gene sequence mutated.

The results of the editing rates by use of the compound of Example 83 (AD1_NPHS2.39e) and the compound of Example 87 (AD1_NPHS2.52e) are shown in Fig. 15D. The RNA editing efficiency of the chemically modified compound of Example 77 (AD1_NPHS2.39), with ADAR1p110, was exhibited as a high editing rate as compared with one where no gRNA was added. The compounds of the present Examples are indicated to be able to repair the NPHS2 gene sequence mutated.

### (Test Example 27) Evaluation of editing inducibility of RNA having GRIA2 gene sequence

### (A) Synthesis of target RNA

The same operation as in the method described in (A) Synthesis of AGXT_RNA in Test Example 5 was performed. Here, one having a GRIA2 gene sequence, described in Test Example 21, was used as oligo DNA. The GRIA2 gene sequence here used contains the 2121-th to 2145-th base sequences of GenBank accession no. NM_000826.4, and furthermore the 2135-th adenosine corresponds to a site at which the editing efficiency to inosine is to be reduced in the case of a reduction in expression of ADAR2 in ALS disease.

### (B) Evaluation of editing inducibility and results thereof

The method of evaluation of editing inducibility of each of the compounds of Examples was the same method as in (B) to (D) of Test Example 5.

### (C) Results of editing analysis

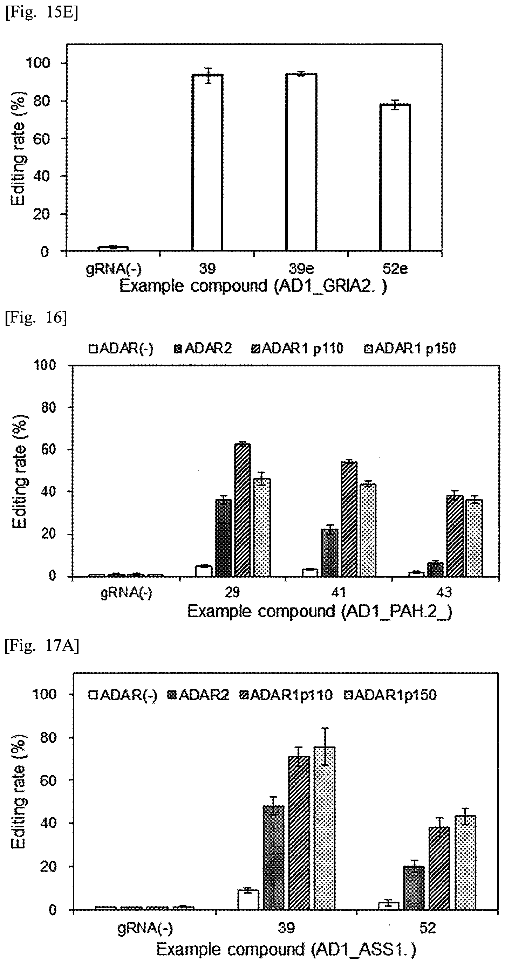

The results of the editing rates by use of the compound of Example 78 (AD1_GRIA2.39), the compound of Example 80 (AD1_GRIA2.39e) and the compound of Example 84 (AD1_GRIA2.52e) are shown in Fig. 15E. The RNA editing efficiency of each of the chemically modified compound of Example 78 (AD1_GRIA2.39), the compound of Example 80 (AD1_GRIA2.39e) and compound of Example 84 (AD1_GRIA2.52e), with ADAR1p110, was exhibited as a high editing rate as compared with one where no gRNA was added. The compounds of the present Examples are indicated to be able to repair the GRIA2 gene sequence mutated. The compounds of the present Examples are indicated to be able to enhance the editing efficiency of the 2279-th adenosine site of the GRIA2 gene sequence mutated, to inosine.

### (Test Example 28) Evaluation of editing inducibility of PAH gene sequence, in cultured cells, with gRNA as compound of Example

The same method as in (A) to (C) of Test Example 10 was used except that the following target RNA expression plasmid expressing a PAH gene was constructed, the compounds of Examples 72 to 74 were each used as gRNA, and a primer corresponding to the PAH gene was used. The PAH gene sequence here used contains the 114638-th to 114662-th base sequences of GenBank accession no. NM_001354304.2, and furthermore contains the mutation (rs5030855) of nucleoside, called c.1066-11G>A.

The editing rates (%) in the case of use of each of the compounds of Examples 72 to 74 (AD1_PAH.2.29, AD1_PAH.2.41, AD1_PAH.2.43) are shown in Fig. 16. In the case where the target RNA expression plasmid and gRNA as the compound of Example were transfected, an editing rate of about 5% was exhibited. Furthermore, in the case where the target RNA expression plasmid and gRNA as each of the compounds of the Examples were transfected together with the plasmid expressing ADAR2, a high editing rate of about 10% to 40% was exhibited. On the other hand, in the case where the target RNA expression plasmid and gRNA as each of the compounds of the Examples were transfected together with the plasmid expressing ADARlp110 or ADAR1p150, a higher editing rate of about 40% to 60% was exhibited. The compounds of the present Examples are indicated to be able to repair the PAH gene sequence mutated.

### (Test Example 29) Evaluation of editing inducibility of ASS1 gene sequence, in cultured cells, with gRNA as compound of Example

The same method as in (A) and (B) of Test Example 10 was used except that the following target RNA expression plasmid expressing an ASS1 gene was constructed, the compounds of Examples 75, 79, 81, and 85 were each used as gRNA, and a primer corresponding to the ASS1 was used. The ASS1 gene sequence here used contains the 1510-th to 1534-th base sequences of GenBank accession no. NM_000050.4, and furthermore contains the mutation (rs121908641) of nucleoside, called c.1168G>A.

Editing analysis was performed as follows. Total RNA was extracted from the cells cultured in a 24-well plate, by use of RNeasy Plus Mini Kit (QIAGEN). cDNA was amplified by performing reverse transcription reaction using PrimeScript II Reverse Transcriptase (TaKaRa), 0.5 µg of Total RNA, and 0.25 µM Oligo(dT)17. PrimeStar GXL DNA polymerase (TaKaRa), T7proGGG primer (SEQ ID NO: 35), and 3'-Adp primer (SEQ ID NO: 48) were used to perform 1st PCR. With cDNA obtained by dilution of the 1st PCR product at 200-fold, used as a template, the ASS1 fragment was amplified by 2nd PCR by use of PrimeStar GXL DNA polymerase (TaKaRa), DS_disease_XhoF01 primer (SEQ ID NO: 44), and DS_disease_KpnR01 primer (SEQ ID NO: 45). Big Dye Terminator v3.1 Cycle Sequence Kit and 0.165 µM DS_disease_XhoF01 primer were used to perform sequencing reaction, and analysis was performed by Applied Biosystem 3500 Genetic Analyzer. The chromatographic chart obtained by the sequencing was adopted and thus the editing rate was calculated from the peak height ratio (G/(G+A)) of the target site.

The editing rates (%) in the case of use of each of the compounds of Example 75 and Example 79 (AD1_ASS1.39, AD1_ASS1.52) are shown in Fig. 17A. In the case where the target RNA expression plasmid and gRNA as each of the compounds of the Examples were transfected, an editing rate of about 5% to 10% was exhibited. Furthermore, in the case where the target RNA expression plasmid and gRNA as each of the compounds of the Examples were transfected together with the plasmid expressing ADAR2, a high editing rate of about 20% to 50% was exhibited. On the other hand, in the case where the target RNA expression plasmid and gRNA as each of the compounds of the Examples were transfected together with the plasmid expressing ADAR1p110 or ADAR1p150, a higher editing rate of about 40% to 80% was exhibited. The compounds of the present Examples are indicated to be able to repair the ASS1 gene sequence mutated.

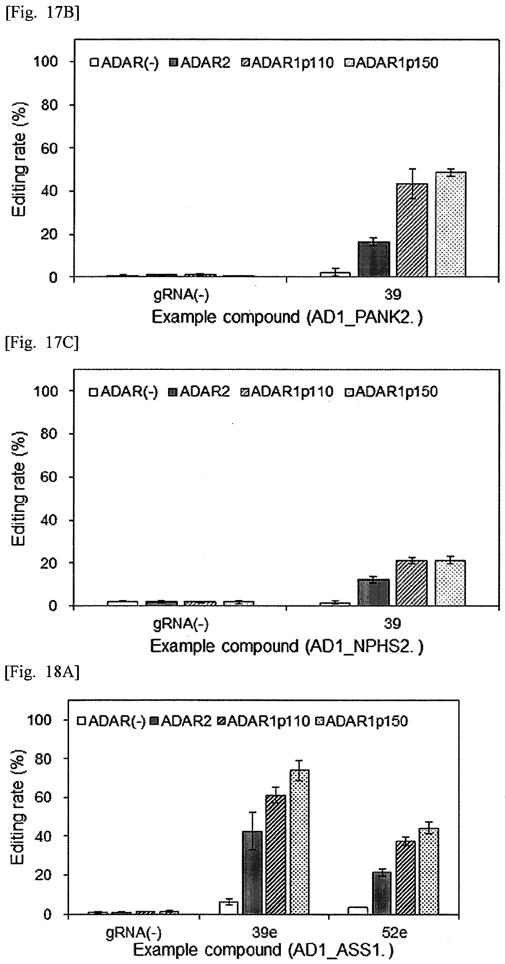

The editing rates (%) in the case of use of each of the compounds of Example 81 and Example 85 (AD1_ASS1.39e, AD1_ASS1.52e) are shown in Fig. 18A. In the case where the target RNA expression plasmid and gRNA as each of the compounds of the Examples were transfected, an editing rate of about 5% was exhibited. Furthermore, in the case where the target RNA expression plasmid and gRNA as each of the compounds of the Examples were transfected together with the plasmid expressing ADAR2, a high editing rate of about 20% to 40% was exhibited. On the other hand, in the case where the target RNA expression plasmid and gRNA as each of the compounds of the Examples were transfected together with the plasmid expressing ADAR1p110 or ADAR1p150, a higher editing rate of about 40% to 80% was exhibited. The compounds of the present Examples are indicated to be able to repair the ASS1 gene sequence mutated.

### (Test Example 30) Evaluation of editing inducibility of PANK2 gene sequence, in cultured cells, with gRNA as compound of Example

The same method as in (A) and (B) of Test Example 10 was used except that the following target RNA expression plasmid expressing a PANK2 gene was constructed, the compounds of Examples 76, 82, and 86 were each used as gRNA and a primer corresponding to the PANK2 gene was used, and the same method as the editing analysis method described in Test Example 29 was used for editing analysis. The PANK2 gene sequence here used contains the 1714-th to 1738-th base sequences of GenBank accession no. NM_153638.3, and furthermore contains the mutation (rs137852959) of nucleoside, called c.1561G>A.

The editing rates (%) in the case of use of the compound of Example 76 (AD1_PINK2.39) are shown in Fig. 17B. In the case where the target RNA expression plasmid and gRNA as the compound of Example were transfected, an editing rate of about 5% was exhibited. In the case where the target RNA expression plasmid and gRNA as the compound of Example were transfected together with the plasmid expressing ADAR2, a high editing rate of about 20% was exhibited. In the case where the target RNA expression plasmid and gRNA as the compound of Example were transfected together with the plasmid expressing ADAR1p110 or ADAR1p150, a high editing rate of about 40% to 50% was exhibited. The compound of the present Example is indicated to be able to repair the PANK2 gene sequence mutated.

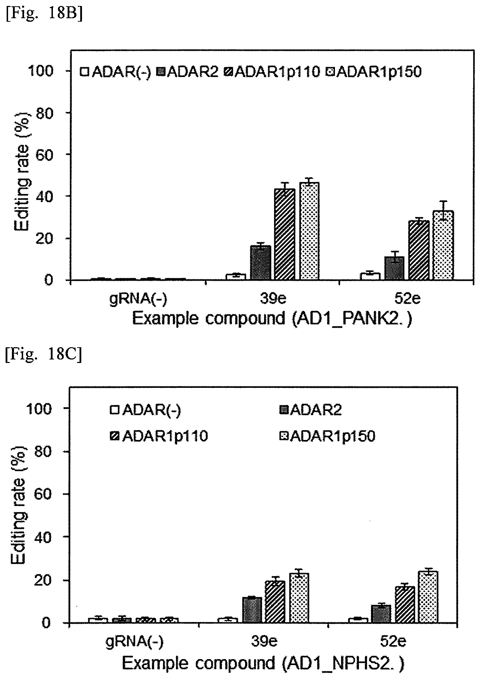

The editing rates (%) in the case of use of each of the compound of Example 82 (AD1_PINK2.39e) and the compound of Example 85 (AD1_PINK2.52e) are shown in Fig. 18B. In the case where the target RNA expression plasmid and gRNA as each of the compounds of the Examples were transfected, an editing rate of about 5% was exhibited. In the case where the target RNA expression plasmid and gRNA as each of the compounds of the Examples were transfected together with the plasmid expressing ADAR2, a high editing rate of about 10% to 20% was exhibited. In the case where the target RNA expression plasmid and gRNA as each of the compounds of the Examples were transfected together with the plasmid expressing ADAR1p110 or ADAR1p150, a high editing rate of about 30% to 50% was exhibited. The compounds of the present Examples are indicated to be able to repair the PANK2 gene sequence mutated.

### (Test Example 31) Evaluation of editing inducibility of NPHS2 gene sequence, in cultured cells, with gRNA as compound of Example

The same method as in (A) and (B) of Test Example 10 was used except that the following target RNA expression plasmid expressing an NPHS2 gene was constructed, the compounds of Examples 77, 83, and 87 were each used as gRNA and a primer corresponding to the NPHS2 gene was used, and the same method as the editing analysis method described in Test Example 29 was used for editing analysis. The NPHS2 gene sequence here used contains the 483-th to 501-th base sequences of GenBank accession no. NM_014625.4, and furthermore contains the mutation (rs74315342) of nucleoside, called c.413G>A.

The results of the editing rate (%) in the case of use of the compound of Example 77 (AD1_NPHS2.39) are shown in Fig. 17C. In the case where the target RNA expression plasmid and gRNA as the compound of Example were transfected together with the plasmid expressing ADAR2, a high editing rate of about 10% was exhibited. In the case where the target RNA expression plasmid and gRNA as the compound of Example were transfected together with the plasmid expressing ADAR1p110 or ADAR1p150, a high editing rate of about 20% was exhibited. The compound of the present Example is indicated to be able to repair the NPHS2 gene sequence mutated.

The results of the editing rates (%) in the cases of use of the compound of Example 83 (AD1_NPHS2.39e) and the compound of Example 87 (AD1_NPHS.52e) are shown in Fig. 18C. In the case where the target RNA expression plasmid and gRNA as each of the compounds of the Examples were transfected together with the plasmid expressing ADAR2, a high editing rate of about 10% was exhibited. In the case where the target RNA expression plasmid and gRNA as each of the compounds of the Examples were transfected together with the plasmid expressing ADAR1p110 or ADAR1p150, a high editing rate of about 20% was exhibited. The compounds of the present Examples are indicated to be able to repair the NPHS2 gene sequence mutated.

### (Test Example 32) Evaluation of editing inducibility of GRIA2 gene sequence, in cultured cells, with gRNA as compound of Example

The same method as in (A) and (B) of Test Example 10 was used except that the following target RNA expression plasmid expressing a GRIA2 gene was constructed, the compounds of Examples 78, 80, and 84 were each used as gRNA and a primer corresponding to the GRIA2 gene was used, and the same method as the editing analysis method described in Test Example 29 was used for editing analysis. The GRIA2 gene sequence here used contains the 2265-th to 2289-th base sequences of GenBank accession no. NM 000826.3, and furthermore the 2279-th adenosine corresponds to a site at which the editing efficiency to inosine is to be reduced in the case of a reduction in expression of ADAR2 in ALS disease.

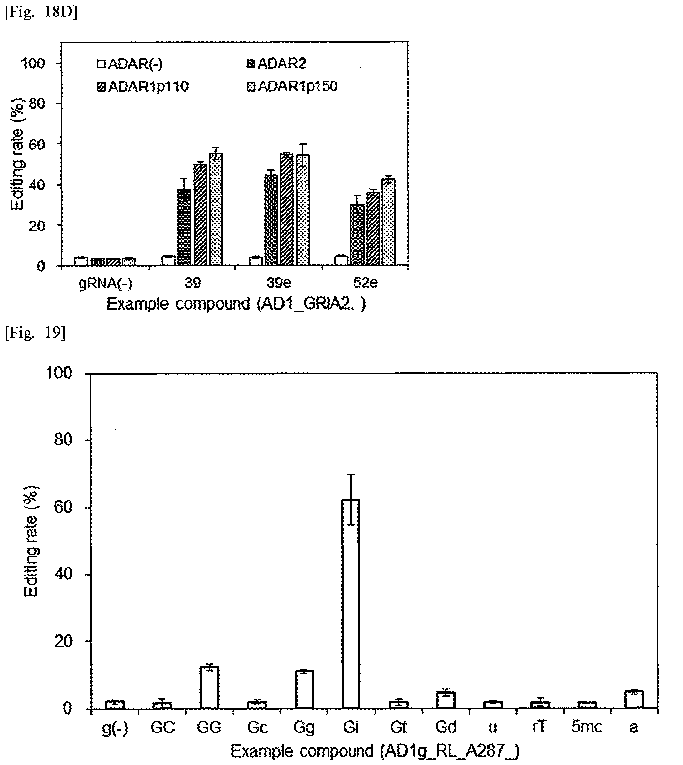

The results of the editing rates (%) in the cases of use of the compound of Example 78 (AD1_GRIA2.39), the compound of Example 80 (AD1_GRIA2.39e) and the compound of Example 84 (AD1_GRIA2.52e) are shown in Fig. 18D. In the case where the target RNA expression plasmid and gRNA as each of the compounds of the Examples were transfected together with the plasmid expressing ADAR2, a high editing rate of about 40% was exhibited. In the case where the target RNA expression plasmid and gRNA as each of the compounds of the Examples were transfected together with the plasmid expressing ADAR1p110 or ADAR1p150, a high editing rate of about 40% to 60% was exhibited. The compounds of the present Examples are indicated to be able to enhance the editing efficiency of the 2279-th adenosine site of the GRIA2 gene sequence mutated, to inosine.

### (Test Example 33) Evaluation of editing inducibility of model target RNA (Rluc_sRNA) with gRNA as compound of Example

### (A) Synthesis of RNA with G as base sequence adjacent to 5'-side of editing site

Rluc_sRNA described in Example 10 of WO2021/060527 was used as one with G as the base sequence adjacent to the 5'-side of the editing site. One with A as the base sequence adjacent to the 5'-side of the editing site was prepared with oligo DNA described below.

### (B) Synthesis of RNA with A as base sequence adjacent to 5'-side of editing site

With psiCHECK^{™}-2_Rluc_W104X prepared by an ordinary method, used as a template, 1st PCR (30 cycles, denaturation: 98°C, 10 seconds, annealing: 55°C, 15 seconds, extension: 68°C, 20 seconds) was performed with PrimeStar GXL DNA Polymerase (TaKaRa), by use of RL_s01_G286A_EcoF1 primer (SEQ ID NO: 64) and Rluc_G286A_R01 primer (SEQ ID NO: 65) for the 5'-side fragment and Rluc_G286A_F01 primer (SEQ ID NO: 66) and RL_s01_G286A_HinR1 primer (SEQ ID NO: 67) for the 3'-side fragment. Next, each PCR product was diluted 100-fold, subjected to 2nd PCR (30 cycles, denaturation: 98°C, 10 seconds, annealing: 55°C, 15 seconds, extension: 68°C, 20 seconds) with PrimeStar GXL DNA Polymerase (TaKaRa), by use of RL_s01_G286A_EcoF1 primer (SEQ ID NO: 64) and RL_s01_G286A_HinR1 primer (SEQ ID NO: 67), and purified by phenol/chloroform extraction and ethanol precipitation, and DNA (Rluc_K41R_W104X_G286A_Eco/Hid) (SEQ ID NO: 72) having a sequence where G286 of Rluc_K41R_W104X was mutated to A and having a restriction enzyme site was obtained. pUC19 and the obtained DNA were subjected to restriction enzyme digestion using EcoRI (TaKaRa) and HindIII (TaKaRa) at 37°C for 1 hour, and then each DNA was purified by phenol/chloroform extraction and ethanol precipitation. Rluc_K41R_W104X_G286A and pUC19 after the restriction enzyme digestion were mixed at a molar ratio of 1:3, and ligation reaction was performed by use of DNA Ligation Kit <Mighty Mix> (TaKaRa). The obtained ligation sample was transformed into DH5α, and cultured with an LB agar plate at 37°C overnight. Colonies selected were cultured in an LB liquid medium at 37°C overnight, and a plasmid was extracted with QIAprep Spin Miniprep Kit (QIAGEN). Thereafter, the sequence of the plasmid, obtained by base sequence analysis, was confirmed. With the sequence-confirmed plasmid used as a template, a template DNA for *in vitro* transcription reaction was amplified by PCR (30 cycles (denaturation: 98°C 10 seconds, annealing: 55°C 15 seconds, extension: 68°C 20 seconds)), by use of T7_Rluc_sRNA_F01 (SEQ ID NO: 68) and Rluc_sRNA_R01 primer (SEQ ID NO: 69), and PrimeStar GXL DNA Polymerase (TaKaRa), and purified by phenol/chloroform extraction and ethanol precipitation. Rluc_sRNA_G286A (SEQ ID NO: 73) was performed by *in vitro* transcription with AmpliScribe T7 Kit (Epicentre Biotechnologies). Rluc_sRNA_G286A synthesized was subjected to cutout and purification with a 5% polyacrylamide gel containing 8 M urea, and used for the subsequent tests.

**[Table 35]**

| Primer | Sequence | SEQ ID NO: |
|---|---|---|
| RL_s01_G286A_EcoF1 | GCAGAATTCGGGCTGGACTCCTTCATCAAC | 66 |
| Rluc_G286A_R01 | CTTGTAGTGATTCAGGAGGCGATATG | 67 |
| Rluc_G286A_F01 | CATATCGCCTCCTGAATCACTACAAG | 68 |
| RL_s01_G286A_HinR1 | CGTAAGCTTCCAGTCGTGGCCCACAAAG | 69 |
| T7_Rluc_sRNA_F01 | CTAATACGACTCACTATAGGGCTGGACTCCTT CATCAAC | 70 |
| Rluc_sRNA_R01 | CCAGTCGTGGCCCACAAAG | 71 |

**[Table 36]**

| | Sequence | SEQ ID NO: |
|---|---|---|
| Rluc_sRNA_G286A_Eco/Hind | GCAGGATCCGGGCTGGACTCCTTCATCAACT ACTATGATTCCGAGAGGCACGCCGAGAACG CCGTGATTTTTCTGCATGGTAACGCTGCCTCC AGCTACCTGTGGAGGCACGTCGTGCCTCACA TCGAGCCCGTGGCTAGATGCATCATCCCTGA TCTGATCGGAATGGGTAAGTCCGGCAAGAG CGGGAATGGCTCATATCGCCTCCTGAATCAC TACAAGTACCTCACCGCTTAGTTCGAGCTGCT GAACCTTCCAAAGAAAATCATCTTTGTGGGC CACGACTGGAAGCTTACG | 72 |
| Rluc_sRNA_G286A | GGGCUGGACUCCUUCAUCAACUACUAUGAU UCCGAGAGGCACGCCGAGAACGCCGUGAU UUUUCUGCAUGGUAACGCUGCCUCCAGCUA CCUGUGGAGGCACGUCGUGCCUCACAUCG AGCCCGUGGCUAGAUGCAUCAUCCCUGAU CUGAUCGGAAUGGGUAAGUCCGGCAAGAG CGGGAAUGGCUCAUAUCGCCUCCUGAAUC ACUACAAGUACCUCACCGCUUAGUUCGAGC UGCUGAACCUUCCAAAGAAAAUCAUCUUUG UGGGCCACGACUGG | 73 |

### (C) Evaluation of editing inducibility

The editing inducibility was evaluated according to the method described in Example 10 of WO2021/060527.

### (D) Results of editing analysis

The editing activity to Rluc_sRNA with G as nucleoside adjacent to the 5'-side of the editing site was evaluated with respect to the compounds of Examples 88 to 96. The results are shown in Fig. 19. AD1g_03_RL_A287 and AD1g_03_RL_A287_GG described in Example 10 of WO2021/060527 were respectively defined as Reference Examples 15 and 16, and compared. The compound of Reference Example 15 was designated as AD1g_RL_A287_GC in Fig. 19, and the compound of Reference Example 16 was designated as AD1g_RL_A287_GG in Fig. 19. The compound of Reference Example 16 exhibited a higher editing activity than the compound of Reference Example 15, in accord with the description in Example 10 of WO2021/060527. Furthermore, the compounds of Examples 88 to 96 were evaluated, and thus each of the compounds of Examples 89 and 90, in which G as nucleoside adjacent to the 5'-side of the editing site was 2'-deoxyguanosine or 2'-deoxyinosine, exhibited a high editing activity as compared with the compound of Reference Example 16. In particular, the compound of Example 90, in which G as nucleoside adjacent to the 5'-side of the editing site was 2'-deoxyinosine, exhibited the highest activity.

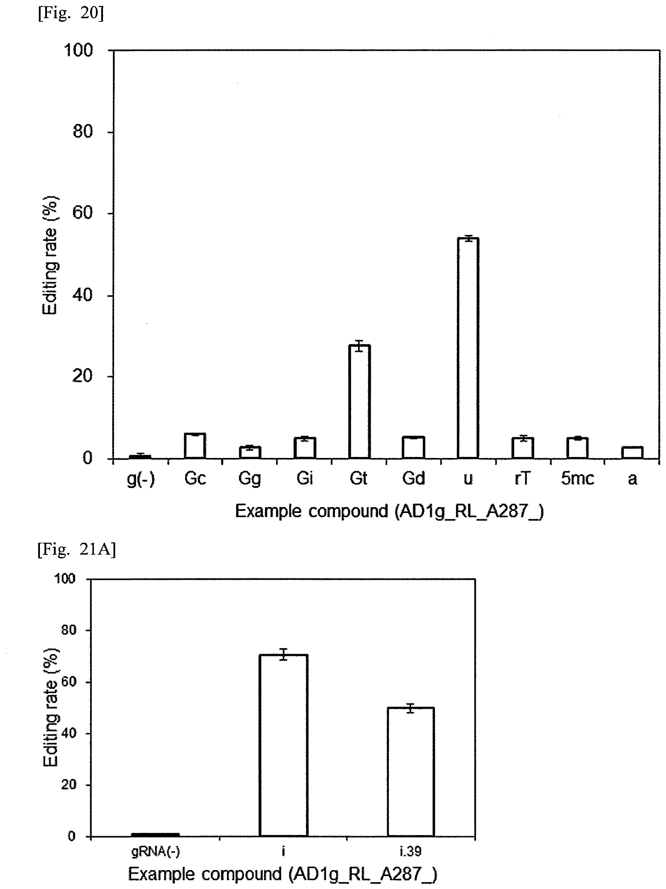

The editing activity to Rluc_sRNA with A as nucleoside adjacent to the 5'-side of the editing site was evaluated with respect to the compounds of Examples 88 to 96. The results are shown in Fig. 20. The compounds of Examples 88 to 96 were evaluated, and thus each of the compounds of Examples 91 and 93, in which A as nucleoside adjacent to the 5'-side of the editing site was thymidine or 2'-deoxyuridine, exhibited a high editing activity. In particular, the compound of Example 93, in which A as nucleoside adjacent to the 5'-side of the editing site was 2'-deoxyuridine, exhibited the highest activity.

The editing activity to Rluc_sRNA with G as nucleoside adjacent to the 5'-side of the editing site was evaluated with respect to the compounds of Examples 90 and 97. The results are shown in Fig. 21A. The compound of Example 97, entirely chemically modified, exhibited a high activity at the same level of efficiency as that of the compound of Example 90 in which G as nucleoside adjacent to the 5'-side of the editing site was 2'-deoxyinosine.

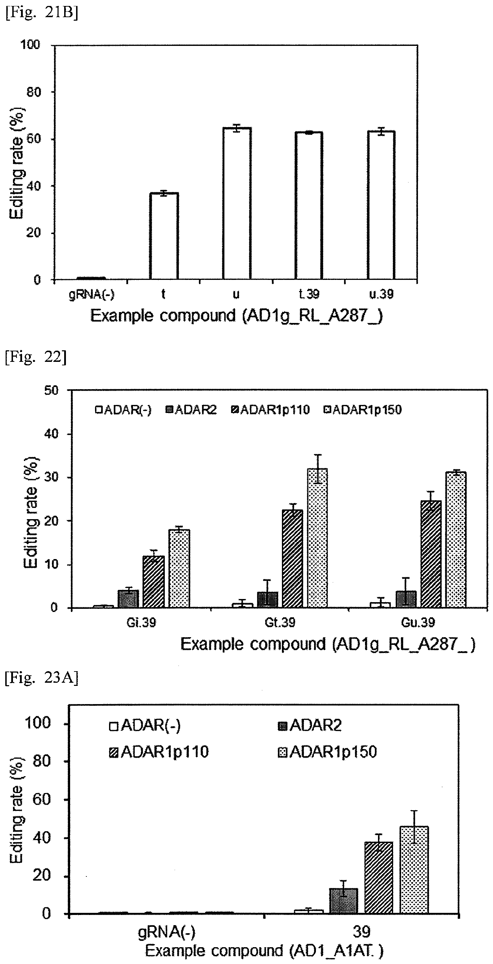

The editing activity to Rluc_sRNA with A as nucleoside adjacent to the 5'-side of the editing site was evaluated with respect to the compounds of Examples 91, 93, 98, and 99. The results are shown in Fig. 21B. Each of the compounds of Examples 98 and 99, entirely chemically modified, exhibited a high activity at a level equal to or more than those of the compounds of Examples 91 and 93, in which A as nucleoside adjacent to the 5'-side of the editing site was thymidine or 2'-deoxyuridine.

### (Test Example 34) Evaluation of editing inducibility of model target RNA (Rluc_sRNA), in cultured cells, with gRNA as compound of Example

### (A) Synthesis of RNA (GAN_RNA) expression plasmid

(pcDNA3.1(-)Hygro_Rluc_sRNA) with G as base sequence adjacent to 5'-side of editing site and RNA (AAN_RNA) expression plasmid
(pcDNA3.1(-)Hygro_Rluc_sRNA_G286A) with A as base sequence adjacent to 5'-side of editing site

A GAN_RNA expression plasmid was constructed with psiCHECK^{™}-2_Rluc_W104X prepared by an ordinary method, used as a template, and an AAN RNA expression plasmid was constructed with pUC19_Rluc_K41R_W104X_G286A prepared by an ordinary method, used as a template. An insert DNA was produced to the template by PCR (30 cycles, denaturation: 98°C, 10 seconds, annealing: 55°C, 15 seconds, extension: 68°C, 20 seconds), with Rluc_s01_BamF1 primer (SEQ ID NO: 74) and RL_s01_G286A_HinR1 primer (SEQ ID NO: 75), and PrimeStar GXL DNA Polymerase (TaKaRa), and purified by phenol/chloroform extraction and ethanol precipitation. The insert DNA and pcDNA3.1(-)Hygro were subjected to reaction by use of BamHI (TaKaRa) and HindIII (TaKaRa) at 37°C for 1 hour, and then the resultant was purified by phenol/chloroform extraction and ethanol precipitation. The insert and plasmid were added at a molar ratio of 3:1, and ligation reaction was performed by use of DNA Ligation Kit<Mighty Mix> (TaKaRa). The obtained ligation sample was transformed into DH5α, and cultured with an LB agar plate at 37°C overnight. Colonies selected were cultured in an LB liquid medium at 37°C overnight, and the plasmid was extracted with QIAprep Spin Miniprep Kit (QIAGEN). One hundred ng of the obtained plasmid was subjected to sequencing reaction using Big Dye Terminator v3.1 Cycle Sequence Kit (Thermo Fisher Scientific), 0.165 µM of pcDNA3_lpro_F01 primer (SEQ ID NO: 76) and 0.165 µM of pcDNA31-seqR01 primer (SEQ ID NO: 77), and it was confirmed by sequence analysis with Applied Biosystems 3500 Genetic Analyzer (Thermo Fisher Scientific) that pcDNA3.1(-)Hygro_Rluc_sRNA and pcDNA3.1(-)Hygro_Rluc_sRNA_G286A was synthesized.

**[Table 37]**

| Primer | Sequence | SEQ ID NO: |
|---|---|---|
| Rluc_s01_BamF1 | GCAGGATCCGGGCTGGACTCCTTCATCAAC | 74 |
| RL_s01_G286A_HinR1 | CGTAAGCTTCCAGTCGTGGCCCACAAAG | 75 |
| pcDNA3_1pro_F01 | TGGCACCAAAATCAACGGG | 76 |
| pcDNA31-seqR01 | GCTATTGTCTTCCCAATCCTCC | 77 |

### (B) Cell culture

The same method as in (B) of Test Example 10 was used except that cDNA3.1(-)Hygro_Rluc_sRNA and pcDNA3.1 (-)Hygro_Rluc_sRNA_G286A were used instead of pcDNA3.1(-)Hygro_AGXT, and the compounds of Examples 97 to 99 were each used as gRNA.

### (C) Editing analysis

Total RNA was extracted from the cells cultured in a 24-well plate, by use of RNeasy Plus Mini Kit (QIAGEN). cDNA was amplified by performing a reverse transcription reaction using ReverTra Ace^{(registered trademark)} qPCR RT Master Mix with gDNA Remover (TOYOBO), and 0.5 µg of Total RNA. PrimeStar GXL DNA polymerase (TaKaRa), Rluc_sRNA_F01 primer (SEQ ID NO: 78), and Rluc_sRNA_R01 primer (SEQ ID NO: 79) were used to perform PCR for fragment amplification. Big Dye Terminator v3.1 Cycle Sequence Kit and 0.165 µM Rluc_sRNA_F01 primer were used to perform sequencing reaction, and analysis was performed by Applied Biosystem 3500 Genetic Analyzer. The chromatographic chart obtained by the sequencing was adopted and thus the editing rate was calculated from the peak height ratio (G/(G+A)) of the target site.

**[Table 38]**

| Primer | Sequence | SEQ ID NO: |
|---|---|---|
| Rluc_sRNA_F01 | GCTGGACTCCTTCATCAAC | 78 |
| Rluc_sRNA_R01 | CCAGTCGTGGCCCACAAAG | 79 |

### (D) Results of editing analysis

The editing rates (%) of Rluc_sRNA where nucleoside adjacent to the 5'-side of the editing site was G, in the case of use of the compound of Example 97, are shown in Fig. 22. In the case where the target RNA expression plasmid and gRNA as the compound of Example were transfected together with the plasmid expressing ADAR2, a high editing rate of about 5% was exhibited. In the case where the target RNA expression plasmid and gRNA as the compound of Example were transfected together with the plasmid expressing ADAR1p110 or ADAR1p150, a high editing rate of about 10% to 20% was exhibited.

The editing rates (%) of Rluc_sRNA where nucleoside adjacent to the 5'-side of the editing site was A, in the case of use of each of the compounds of Examples 98 and 99, are shown in Fig. 22. In the case where the target RNA expression plasmid and gRNA as each of the compounds of the Examples were transfected together with the plasmid expressing ADAR2, a high editing rate of about 5% was exhibited. In the case where the target RNA expression plasmid and gRNA as each of the compounds of the Examples were transfected together with the plasmid expressing ADAR1p110 or ADAR1p150, a high editing rate of about 25% to 35% was exhibited.

### (Test Example 35) Evaluation of editing inducibility of A1AT gene sequence, in cultured cells, with gRNA as compound of Example

The same method as in (A) and (B) of Test Example 10 was used except that the following target RNA expression plasmid expressing an A1AT gene was constructed, the compounds of Examples 72 to 74 were each used as gRNA and a primer corresponding to the A1AT gene was used, and the same method as the editing analysis method described in Test Example 29 was used for editing analysis. The A1AT (also referred to as "SERPINA1") gene sequence here used contains the 1129-th to 1153-th base sequences of GenBank accession no. NM_000295.5, and furthermore contains the mutation (rs28929474) of nucleoside, called c.1096G>A.

The editing rates (%) in the case of use of the compound of Example 136 (AD1_A1 AT.39) are shown in Fig. 23A. In the case where the target RNA expression plasmid and gRNA as the compound of the Example were transfected together with the plasmid expressing ADAR2, a high editing rate of about 20% was exhibited. In the case where the target RNA expression plasmid and gRNA as the compound of Example were transfected together with the plasmid expressing ADAR1p110 or ADAR1p150, a high editing rate of about 40% to 50% was exhibited. The compound of the present Example is indicated to be able to repair the A1AT gene sequence mutated.

### (Test Example 36) Evaluation of editing inducibility of A1AT gene sequence, in cultured cells, with gRNA as compound of Example

The A1AT (also referred to as "SERPINA1") gene sequence here used contains the base sequence of GenBank accession no. NM_000295.5, and furthermore Z-A1AT contains the mutation (rs28929474) of nucleoside, called c.1096G>A.

### (A) Synthesis of A1AT and Z-A1AT expression plasmids

Total RNA was extracted from HepG2 cells, by use of Sepasol RNA I Super G (NACALAI TESQUE, INC.), subjected to DNase treatment using Recombinant DNase I (TaKaRa), and then purified by phenol/chloroform extraction and ethanol precipitation. cDNA was amplified by performing reverse transcription reaction using PrimeScript II Reverse Transcriptase (TaKaRa), 0.5 µg of Total RNA, and 0.25 µM Oligo(dT)17. DNA having CDS sequence of SERPINA1 (SERPINA1_CDS) (SEQ ID NO: 88) was obtained by PCR (30 cycles, denaturation: 98°C, 10 seconds, annealing: 55°C, 15 seconds, extension: 68°C, one minute and thirty seconds) by use of PrimeStar GXL DNA polymerase (TaKaRa), SERPINA1_CDS_XbaF1 primer (SEQ ID NO: 80), and SERPINA1_CDS_HinR1 primer (SEQ ID NO: 81), and purification by phenol/chloroform extraction and ethanol precipitation. pUC19 and the obtained DNA were subjected to reaction by use of XbaI (TaKaRa) and HindIII (TaKaRa) at 37°C for 1 hour, and then purified by phenol/chloroform extraction and ethanol precipitation. The insert and plasmid were added at a molar ratio of 3:1, and ligation reaction was performed by use of DNA Ligation Kit<Mighty Mix> (TaKaRa). The obtained ligation sample was transformed into DH5α, and cultured with an LB agar plate at 37°C overnight. Colonies selected were cultured in an LB liquid medium at 37°C overnight, and the plasmid was extracted with QIAprep Spin Miniprep Kit (QIAGEN). One hundred ng of the obtained plasmid was subjected to sequencing reaction using Big Dye Terminator v3.1 Cycle Sequence Kit (Thermo Fisher Scientific), 0.165 µM of pUC19-seqF01 primer (SEQ ID NO: 82) and 0.165 µM of pUC19_seqR01 primer (SEQ ID NO: 83), and it was confirmed by sequence analysis with Applied Biosystems 3500 Genetic Analyzer (Thermo Fisher Scientific) that pUC19_SERPINA1 was synthesized.

With pUC 19_SERPINA1 used as a template, 1 st PCR was performed with PrimeStar GXL DNA Polymerase (TaKaRa), by use of SERPINA1_CDS_XbaF1 primer (SEQ ID NO: 80) and SERPINA1_G1096A_R1 primer (SEQ ID NO: 84) for the 5'-side fragment and SERPINA1_G1096A_F1 primer (SEQ ID NO: 85) and SERPINA1_CDS_HinR1 primer (SEQ ID NO: 81) for the 3'-side fragment. Next, each PCR product was diluted 100-fold, subjected to 2nd PCR (30 cycles, denaturation: 98°C, 10 seconds, annealing: 55°C, 15 seconds, extension: 68°C, one minute and thirty seconds) with PrimeStar GXL DNA Polymerase (TaKaRa), by use of SERPINA1_CDS_XbaF1 primer (SEQ ID NO: 80) and SERPINA1_CDS_HinR1 primer (SEQ ID NO: 81), and purified by phenol/chloroform extraction and ethanol precipitation, and DNA having a sequence where G1096 of SERPINA1 was mutated to A (SERPINA1_G1096A) (SEQ ID NO: 89) was obtained. With pUC19_SERPINA1 used as a template, a CDS sequence of SERPINA1 (SERPINA1_CDS) (SEQ ID NO: 88) was obtained by PCR (30 cycles, denaturation: 98°C, 10 seconds, annealing: 55°C, 15 seconds, extension: 68°C, one minute and thirty seconds) by PrimeStar GXL DNA Polymerase (TaKaRa), by use of SERPINA1_CDS_XbaF1 primer (SEQ ID NO: 80) and SERPINA1_CDS_HinR1 primer (SEQ ID NO: 81), and purification by phenol/chloroform extraction and ethanol precipitation. The obtained DNA and pcDNA3.1(-)Hygro were subjected to reaction by use of XbaI (TaKaRa) and HindIII (TaKaRa) at 37°C for 1 hour, and then the resultant was purified by phenol/chloroform extraction and ethanol precipitation. The insert and plasmid were added at a molar ratio of 3:1, and ligation reaction was performed by use of DNA Ligation Kit<Mighty Mix> (TaKaRa). The obtained ligation sample was transformed into DH5α, and cultured with an LB agar plate at 37°C overnight. Colonies selected were cultured in an LB liquid medium at 37°C overnight, and the plasmid was extracted with QIAprep Spin Miniprep Kit (QIAGEN). One hundred ng of the obtained plasmid was subjected to sequencing reaction using Big Dye Terminator v3.1 Cycle Sequence Kit (Thermo Fisher Scientific), 0.165 µM of pcDNA3_1pro_F01 primer (SEQ ID NO: 86) and 0.165 µM of pcDNA31-seqR01 primer (SEQ ID NO: 87), and it was confirmed by sequence analysis with Applied Biosystems 3500 Genetic Analyzer (Thermo Fisher Scientific) that pcDNA3.1(-)Hygro_SERPINA1_G1096A and pcDNA3.1(-)Hygro_SERPINA1 was synthesized.

**[Table 39]**

| Primer | Sequence | SEQ ID NO: |
|---|---|---|
| SERPINA1_CDS_XbaF1 | CGATCTAGAATGCCGTCTTCTGTCTCGTG | 80 |
| SERPINA1_CDS_HinR1 | GCAAAGCTTTTATTTTTGGGTGGGATTCACC | 81 |
| pUC19-seqF01 | CAACTGTTGGGAAGGGCGATC | 82 |
| pUC19_seqR01 | CGACAGGTTTCCCGACTGGAAAG | 83 |
| SERPINA1_G1096A_R1 | GTCCCTTTCTTGTCGATGGTC | 84 |
| SERPINA1_G1096A_F1 | GACCATCGACAAGAAAGGGAC | 85 |
| pcDNA3_1pro_F01 | TGGCACCAAAATCAACGGG | 86 |
| pcDNA3.1-seqR01 | GCTATTGTCTTCCCAATCCTCC | 87 |

**[Table 40]**

| | Sequence | SEQ ID NO: |
|---|---|---|
| SERPINA1_CDS | CGATCTAGAATGCCGTCTTCTGTCTCGTGGGGCATCCTCCTGCTGGCAGGCCT GTGCTGCCTGGTCCCTGTCTCCCTGGCTGAGGATCCCCAGGGAGATGCTGCC CAGAAGACAGATACATCCCACCATGATCAGGATCACCCAACCTTCAACAAGAT CACCCCCAACCTGGCTGAGTTCGCCTTCAGCCTATACCGCCAGCTGGCACAC CAGTCCAACAGCACCAATATCTTCTTCTCCCCAGTGAGCATCGCTACAGCCTTT GCAATGCTCTCCCTGGGGACCAAGGCTGACACTCACGATGAAATCCTGGAGG GCCTGAATTTCAACCTCACGGAGATTCCGGAGGCTCAGATCCATGAAGGCTTC CAGGAACTCCTCCGTACCCTCAACCAGCCAGACAGCCAGCTCCAGCTGACCA CCGGCAATGGCCTGTTCCTCAGCGAGGGCCTGAAGCTAGTGGATAAGTTTTTG GAGGATGTTAAAAAGTTGTACCACTCAGAAGCCTTCACTGTCAACTTCGGGGA CACCGAAGAGGCCAAGAAACAGATCAACGATTACGTGGAGAAGGGTACTCA AGGGAAAATTGTGGATTTGGTCAAGGAGCTTGACAGAGACACAGTTTTTGCTC TGGTGAATTACATCTTCTTTAAAGGCAAATGGGAGAGACCCTTTGAAGTCAAG GACACCGAGGAAGAGGACTTCCACGTGGACCAGGTGACCACCGTGAAGGTG CCTATGATGAAGCGTTTAGGCATGTTTAACATCCAGCACTGTAAGAAGCTGTCC AGCTGGGTGCTGCTGATGAAATACCTGGGCAATGCCACCGCCATCTTCTTCCT GCCTGATGAGGGGAAACTACAGCACCTGGAAAATGAACTCACCCACGATATC ATCACCAAGTTCCTGGAAAATGAAGACAGAAGGTCTGCCAGCTTACATTTACC CAAACTGTCCATTACTGGAACCTATGATCTGAAGAGCGTCCTGGGTCAACTGG GCATCACTAAGGTCTTCAGCAATGGGGCTGACCTCTCCGGGGTCACAGAGGA GGCACCCCTGAAGCTCTCCAAGGCCGTGCATAAGGCTGTGCTGACCATCGAC GAGAAAGGGACTGAAGCTGCTGGGGCCATGTTTTTAGAGGCCATACCCATGT CTATCCCCCCCGAGGTCAAGTTCAACAAACCCTTTGTCTTCTTAATGATTGAAC AAAATACCAAGTCTCCCCTCTTCATGGGAAAAGTGGTGAATCCCACCCAAAAA TAAAAGCTTTGC | 88 |
| SERPINA1_G1096A | CGATCTAGAATGCCGTCTTCTGTCTCGTGGGGCATCCTCCTGCTGGCAGGCCT GTGCTGCCTGGTCCCTGTCTCCCTGGCTGAGGATCCCCAGGGAGATGCTGCC CAGAAGACAGATACATCCCACCATGATCAGGATCACCCAACCTTCAACAAGAT CACCCCCAACCTGGCTGAGTTCGCCTTCAGCCTATACCGCCAGCTGGCACAC CAGTCCAACAGCACCAATATCTTCTTCTCCCCAGTGAGCATCGCTACAGCCTTT GCAATGCTCTCCCTGGGGACCAAGGCTGACACTCACGATGAAATCCTGGAGG GCCTGAATTTCAACCTCACGGAGATTCCGGAGGCTCAGATCCATGAAGGCTTC CAGGAACTCCTCCGTACCCTCAACCAGCCAGACAGCCAGCTCCAGCTGACCA CCGGCAATGGCCTGTTCCTCAGCGAGGGCCTGAAGCTAGTGGATAAGTTTTTG GAGGATGTTAAAAAGTTGTACCACTCAGAAGCCTTCACTGTCAACTTCGGGGA CACCGAAGAGGCCAAGAAACAGATCAACGATTACGTGGAGAAGGGTACTCA AGGGAAAATTGTGGATTTGGTCAAGGAGCTTGACAGAGACACAGTTTTTGCTC TGGTGAATTACATCTTCTTTAAAGGCAAATGGGAGAGACCCTTTGAAGTCAAG GACACCGAGGAAGAGGACTTCCACGTGGACCAGGTGACCACCGTGAAGGTG CCTATGATGAAGCGTTTAGGCATGTTTAACATCCAGCACTGTAAGAAGCTGTCC AGCTGGGTGCTGCTGATGAAATACCTGGGCAATGCCACCGCCATCTTCTTCCT GCCTGATGAGGGGAAACTACAGCACCTGGAAAATGAACTCACCCACGATATC ATCACCAAGTTCCTGGAAAATGAAGACAGAAGGTCTGCCAGCTTACATTTACC CAAACTGTCCATTACTGGAACCTATGATCTGAAGAGCGTCCTGGGTCAACTGG GCATCACTAAGGTCTTCAGCAATGGGGCTGACCTCTCCGGGGTCACAGAGGA GGCACCCCTGAAGCTCTCCAAGGCCGTGCATAAGGCTGTGCTGACCATCGAC AAGAAAGGGACTGAAGCTGCTGGGGCCATGTTTTTAGAGGCCATACCCATGT CTATCCCCCCCGAGGTCAAGTTCAACAAACCCTTTGTCTTCTTAATGATTGAAC AAAATACCAAGTCTCCCCTCTTCATGGGAAAAGTGGTGAATCCCACCCAAAAA TAAAAGCTTTGC | 89 |

### (B) Cell culture

HEK293 cells were subcultured on a 24-well plate at 1.0 × 10⁵ cells/well by use of DMEM (SIGMA) containing 10% FBS, and cultured for 48 hours. The resultant was washed once with PBS(-) (NACALAI TESQUE, INC.), and the medium was exchanged with DMEM (SIGMA) containing 0.2% FBS. Lipofectamine 3000 (Thermo) was used for transfecting 50 ng of a plasmid (pcDNA3.1(-)Hygro_SERPINA1_G1096A, or pcDNA3.1(-)Hygro_SERPINA1) expressing target RNA, 500 ng of a plasmid (pcDNA3.1(-)Hygro_ADAR1p110 or pcDNA3.1(-)Hygro_ADAR1p150) expressing ADAR, and 50 nM gRNA as the compound of Example, and culture for 48 hours.

### (C) Editing analysis and results thereof

Total RNA was extracted from the cells cultured in a 24-well plate, by use of RNeasy Plus Mini Kit (QIAGEN). cDNA was amplified by performing reverse transcription reaction using ReverTra Ace^{(registered trademark)}qPCR RT Master Mix with gDNA Remover (TOYOBO), and 0.5 µg of Total RNA. PrimeStar GXL DNA polymerase (TaKaRa), SERPINA1_1stF01 primer (SEQ ID NO: 90), and SERPINA1_CDS_HinR1 primer (SEQ ID NO: 91) were used to perform 1st PCR. With cDNA obtained by dilution of the 1st PCR product at 400-fold, used as a template, the SERPINA1 fragment was amplified by 2nd PCR by use of PrimeStar GXL DNA polymerase (TaKaRa), SERPINA1_2ndF01 primer (SEQ ID NO: 92), and SERPINA1_2ndR01 primer (SEQ ID NO: 93). Big Dye Terminator v3.1 Cycle Sequence Kit and 0.165 µM SERPINA1_editF01 primer (SEQ ID NO: 94) were used to perform sequencing reaction, and analysis was performed by Applied Biosystem 3500 Genetic Analyzer. The chromatographic chart obtained by the sequencing was adopted and thus the editing rate was calculated from the peak height ratio (G/(G+A)) of the target site.

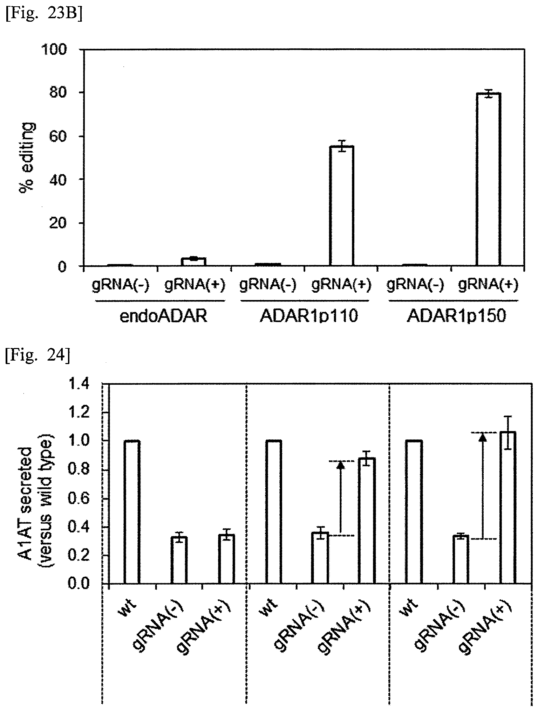

The editing rates (%) in the case of use of the compound of Example 136 (AD1_A1AT.39) are shown in Fig. 23B. In the case where the target RNA (Z-A1AT) expression plasmid and gRNA as the compound of Example were transfected, a high editing rate of about 5% was obtained. In the case where the target RNA expression plasmid and gRNA as the compound of Example were transfected together with the plasmid expressing ADAR1p110, a high editing rate of about 60% was exhibited. Furthermore, in the case where the target RNA expression plasmid and gRNA as the compound of Example were transfected together with the plasmid expressing ADAR1p150, a high editing rate of about 80% was exhibited. The compound of the present Example is indicated to be able to repair the A1AT gene sequence mutated.

**[Table 41]**

| Primer | Sequence | SEQ ID NO: |
|---|---|---|
| SERPINA1_1stF01 | GGCATGTTTAACATCCAGCACTG | 90 |
| SERPINA1_CDS_HinR1 | GCAAAGCTTTTATTTTTGGGTGGGATTCACC | 91 |
| SERPINA1_2ndF01 | CCATCTTCTTCCTGCCTGATG | 92 |
| SERPINA1_2ndR01 | CATGAAGAGGGGAGACTTGG | 93 |
| SERPINA1_editF01 | GAACTCACCCACGATATCATCAC | 94 |

### (D) Analysis of amount of secretion of A1AT by ELISA, and results thereof

After transfection, the medium after culture for 48 hours was recovered, and subjected to centrifugation at 4°C and 3000 × g for 10 minutes. The amount of secretion of A1AT was measured by use of 50 µL of the medium and Human alpha 1 Antitrypsin (SERPINA1) ELISA Kit (abcam), according to the attached company protocol. The absorbance was measured with a Nivo multimode microplate reader (PerkinElmer).

The amount of secretion of A1AT in the medium in the case of use of the compound of Example 136 (AD1_A1AT.39) is shown in Fig. 24. In the case where the target RNA (Z-A1AT) expression plasmid was transfected together with the plasmid expressing ADAR1p110, the amount of A1AT secreted in the medium was about 30% of the amount of A1AT secreted in the medium in the case of transfection of a plasmid expressing normal A1AT. In the case where the target RNA (Z-A1AT) expression plasmid and the compound of Example 136 were simultaneously transfected together with the plasmid expressing ADAR1p110, the amount of A1AT secreted in the medium was restored to the same level as the amount of A1AT secreted in the medium in the case of transfection of a plasmid expressing normal A1AT.

Furthermore, in the case where the target RNA (Z-A1AT) expression plasmid was transfected together with a plasmid expressing ADAR1p110 and ADAR1p150, the amount of A1AT secreted in the medium was about 30% of the amount of A1AT secreted in the medium in the case of transfection of a plasmid expressing normal A1AT. In the case where the target RNA (Z-A1AT) expression plasmid and the compound of Example 136 were simultaneously transfected together with a plasmid expressing ADAR1p110 and ADAR1p150, the amount of A1AT secreted in the medium was restored to the same level as the amount of A1AT secreted in the medium in the case of transfection of a plasmid expressing normal A1AT.

It was considered from the results of the RNA editing activity shown in (C) and the analysis results of the amount of secretion of A1AT shown in (D) that the compound of Example 136 repaired mutation of mRNA of Z-ALAT to normal A1AT mRNA in cells and furthermore secrete a normal A1AT protein from cells up to a level of a normal amount.

The disclosures of Japanese Patent Application No. 2020-203658 (filing date: December 8, 2020) and Japanese Patent Application No. 2021-157151 (filing date: September 27, 2021) are herein incorporated by reference in their entirety. All publications, patent applications, and technical standards recited herein are incorporated by reference to the same extent as if each individual publication, patent application, and technical standard were specifically set forth herein.

Sequence List

## Claims

1. An oligonucleotide, or a pharmaceutically acceptable salt thereof, comprising:
a first oligonucleotide identifying a target RNA; and
a second oligonucleotide linked to the 5'-side of the first oligonucleotide, wherein
the first oligonucleotide consists of
a target-corresponding nucleotide residue corresponding to an adenosine residue in the target RNA,
an oligonucleotide of 10 to 24 residues, linked to the 3'-side of the target-corresponding nucleotide residue and having a base sequence complementary to the target RNA, and
an oligonucleotide of 3 to 6 residues, linked to the 5'-side of the target-corresponding nucleotide residue and having a base sequence complementary to the target RNA,
the second oligonucleotide has no nucleotide residue corresponding to a nucleotide residue of the target RNA or has a nucleotide residue which does not form a complementary pair with a nucleotide residue of the target RNA, at the 3'-end thereof,
a number of residues in the second oligonucleotide is 2 to 10, and at least the nucleotide residues other than at the 3'-end form a double-stranded structure complementary to the target RNA,
a counter region consisting of the target-corresponding nucleotide residue and each one residue at the 3'-side and the 5'-side thereof is contained,
the nucleotide residue linked to the 3'-side of the target-corresponding nucleotide residue is a 2'-deoxynucleotide residue,
the third nucleotide residue counted in the 3'-direction from the target-corresponding nucleotide in the oligonucleotide linked to the 3'-side of the target-corresponding nucleotide residue is a 2'-deoxy-2'-fluoronucleotide residue, and
the oligonucleotide, or a pharmaceutically acceptable salt thereof induces site-specific editing for the target RNA.

2. The oligonucleotide, or a pharmaceutically acceptable salt thereof, according to claim 1, wherein the number of residues in the second oligonucleotide is 4 to 8.

3. The oligonucleotide, or a pharmaceutically acceptable salt thereof, according to claim 1 or 2, wherein the 3'-end of the second oligonucleotide has no nucleotide residue corresponding to a nucleotide residue of the target RNA.

4. The oligonucleotide, or a pharmaceutically acceptable salt thereof, according to any one of claims 1 to 3, wherein the site-specific editing is due to an enzyme reaction by adenosine deaminase 1.

5. The oligonucleotide, or a pharmaceutically acceptable salt thereof, according to any one of claims 1 to 4, comprising a linking portion containing an alkyleneoxy unit between the first oligonucleotide and the second oligonucleotide.

6. The oligonucleotide, or a pharmaceutically acceptable salt thereof, according to any one of claims 1 to 5, wherein the first oligonucleotide contains a phosphorothioate bond.

7. The oligonucleotide, or a pharmaceutically acceptable salt thereof, according to any one of claims 1 to 6, wherein the first oligonucleotide contains at least one modified nucleotide residue selected from the group consisting of a 2'-O-alkyl ribonucleotide residue, a 2'-deoxy-2'-fluoro ribonucleotide residue, a bridged nucleotide residue, and a 2'-deoxyribonucleotide.

8. The oligonucleotide, or a pharmaceutically acceptable salt thereof, according to any one of claims 1 to 7, wherein the counter region contains a phosphorothioate bond.

9. The oligonucleotide, or a pharmaceutically acceptable salt thereof, according to any one of claims 1 to 8, wherein the target-corresponding nucleotide residue contains a phosphorothioate bond.

10. The oligonucleotide, or a pharmaceutically acceptable salt thereof, according to any one of claims 1 to 9, wherein the nucleotide residue linked to the 5'-side of the target-corresponding nucleotide residue is a 2'-O-alkyl ribonucleotide residue or a deoxynucleotide residue.

11. The oligonucleotide, or a pharmaceutically acceptable salt thereof, according to any one of claims 1 to 10, wherein the second oligonucleotide contains at least one modified nucleotide residue selected from the group consisting of a 2'-O-alkyl ribonucleotide residue, a 2'-deoxy-2'-fluoro ribonucleotide residue, and a bridged nucleotide residue.

12. The oligonucleotide, or a pharmaceutically acceptable salt thereof, according to any one of claims 1 to 11, wherein the oligonucleotide linked to the 3'-side of the target-corresponding nucleotide residue has a base sequence in which a 2'-deoxy-2'-fluoronucleotide residue and a 2'-O-alkyl ribonucleotide residue are alternately linked.

13. The oligonucleotide, or a pharmaceutically acceptable salt thereof, according to any one of claims 1 to 12, wherein the oligonucleotide linked to the 3'-side of the target-corresponding nucleotide residue has a base sequence in which a bridged nucleotide residue and a 2'-O-alkyl ribonucleotide residue are alternately linked.

14. The oligonucleotide, or a pharmaceutically acceptable salt thereof, according to any one of claims 1 to 13, wherein the oligonucleotide linked to the 3'-side of the target-corresponding nucleotide residue has a base sequence in which a 2'-deoxy-2'-fluoronucleotide residue and a bridged nucleotide residue are alternately linked.

15. The oligonucleotide, or a pharmaceutically acceptable salt thereof, according to any one of claims 1 to 14, wherein an oligonucleotide linked to the 5'-side of the target-corresponding nucleotide residue in the first oligonucleotide has a base sequence in which a 2'-O-alkyl ribonucleotide residue is linked.

16. The oligonucleotide, or a pharmaceutically acceptable salt thereof, according to any one of claims 1 to 15, wherein an oligonucleotide linked to the 5'-side of the target-corresponding nucleotide residue in the first oligonucleotide has a base sequence in which a 2'-O-alkyl ribonucleotide residue and a bridged nucleotide residue are alternately linked.

17. The oligonucleotide, or a pharmaceutically acceptable salt thereof, according to any one of claims 1 to 16, wherein an oligonucleotide linked to the 5'-side of the target-corresponding nucleotide residue in the first oligonucleotide has a base sequence in which a 2'-O-alkyl ribonucleotide residue and a 2'-deoxy-2'-fluoronucleotide residue are alternately linked.

18. The oligonucleotide, or a pharmaceutically acceptable salt thereof, according to any one of claims 1 to 17, wherein the second oligonucleotide has a base sequence in which a 2'-O-alkyl ribonucleotide residue is linked.

19. The oligonucleotide, or a pharmaceutically acceptable salt thereof, according to any one of claims 1 to 18, wherein the second oligonucleotide has a base sequence in which a 2'-O-alkyl ribonucleotide residue and a bridged nucleotide residue are alternately linked.

20. The oligonucleotide, or a pharmaceutically acceptable salt thereof, according to any one of claims 1 to 19, wherein the second oligonucleotide has a base sequence in which a 2'-O-alkyl ribonucleotide residue and a 2'-deoxy-2'-fluoronucleotide residue are alternately linked.

21. The oligonucleotide, or a pharmaceutically acceptable salt thereof, according to any one of claims 1 to 20, wherein
a cytidine residue is linked to the 5'-side of the adenosine residue to be edited in the target RNA, and
the nucleotide residue linked to the 3'-side of the target-corresponding nucleotide residue in the first oligonucleotide is a 2'-deoxyinosine residue.

22. The oligonucleotide, or a pharmaceutically acceptable salt thereof, according to any one of claims 1 to 20, wherein
a uridine residue is linked to the 5'-side of the adenosine residue to be edited in the target RNA, and
the nucleotide residue linked to the 3'-side of the target-corresponding nucleotide residue in the first oligonucleotide is a 2'-deoxyadenosine residue.

23. The oligonucleotide, or a pharmaceutically acceptable salt thereof, according to any one of claims 1 to 20, wherein
an adenosine residue is linked to the 5'-side of the adenosine residue to be edited in the target RNA, and
the nucleotide residue linked to the 3'-side of the target-corresponding nucleotide residue in the first oligonucleotide is a thymidine residue or a 2'-deoxyuridine residue.

24. The oligonucleotide, or a pharmaceutically acceptable salt thereof, according to any one of claims 1 to 20, wherein
a guanosine residue is linked to the 5'-side of the adenosine residue to be edited in the target RNA, and
the nucleotide residue linked to the 3'-side of the target-corresponding nucleotide residue in the first oligonucleotide is a 2'-deoxyinosine residue.

25. The oligonucleotide, or a pharmaceutically acceptable salt thereof, according to any one of claims 1 to 24, wherein the target-corresponding nucleotide residue is an N-alkylpyrimidine nucleotide residue or a 2'-deoxycytidine residue.

26. The oligonucleotide, or a pharmaceutically acceptable salt thereof, according to any one of claims 1 to 25, wherein the first oligonucleotide and the second oligonucleotide are each formed by linking nucleotide residues by a phosphorothioate bond.

27. The oligonucleotide, or a pharmaceutically acceptable salt thereof, according to any one of claims 1 to 26, wherein the first oligonucleotide contains a bridged nucleotide residue in the tenth or subsequent nucleotide residues counted in the 3'-direction from the target-corresponding nucleotide.

28. The oligonucleotide, or a pharmaceutically acceptable salt thereof, according to claim 27, wherein an oligonucleotide composed of the tenth or subsequent nucleotide residues counted in the 3'-direction from the target-corresponding nucleotide in the first oligonucleotide has a base sequence in which a 2'-O-alkyl ribonucleotide residue and a bridged nucleotide residue are alternately linked.

29. The oligonucleotide, or a pharmaceutically acceptable salt thereof, according to any one of claims 1 to 28, wherein the second oligonucleotide contains a bridged nucleotide residue in the second or subsequent nucleotide residues counted in the 5'-direction from the nucleotide residue at the 3'-end thereof.

30. The oligonucleotide, or a pharmaceutically acceptable salt thereof, according to claim 29, wherein an oligonucleotide composed of the second or subsequent nucleotide residues counted in the 5'-direction from the nucleotide residue at the 3'-end of the second oligonucleotide has a base sequence in which a 2'-O-alkyl ribonucleotide residue and a bridged nucleotide residue are alternately linked.

31. The oligonucleotide, or a pharmaceutically acceptable salt thereof, according to any one of claims 1 to 30, represented by any of the following formulae: 
U(M)^T(L)^G(M)^A(L)^U(M)^G(F)^A(F)^C(M)^C(M)^c^i^G(M)^U(F)^G(
M)^G(F)^C(M)^A(F)^U(M)^C(F)^A(M)^G(L)^U(M)^T(L)^G(M) (AD1_ASS1.39)
 
A(M)^A(L)^G(M)^A(L)^A(M)^A(F)^U(F)^U(M)^C(M)^c^a^A(M)^C(F)^A
(M)^A(F)^A(M)^U(F)^A(M)^C(F)^C(M)^A(L)^C(M)^C(L)^U(M)
(AD1_PANK2.39)
 A(M)^T(L)^G(M)^T(L)^C(M)^C(F)^A(F)^G(M)^U(M)^c^i^G(M)^A(F)^A( 
M)^U(F)^A(M)^U(F)^A(M)^A(F)^U(M)^T(L)^A(M)^C(L)^U(M)
(AD1_NPHS2.39)
 
G(M)^C(L)^A(M)^T(L)^C(M)^C(F)^U(F)^G(M)^C(M)^c^i^C(M)^A(F)^U(
M)^A(F)^A(M)^A(F)^G(M)^G(F)^C(M)^A(L)^C(M)^C(L)^C(M)
(AD1_GRIA2.39)
 
U(M)^G(L)^A(M)^U(L)^G(F)^A(F)^C(M)^C(M)^c^i^G(M)^U(F)^G(M)^G(
F)^C(M)^A(F)^U(M)^C(F)^A(L)^G(M)^U(L)^U(M) (AD1_ASS1.52)
 
G(M)^C(E)^A(M)^T(E)^C(M)^C(F)^U(F)^G(M)^C(M)^c^i^C(M)^A(F)^U(
M)^A(F)^A(M)^A(F)^G(M)^G(F)^C(M)^A(E)^C(M)^C(E)^C(M)
(AD1_GRIA2.39e)
 
U(M)^T(E)^G(M)^A(E)^U(M)^G(F)^A(F)^C(M)^C(M)^c^i^G(M)^U(F)^G(
M)^G(F)^C(M)^A(F)^U(M)^C(F)^A(M)^G(E)^U(M)^T(E)^G(M)
(AD1_ASS1.39e)
 
A(M)^A(E)^G(M)^A(E)^A(M)^A(F)^U(F)^U(M)^C(M)^c^a^A(M)^C(F)^A
(M)^A(F)^A(M)^U(F)^A(M)^C(F)^C(M)^A(E)^C(M)^C(E)^U(M)
(AD1_PANK2.39e)
 
A(M)^T(E)^G(M)^T(E)^C(M)^C(F)^A(F)^G(M)^U(M)^c^i^G(M)^A(F)^A(
M)^U(F)^A(M)^U(F)^A(M)^A(F)^U(M)^T(E)^A(M)^C(E)^U(M)
(AD1_NPHS2.39e)
 
C(M)^A(E)^U(M)^C(E)^C(F)^U(F)^G(M)^C(M)^c^i^C(M)^A(F)^U(M)^A(
F)^A(M)^A(F)^G(M)^G(F)^C(E)^A(M)^C(E)^C(M)) (AD1_GRIA2.52e)
 
U(M)^G(E)^A(M)^T(E)^G(F)^A(F)^C(M)^C(M)^c^i^G(M)^U(F)^G(M)^G(
F)^C(M)^A(F)^U(M)^C(F)^A(E)^G(M)^T(E)^U(M) (AD1_ASS1.52e)
 
A(M)^G(E)^A(M)^A(E)^A(F)^U(F)^U(M)^C(M)^c^a^A(M)^C(F)^A(M)^A
(F)^A(M)^U(F)^A(M)^C(F)^C(E)^A(M)^C(E)^C(M) (AD1_PANK2.52e)
 
U(M)^G(E)^U(M)^C(E)^C(F)^A(F)^G(M)^U(M)^c^i^G(M)^A(F)^A(M)^U(
F)^A(M)^U(F)^A(M)^A(F)^T(E)^U(M)^A(E)^C(M) (AD1_NPHS2.52e)
 
G(M)^T(L)^C(M)^C(L)^C(M)^U(F)^U(F)^C(M)^U(M)^c^i^U(M)^C(F)^G(
M)^A(F)^U(M)^G(F)^G(M)^U(F)^C(M)^A(L)^G(M)^C(L)^A(M) (AD1_A1AT.39)
 wherein a capital letter denotes a ribonucleotide residue, a small letter represents a 2'-deoxyribonucleotide residue, N(M) represents a 2'-O-methyl- ribonucleotide residue, N(F) represents a 2'-fluoro-2'-deoxyribonucleotide residue, N(L) represents a 2'-O,4'-C-methylenated ribonucleotide residue, N(E) represents a 2'-O,4'-C-ethylenated ribonucleotide residue, and "^" represents that nucleoside units are linked by -P(=S)(OH)-.

32. The oligonucleotide, or a pharmaceutically acceptable salt thereof, according to any one of claims 1 to 31, wherein a delivery molecule containing GalNAc, cholesterol, and a fatty acid is bound via a linker or a phosphodiester bond (including a phosphorothioate bond) at the 5'-end or the 3'-end of the oligonucleotide and the pharmaceutically acceptable salt thereof.

33. A hereditary disease therapeutic agent comprising the oligonucleotide, or a pharmaceutically acceptable salt thereof, according to any one of claims 1 to 32, for use in the treatment of a disease associated with the target RNA.

34. A pharmaceutical composition comprising the oligonucleotide, or a pharmaceutically acceptable salt thereof, according to any one of claims 1 to 32, as an active component, for use in the therapy of a disease associated with the target RNA.

35. The pharmaceutical composition according to claim 34, for the prevention or therapy of a hereditary disease.

36. The pharmaceutical composition according to claim 35, wherein the hereditary disease is a disease curable by conversion of an adenosine residue into an inosine residue in the target RNA.

37. The pharmaceutical composition according to claim 35, wherein the hereditary disease is a hereditary disease caused by mutation of a guanosine residue to an adenosine residue in a gene.

38. Use of the oligonucleotide, or a pharmaceutically acceptable salt thereof, according to any one of claims 1 to 32, for production of a medicine for prevention or therapy of a disease associated with the target RNA.

39. The oligonucleotide, or a pharmaceutically acceptable salt thereof, according to any one of claims 1 to 32, for use in the prevention or therapy of a disease associated with the target RNA.

40. A method comprising administering a pharmacologically effective amount of the oligonucleotide, or a pharmaceutically acceptable salt thereof, according to any one of claims 1 to 32, to a warm-blooded animal, to prevent or cure a disease associated with the target RNA.

41. The method according to claim 40, wherein the disease is a hereditary disease.

42. The method according to claim 41, wherein the hereditary disease is a disease curable by conversion of an adenosine residue into an inosine residue in the target RNA.

43. The method according to claim 41, wherein the hereditary disease is a hereditary disease caused by mutation of a guanosine residue to an adenosine residue in a gene.

44. The pharmaceutical composition according to any one of claims 34 to 37, wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.

45. The use of the oligonucleotide, or a pharmaceutically acceptable salt thereof, according to claim 38, wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.

46. The oligonucleotide, or a pharmaceutically acceptable salt thereof, according to claim 39, wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.

47. The method according to any one of claims 40 to 43, wherein the disease comprises at least one selected from the group consisting of type I citrullinemia, hemophilia (thrombosis), ALS, pantothenic acid-related neurodegenerative disease, homocystinuria, focal segmental glomerulosclerosis, α1 anti-trypsin deficiency, phenyl ketonuria, pachydermoperiostosis, Alexander disease, primary hyperoxaluria, Gilbert syndrome, retinitis pigmentosa, distal myopathy, and hemochromatosis.
